# EUROPEAN PATENT SPECIFICATION

(11) **EP 0 923 387 B1**
(45) Date of publication and mention of the grant of the patent: **26.09.2001**
(21) Application number: 97933150.1
(22) Date of filing: 20.06.1997
(51) Int. Cl.: A61L 27/00

(54) **HEPARIN-COATED MEDICAL DEVICES FOR INTRAVENOUS USE CONTAINING HEPARIN-BINDING GROWTH FACTOR CONJUGATES**
HEPARIN-BESCHICHTETE MEDIZINISCHE GERÄTE ZUM INTRAVENÖSEN GEBRAUCH, DIE HEPARIN-BINDENDE WACHSTUMFAKTOR-KONJUGATE ENTHALTEN
INSTRUMENTS MEDICAUX RECOUVERTS D' HEPARINE A USAGE INTRAVEINEUX CONTENANT DES CONJUGUES DE FACTEUR DE CROISSANCE DES FIBROBLASTES

(30) Priority: 24.06.1996 US 672353
(43) Date of publication of application: 23.06.1999
(73) Proprietor: Selective Genetics, Inc., San Diego, CA 92121-1204 (US)
(72) Inventor: PIERCE, Glenn, Rancho Sante Fe, CA 92067 (US); BAIRD, J., Andrew, San Diego, CA 92122 (US)
(74) Representative: Gowshall, Jonathan Vallance
(86) International application number: US9710685
(87) International publication number: WO9749434

(56) References cited:
- EP-A- 0 686 395
- EP-A- 0 701 802
- EP-A- 0 716 836
- WO-A-90/12597
- WO-A-92/04918
- WO-A-92/11872
- WO-A-93/25688
- WO-A-96/06641
- WO-A-96/08274
- MATTAR S G ET AL.: "LOCAL INFUSION OF FGF-SAPORIN REDUCES INTIMAL HYPERPLASIA" JOURNAL OF SURGICAL RESEARCH, vol. 60, no. 2, 1 February 1996, US, pages 339-344, XP002049896
- BEHAR-COHEN F F ET AL.: "CYTOTOXIC EFFECTS OF FGF2-SAPORIN ON BOVINE EPITHELIAL LENS CELLS IN VITRO." INVESTIGATIVE OPHTHALMOLOGY & VISUAL SCIENCE, vol. 36, no. 12, 1995, pages 2425-2433, XP002049897

## Description

### Technical Field

The present invention relates generally to the delivery of cytotoxic agents, and specifically to the binding of heparin-binding growth factor-cytotoxic agent conjugates to heparinized intravenous medical devices.

### Background of the Invention

Growth factors play a critical role in many disorders and diseases, especially in promoting the unwanted or excessive growth of cells. Tumor growth, arthritis progression, atherosclerosis, and restenosis are mediated at least in part by the paracrine action of growth factors such as FGF, VEGF, and PDGF.

Restenosis in particular is a devastating condition occurring after intervention therapy, such as angioplasty, to open clogged arteries. As many as 40-60% of angioplasties result in restenosis, in which the arteries narrow more than 50% of the diameter at six months. Patients suffering from restenosis must often undergo additional interventional procedures to reopen arteries. Restenosis appears to involve smooth muscle cell proliferation, elastic recoil of the artery, and possibly vascular remodeling. Thrombosis may also play a role. Recoiling occurs due to the natural elasticity of a vessel, such that after the vessel is stretched by balloon angioplasty, the vessel naturally shrinks back to nearly its original size.

Preventing recoiling of the arteries is one approach to limiting restenosis. Recently, the stent has been introduced to prevent recoil. A stent is a wire-mesh cylindrical device, which is implanted into the vessel and physically resists the vessel's tendency to shrink. Using a stent, the restenosis rate is reduced to approximately 20-30%. Many of the stents tested in clinical trails are coated with heparin to inhibit formation of blood clots, called thromboses, which are promoted by metallic stents. The use of stents is gaining wide popularity in cardiac intervention therapies. Thus, there is a need in the art to further reduce complications of restenosis that occur after implantation of a stent, as well as through the use of other medical devices.

The present invention discloses the use of heparinized medical devices, including stents, bound with a growth factor-cytotoxic agent conjugate to deliver a cytotoxic agent in order to prevent unwanted cell growth, while providing other related advantages.

### Summary of the Invention

The present invention generally provides intravenous medical devices as claimed coated with a device-binding moiety. In one aspect, the moiety is a glycosaminoglycan and further has a glycosaminoglycan-binding moiety bound to a cytotoxic or cytocide encoding agent. In another aspect the medical device is coated with heparin and a heparin-binding growth factor-cytotoxic or cytocide encoding agent conjugate. Within the subject invention, the heparin is coated onto the medical device, and the conjugate is bound to the heparin. In one embodiment, the heparin-binding growth factor is heparin binding epidermal growth factor. In a related embodiment, it is a polypeptide reactive with a fibroblast growth factor receptor. In various embodiments, the cytotoxic agent is a ribosome inhibiting protein, and preferably saporin. In certain preferred embodiments, the medical device is a stent or a synthetic graft.

In the invention, devices are provided to inhibit proliferation of smooth muscle cells or to inhibit stenosis or restenosis complications after cardiac intervention therapy, including balloon angioplasty, vein graft, endarterectomy, arteriovenous shunt, or synthetic grafts. In certain aspects, a stent coated with heparin and a heparin-binding growth factor-cytotoxic agent conjugate is implanted subsequent to the noted procedure. In other aspects the graft is impregnated with the conjugate.

These and other aspects of the present invention will become evident upon reference to the following detailed description and attached drawings. In addition, various references are set forth below which describe in more detail certain procedures or compositions (*e.g*., plasmids, etc.).

### Brief Description of the Drawings and Sequence Listing

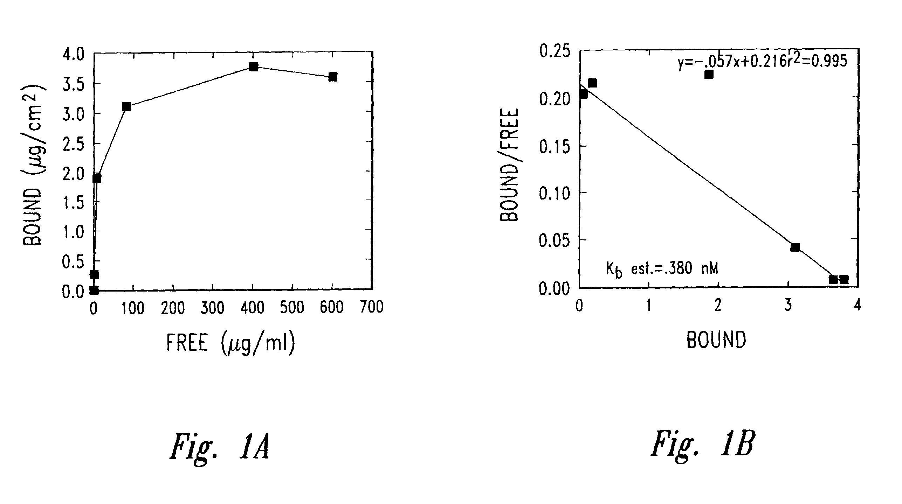

Figure 1 presents graphs showing the capacity of binding of ¹²⁵I-FGF-SAP to stents with heparin coating. Heparin-coated stents were washed in 10 mM NaCitrate, 140mM NaCl, 0.1 mM EDTA, 0.1% gelatin, 0.05% Tween-20, pH 6 (CBSGT), incubated in varying concentrations of FGF-SAP (0-600 ug/ml) in the presence of a fixed quantity of ¹²⁵I-FGF-SAP (60 ng/ml) for 30 min. at room temperature with shaking, then washed three times (5 min. each in CBSGT) to remove unbound drug. The quantity of radioactivity bound to the stent was assessed by gamma counting, and the total quantity of protein bound was calculated based on the specific activity of the ¹²⁵I-FGF-SAP and the ratio of labeled to unlabeled FGF-SAP. Scatchard analysis was performed on the data obtained.

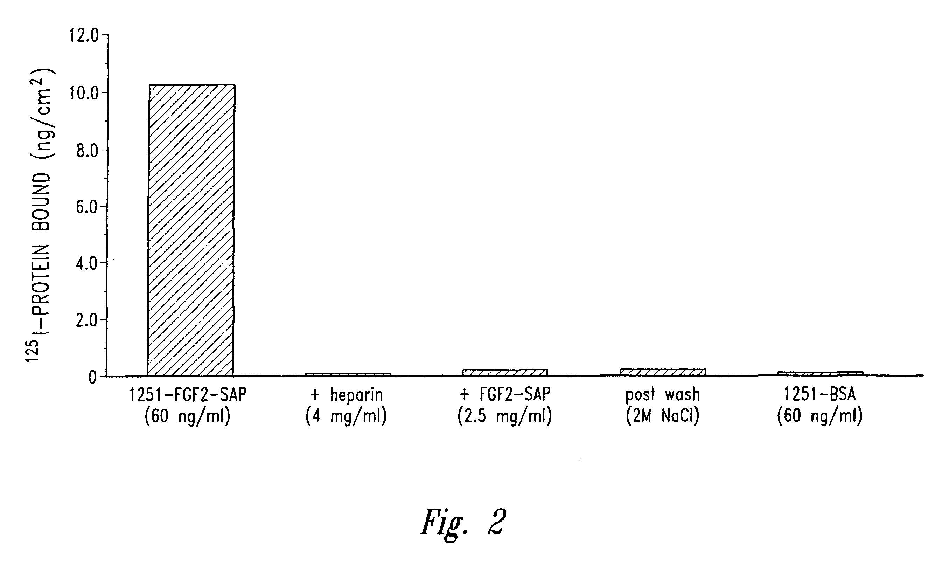

Figure 2 presents a graph showing the specificity of binding of ¹²⁵I-FGF-SAP to stents with heparin coating. Heparin-coated stents were incubated with a fixed quantity of ¹²⁵I-FGF-SAP in the presence or absence of 4 mg/ml heparin or 2.5 mg/ml unlabeled FGF-SAP. Stents were then washed and counted. Stents incubated with FGF-SAP alone were also washed in 2M NaCl to disrupt specific FGF-heparin binding. The last column shows the amount of the non-specific protein, ¹²⁵I-bovine serum albumin (BSA) that binds to the surface under the same conditions as FGF-SAP.

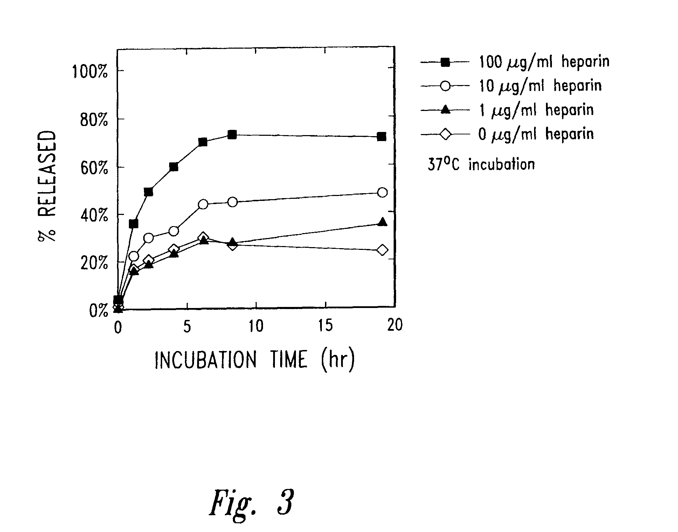

Figure 3 is a graph showing the release of FGF-SAP from stents coated with heparin. Heparin-coated stents were incubated with a fixed quantity of ¹²⁵I-FGF-SAP and unlabeled FGF-SAP (100 ug/ml) in the incubation mixture. Approximately 3 ug/cm² was bound to the surface. Stents with FGF-SAP bound were then incubated in normal human plasma (collection EDTA) at 37°C in the presence or absence of increasing concentrations of sodium heparin. Samples of the plasma were taken over a 19-hour period and counted to quantitate released drug (shown as a % of total bound).

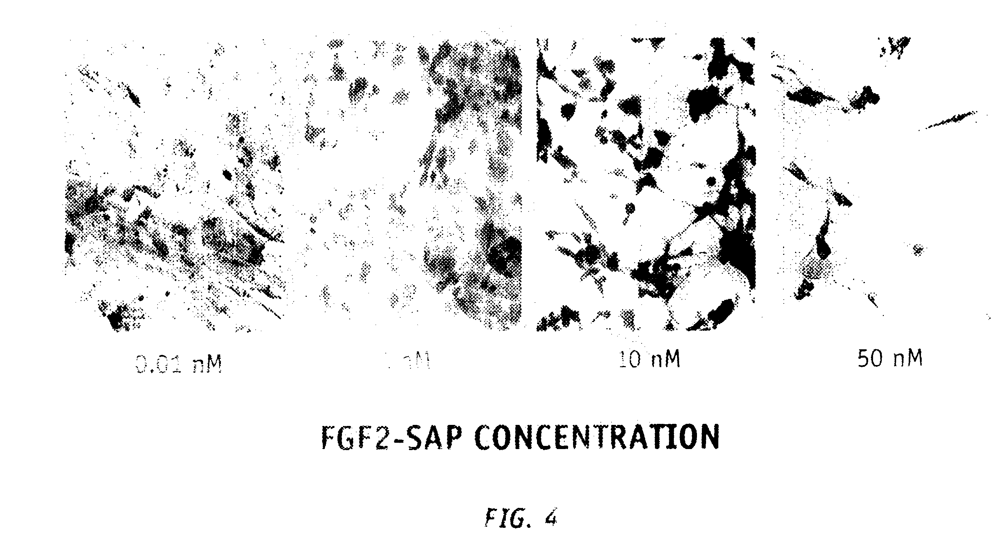

Figure 4 presents photographs of stained smooth muscle cells incubated with various concentrations of FGF-SAP in solution. Porcine aortic smooth muscle cells were seeded at a subconfluent density and 48 hours later, FGF-SAP was added. Cells were further cultured for 5 days. At the end of the 5 days, cells were fixed and stained with methylene blue.

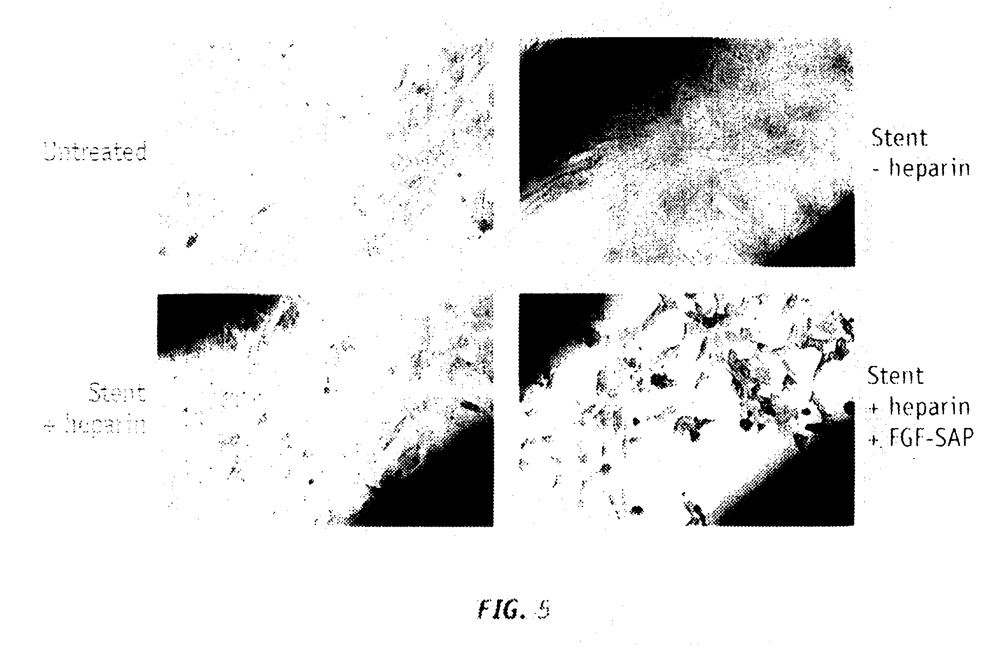

Figure 5 presents photographs of stained proliferating smooth muscle cells incubated with stents, heparin-coated stents, or heparin-coated stents bound with FGF-SAP. Porcine aortic smooth muscle cells were seeded at a subconfluent density. Forty-eight hours later, various stents were added to the cells. Five days later, cells were fixed and stained with methylene blue.

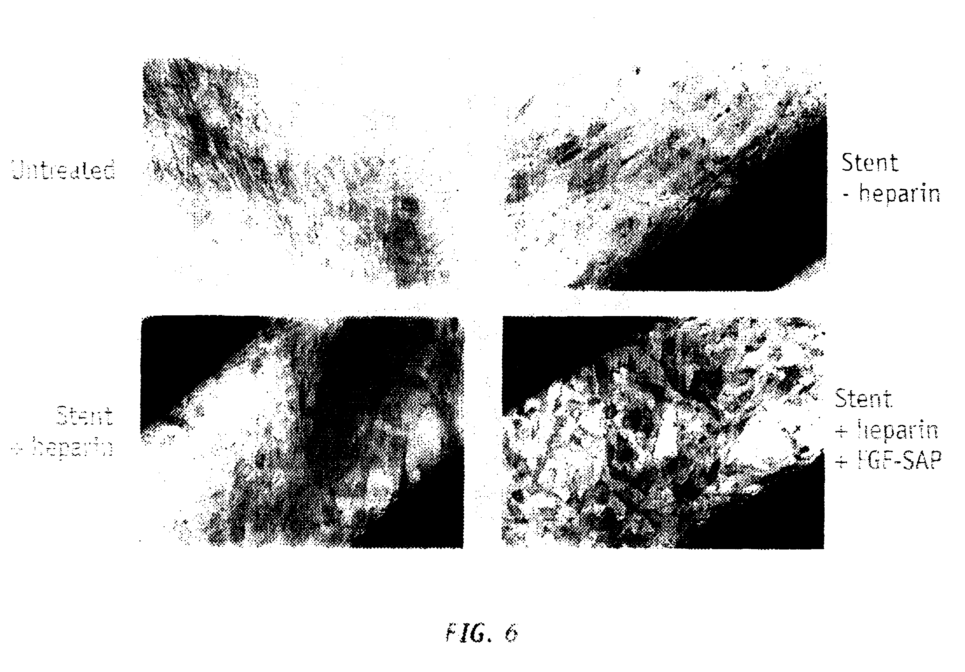

Figure 6 presents photographs of stained quiescent smooth muscle cells incubated with stents, heparin-coated stents, or heparin-coated stents bound with FGF-SAP. Porcine aortic smooth muscle cells were seeded at subconfluent density. Seven days later, the various stents were added to the wells, five days later, cells were fixed and stained with methylene blue.
SEQ ID No. 1 is the cDNA and protein sequence of FGF-2.
SEQ ID No. 2 is the cDNA and protein sequence of FGF-2-SAP.
SEQ ID No. 3 is the amino acid sequence of FGF-1.
SEQ ID No. 4 is the amino acid sequence of FGF-2.
SEQ ID No. 5 is the amino acid sequence of FGF-3.
SEQ ID No. 6 is the amino acid sequence of FGF-4.
SEQ ID No. 7 is the amino acid sequence of FGF-5.
SEQ ID No. 8 is the amino acid sequence of FGF-6.
SEQ ID No. 9 is the amino acid sequence of FGF-7.
SEQ ID No. 10 is the amino acid sequence of FGF-8.
SEQ ID No. 11 is the amino acid sequence of FGF-9.
SEQ ID No. 12 is the cDNA sequence of human HBEGF precursor protein.
SEQ ID No. 13 is the amino acid sequence of human HBEGF precursor protein.
SEQ ID No. 14 is the amino acid sequence of human mature HBEGF protein.
SEQ ID No. 15 is the amino acid sequence of monkey HBEGF precursor protein.
SEQ ID No. 16 is the amino acid sequence of rat HBEGF precursor protein.
SEQ ID No. 17 is the DNA sequence of EXON VII of VEGF.
SEQ ID No. 18 is His-Tag™ leader sequence.
SEQ ID No. 19 is a nuclear translocation sequence from SV40 large T antigen.
SEQ ID No. 20 is a nuclear translocation sequence from polyoma large T antigen.
SEQ ID No. 21 is a nuclear translocation sequence from human c-myc protein.
SEQ ID No. 22 is a nuclear translocation sequence from adenovirus EIA protein.
SEQ ID No. 23 is a nuclear translocation sequence from yeast mat α2 protein.
SEQ ID No. 24 is a nuclear translocation sequence from *c-erb-A* protein.
SEQ ID No. 25 is a nuclear translocation sequence from *c-erb-A* protein.
SEQ ID No. 26 is a nuclear translocation sequence from *c-erb-A* protein.
SEQ ID No. 27 is a nuclear translocation sequence from *c-myb* protein.
SEQ ID No. 28 is a nuclear translocation sequence from p53 protein.
SEQ ID No. 29 is a nuclear translocation sequence from nucleolin protein.
SEQ ID No. 30 is a nuclear translocation sequence from HIV *tat* protein.
SEQ ID No. 31 is a nuclear translocation sequence from FGF-1.
SEQ ID No. 32 is a nuclear translocation sequence from FGF-2.
SEQ ID No. 33 is a nuclear translocation sequence from FGF-3.
SEQ ID No. 34 is a nuclear translocation sequence from FGF-4.
SEQ ID No. 35 is a nuclear translocation sequence from FGF-6.
SEQ ID No. 36 is a nuclear translocation sequence from FGF-7
SEQ ID No. 37 is the Gly₄Ser flexible linker.
SEQ ID No. 38 is the (Gly₄Ser)₂ flexible linker.
SEQ ID No. 39 is the (Ser₄Gly)₄ flexible linker.
SEQ ID No. 40 is the (Ser₄Gly)₂ flexible linker.
SEQ ID No. 41 is the (AlaAlaProAla)ₙ flexible linker.
SEQ ID No. 42 is the cytoplasmic translocation signal KDEL.
SEQ ID No. 43 is the cytoplasmic translocation signal RDEL.
SEQ ID No. 44 is the cytoplasmic translocation signal KEEL.
SEQ ID No. 45 is an endosome-disruptive peptide from influenza virus.
SEQ ID No. 46 is an endosome-disruptive peptide from influenza virus.
SEQ ID No. 47 is the nucleotide sequence of saporin from clone M13 mp18-G4.
SEQ ID No. 48 is the nucleotide sequence of saporin from clone M13 mp18-G1.
SEQ ID No. 49 is the nucleotide sequence of saporin from clone M13 mp18-G2.
SEQ ID No. 50 is the nucleotide sequence of saporin from clone M13 mp18-G7.
SEQ ID No. 51 is the nucleotide sequence of saporin from clone M13 mp18-G9.
SEQ ID No. 52 is the N-terminal 40 amino acids of saporin SO-4.
SEQ ID No. 53 is the sense primer for the native saporin signal peptide with an *Eco*RI restriction site.
SEQ ID No. 54 is the antisense primer for the native saporin signal peptide with an *Eco*RI restriction site.
SEQ ID No. 55 is a 5' primer for saporin SO-6.
SEQ ID No. 56 is a 3' primer for saporin SO-6.
SEQ ID No. 57 is the genomic DNA sequence of bacteriophage lambda cII ribosome binding site.
SEQ ID No. 58 is the genomic DNA sequence of *Nco* I restriction enzyme site and N-terminus of saporin.
SEQ ID No. 59 is the genomic DNA sequence of the trp promoter.
SEQ ID No. 60 is the genomic DNA sequence of an *Nco* I restriction enzyme recognition site and carboxy terminus of mature FGF protein.
SEQ ID No. 61 is the DNA sequence of the c-terminus of wild type FGF with an *Nco I* restriction enzyme site.
SEQ ID No. 62 is the DNA sequence of pFGF*Nco*I.
SEQ ID No. 63 is a primer sequence with an *Nde* I restriction site for making Cys+4 saporin mutants.
SEQ ID No. 64 is a primer sequence with an *Nde* I restriction site for generating Cys+10 saporin mutants.
SEQ ID No. 65 is a primer sequence for generating Cys+4 and Cys+10 saporin mutants.
SEQ ID No. 66 is a primer sequence for generating SAP mutants.
SEQ ID No. 67 is the DNA sequence of SAP CYS +4.
SEQ ID No. 68 is the DNA sequence of SAP CYS +10.
SEQ ID No. 69 is a primer sequence for generating SAP mutants.
SEQ ID No. 70 is a primer sequence for generating SAP mutants.
SEQ ID No. 71 is an oligonucleotide sequence used for mutagenesis of cysteine 78 of FGF-2.
SEQ ID No. 72 is an oligonucleotide sequence used for mutagenesis of cysteine 96 of FGF-2.
SEQ ID No. 73 is a primer spanning the *Nde*I site at the 5' end of the FGF-encoding DNA from plasmid pZIB.
SEQ ID No. 74 is the sequence of an antisense primer spanning the Cys 78 codon of FGF-2.
SEQ ID No. 75 is the sequence of a sense primer to nucleotides spanning the Cys 78 codon of FGF-2.
SEQ ID No. 76 is the sequence of an antisense primer spanning the *Nco*I site of FGF in pZIB.
SEQ ID No. 77 is the sequence of a sense primer spanning the Cys 96 codon of FGF-2.
SEQ ID No. 78 is the sequence of an antisense primer spanning the Cys 96 codon of FGF-2.
SEQ ID No. 79 is the DNA sequence of cathepsin B substrate linker.
SEQ ID No. 80 is the DNA sequence of cathepsin D substrate linker.
SEQ ID No. 81 is the DNA sequence of trypsin substrate linker.
SEQ ID No. 82 is the DNA sequence of thrombin substrate linker.
SEQ ID No. 83 is the cDNA sequence of enterokinase substrate linker.
SEQ ID No. 84 is the DNA sequence of Factor Xa substrate linker.

### Detailed Description of the Invention

As an aid to understanding the invention, certain definitions are provided herein.

As used herein, "medical device" refers to any device for use in a patient. Especially of interest in the present invention are stents, tubing, probes, cannulas, catheters, synthetic vascular grafts, blood monitoring devices, and artificial heart valves.

As used herein, "glycosaminoglycans" (GAGs) refer to long, unbranched polysaccharide chains composed of repeating disaccharide units. One of the two sugar residues in the repeat is an amino sugar (N-acetyl glucosamine or N-acetyl galactosamine). GAGs fall into at least seven groups distinguished by their sugar residues, type of linkage between residues, and the number and location of sulfate groups. The groups are hyaluronic acid, chondroitin 4-sulfate, chondroitin 6-sulfate, dermatan sulfate, heparan sulfate, heparin and keratan sulfate.

As used herein, "heparin" refers to heparin or heparan sulfate, which is a group of anionic mucopolysaccharides generally having with a molecular weight from 6000 to 25,000 and composed of N-acetyl-D-glucosamine and D-glucuronic acid repeating disaccharide residues.

As used herein, "heparin-binding growth factor" refers to any member of a family of heparin-binding growth factor proteins, in which at least one member of the family binds heparin. Preferred growth factors in this regard include FGF, VEGF, and HBEGF. Such growth factors encompass isoforms, peptide fragments derived from a family member, splice variants, and single or multiple exons. Heparin-binding growth factors include the fibroblast growth factors (FGF), vascular endothelial growth factors (VEGF), and heparin-binding epidermal growth factor-like growth factors (HBEGF). In addition, other proteins, such as amphiregulin, and insulin growth factor binding protein-3 and -5 bind heparin. Within the context of this invention, the protein bound to the heparin coated stent must bind heparin and bind to a cell surface receptor. The bound protein may be one of the heparin-binding moieties discussed herein or a chimera between the heparin-binding domain derived from one of these moieties and a ligand for a cell surface receptor. For example, an FGF-heparin binding domain conjugated chemically or as a fusion protein with PDGF of TGF-B will bind a heparin-coated stent and a PDGF or TGF receptor, a cell surface receptor.

As used herein, "glycosamino-binding moiety" refers to any compound, including proteins, peptides, and chemicals, that bind a glycosaminoglycan. A molecule containing a GAG binding moiety has the ability to interact, at the molecular level with GAGs so as to be released in a time and concentration-dependent manner. The ability of a molecule to bind GAGs is determined by evaluating whether it will adhere to the GAG when the latter is immobilized to a solid surface or to a resin. If the GAG binding moiety does confer GAG binding to the molecule, then the molecule will be specifically bound to the GAG immobilized on the surface. Alternatively, the GAG binding moiety itself can be immobilized to a surface (or beads of a resin) and its ability to immobilize GAGs evaluated as above. One specific assay is to immobilize one component onto a BIOCOR chip as described by the manufacturer (Pharmacia, Biosensor) and determine the binding and release kinetics of the candidate molecules. Such moieties include, but are not limited to the heparin-binding growth factors noted above, thrombospondin, heparin-binding cytokines, such as GM-CSF, G-CSF, M-CSF, IL-8, IL-12, and δ-IFN, and heparin-binding domains of these proteins.

As used herein, to "target" a targeted agent, such as a cytotoxic agent, means to direct it to a cell that expresses a selected receptor by conjugating the agent to a polypeptide reactive with an FGF receptor, heparin-binding growth factor, or glycosaminoglycan-binding moiety. Upon binding to the receptor the conjugate is internalized by the cell and trafficked through the cell via the endosomal compartment, where cleavage of the conjugate can occur.

As used herein, when a conjugate is "bound" to heparin or glycosaminoglycan, the conjugate contains a domain that binds to heparin or glycosaminoglycan under physiological salt conditions. Preferably, the conjugate remains bound at 0.5M sodium.

### A. Heparin-binding moieties

Numerous growth factors and families of growth factors that share structural and functional features have been identified, including families of growth factors that specifically bind to heparin.

### 1. Fibroblast growth factors

One family of growth factors that has a broad spectrum of activities is the fibroblast growth factor (FGF) family. These proteins share the ability to bind to heparin, induce intracellular receptor-mediated tyrosine phosphorylation and the expression of the c-*fos* mRNA transcript, and stimulate DNA synthesis and cell proliferation. This family of proteins includes FGFs designated FGF-1 (acidic FGF (aFGF)), FGF-2/basic FGF (bFGF), FGF-3 (*int*-2) (*see, e.g.,* Moore et al., *EMBO J. 5*:919-924, 1986), FGF-4 (hst-1/K-FGF) *(see, e.g.,* Sakamoto et al., *Proc. Natl. Acad. Sci. U.S.A. 86*:1836-1840, 1986; U.S. Patent No. 5,126,323), FGF-5 *(see, e.g.*, U.S. Patent No. 5,155,217), FGF-6 (*hst*-2) *(see, e.g.,* published European Application EP 0 488 196 A2; Uda et al., *Oncogene 7*:303-309, 1992), FGF-7 (keratinocyte growth factor) (KGF; *see, e*.*g*., Finch et al., *Science 245*:752-755, 1985; Rubin et al., *Proc. Natl. Acad*. *Sci*. *U.S.A. 86*:802-806, 1989; and International Application WO 90/08771), FGF-8 *(see, e.g*., Tanaka et al., *Proc Natl*. *Acad. Sci. U.S.A. 89*:8528-8532, 1992); and FGF-9 *(see,* Miyamoto et al., *Mol. Cell. Biol. 13*:4251-4259, 1993).

As used herein, the term "polypeptide reactive with an FGF receptor" refers to any polypeptide that specifically interacts with an FGF receptor, preferably the high-affinity FGF receptor, and that is transported into the cell by virtue of its interaction with the FGF receptor. Polypeptides reactive with an FGF receptor are also called FGF proteins. Such polypeptides include, but are not limited to, FGF-1 to FGF-9. For example, bFGF (FGF-2) should be generally understood to refer to polypeptides having substantially the same amino acid sequences and receptor-targeting activity as that of bovine bFGF or human bFGF. It is understood that differences in amino acid sequences can occur among FGFs of different species as well as among FGFs from individual organisms or species. In addition, chimeras or hybrids of any of FGF-1 through FGF-9, or FGFs that have deletions *(see, e.g.*, Published International Application No. WO 90/02800 and national stage applications and patents based thereon), insertions or substitutions of amino acids are within the scope of FGF proteins, as long as the resulting peptide or protein specifically interacts with heparin.

Reference to FGFs is also intended to encompass proteins isolated from natural sources as well as those made synthetically, as by recombinant means or possibly by chemical synthesis. FGF also encompasses muteins of FGF that possess the ability to bind heparin. Such muteins include, but are not limited to, those produced by replacing one or more of the cysteines with serine as herein or that have any other amino acids deleted or replaced. Typically, such muteins will have conservative amino acid changes, such as those set forth below in Table 1. DNA encoding such muteins will, unless modified by replacement of degenerate codons, hybridize under conditions of at least low stringency to DNA encoding bFGF (SEQ ID NO. 1) or DNA encoding any of the FGFs set forth in SEQ ID. NOS. 3-11.

DNA encoding FGF peptides and/or the amino acid sequences of FGFs are known to those of skill in the art. DNA encoding an FGF may be prepared synthetically based on a known amino acid or DNA sequence isolated using methods known to those of skill in the art, or obtained from commercial or other sources. DNA encoding the FGF family of peptides is known. For example, DNA encoding of human FGF-1 (Jaye et al., *Science 233*:541-545, 1986; U.S. Patent No. 5,223,483), bovine FGF-2 (Abraham et al., *Science 233*:545-548, 1986; Esch et al., *Proc. Natl. Acad. Sci. USA 82:6507-6511,* 1985; and U.S. Patent *No. 4,956,455),* human FGF-2 (Abraham et al., *EMBO J*. *5*:2523-2528, 1986; U.S. Patent No. 4,994,559; U.S. Patent No. 5,155,214; EP 470,183B; and Abraham et al., *Quant*. *Biol. 51*:657-668, 1986) and rat FGF-2 *(see* Shimasaki et al., *Biochem. Biophys. Res. Comm.,* 1988, and Kurokawa et al., *Nucleic Acids Res. 16*:5201, 1988), FGF-3, FGF-6, FGF-7 and FGF-9 are known *(see, also,* U.S. Patent No. 5,155,214; U.S. Patent No. 4,956,455; U.S. Patent No. 5,026,839; U.S. Patent No. 4,994,559, EP 0,488,196 A2, DNASTAR, EMBL or GENBANK databases, and references discussed above and below).

Sequences of FGF polypeptides are set forth in SEQ ID NOS. 3-11. Any such DNA molecule may be isolated from a human cell library using any of the preceding DNA fragments as a probe. It is understood that once the complete amino acid sequence of a polypeptide, such as FGF, and the DNA fragment encoding such polypeptide are available to those of skill in this art, it is routine to substitute degenerate codons and produce any of the possible DNA fragments that encode such peptide. It is also generally possible to synthesize DNA encoding such a polypeptide based on the amino acid sequence.

Acidic and basic FGF, which were the first members of the FGF family that were characterized, are about 55% identical at the amino acid level and are highly conserved among species. Basic FGF has a molecular weight of approximately 16 kD, is basic and temperature sensitive and has a high isoelectric point. Acidic FGF has an acidic isoelectric point. The other members of the FGF family have subsequently been identified on the basis of amino acid sequence homologies with aFGF and bFGF and common physical and biological properties, including the ability to bind to one or more FGF receptors. Basic FGF, int-2, hst-1/K-FGF, FGF-5, hst-2/FGF-6 and FGF-8 are oncogenes. bFGF is expressed in melanomas, *int*-2 is expressed in mammary tumor virus and hst-1/K-FGF is expressed in angiogenic tumors. Acidic FGF, bFGF, KGF and FGF-9 are expressed in normal cells and tissues.

FGFs exhibit a mitogenic effect on a wide variety of mesenchymal, endocrine and neural cells. They are also important in differentiation and development. Of particular interest is their stimulatory effect on collateral vascularization and angiogenesis. Such effects have stimulated considerable interest in FGFs as therapeutic agents, for example, as pharmaceuticals for wound healing, neovascularization, nerve regeneration and cartilage repair. In addition to potentially useful proliferative effects, FGF-induced mitogenic stimulation may, in some instances, be detrimental. For example, cell proliferation and angiogenesis are an integral aspect of tumor growth. Members of the FGF family, including bFGF, are thought to play a pathophysiological role, for example, in tumor development, rheumatoid arthritis, proliferative diabetic retinopathies and other complications of diabetes.

The effects of FGFs are mediated by high affinity receptors which are tyrosine kinases in the cell surface membranes of FGF-responsive cells (*see, e.g*., Imamura et al., *Biochem*. *Biophys*. *Res*. *Comm*. *155*:583-590, 1988; Huang et al., *J*. *Biol*. *Chem. 261*:9568-9571, 1986, which are incorporated herein by reference). Lower affinity receptors also play a role in mediating FGF activities. The high affinity receptor proteins, which are single chain polypeptides with molecular weights ranging from 110 to 150 kD, depending on cell type, constitute a family of structurally related FGF receptors. Four FGF receptor genes have been identified, and at least two of these genes generate multiple mRNA transcripts via alternative splicing of the primary transcript. Receptors are described in International Application No. WO 91/00916, U.S. Patent Application Serial No. 07/377,033; International Application No. WO 92/00999, U.S. Patent Application Serial No. 07/549,587; International Application No. WO 90/05522; and International Application No. WO 92/12948; *see, also,* Imamura, *Biochem. Biophys. Res. Comm. 155*:583-590, 1988; Partanen et al., *EMBO J. 10*:1347-1354, 1991; and Moscatelli, *J. Cell. Physiol. 131*:123-130, 1987.

### 2. Vascular endothelial growth factors

Vascular endothelial growth factors (VEGFs) were identified by their ability to directly stimulate endothelial cell growth, but do not appear to have mitogenic effects on other types of cells. VEGFs also cause a rapid and reversible increase in blood vessel permeability. The members of this family have been referred to variously as vascular endothelial growth factor (VEGF), vascular permeability factor (VPF) and vasculotropin *(see, e.g.,* Plouet et al., *EMBO J.* 8:3801-3806, 1989). Herein, they are collectively referred to as VEGF.

VEGF was originally isolated from a guinea pig hepatocarcinoma cell line, line 10, *(see, e.g*., U.S. Patent No. 4,456,550) and has subsequently been identified in humans and in normal cells. It is expressed during normal development and in certain normal adult organs. Purified VEGF is a basic, heparin-binding, homodimeric glycoprotein that is heat-stable, acid-stable and may be inactivated by reducing agents.

DNA sequences encoding VEGF and methods to isolate these sequences may be found primarily in U.S. Patent No. 5,240,848, U.S. Patent No. 5,332,671, U.S. Patent No. 5,219,739, U.S. Patent No. 5,194,596, and Houch et al., *Mol. Endocrin.* 5:180, 1991.

VEGF family members arise from a single gene organized as eight exons and spanning approximately 14 kb in the human genome. Four molecular species of VEGF result from alternative splicing of mRNA and contain 121, 165, 189 and 206 amino acids. The four species have similar biological activities, but differ markedly in their secretion patterns. The predominant isoform secreted by a variety of normal and transformed cells is VEGF₁₆₅. Transcripts encoding VEGF₁₂₁ and VEGF₁₈₉ are detectable in most cells and tissues that express the VEGF gene. In contrast, VEGF₂₀₆ is less abundant and has been identified only in a human fetal liver cDNA library. VEGF₁₂₁ is a weakly acidic polypeptide that lacks the heparin binding domain and does not bind to heparin. VEGF₁₈₉ and VEGF₂₀₆ are more basic than VEGF₁₆₅ and bind to heparin with greater affinity. Although not every identified VEGF isoform binds heparin, all isoforms are considered to be heparin-binding growth factors within the context of this invention.

The secreted isoform, VEGF₁₆₅ is the preferred VEGF protein. The longer isoforms, VEGF₁₈₉ and VEGF₂₀₆, are almost completely bound to the extracellular matrix and need to be released by an agent, such as suramin, heparin or heparinase, and plasmin. Other preferred VEGF proteins contain various combinations of VEGF exons, such that the protein still binds heparin. It is not necessary that a VEGF protein used in the context of this invention retain activity of stimulating endothelial cell growth. It may be desirable in certain contexts for VEGF to manifest certain of its biological activities. For example, if VEGF is used as a carrier for DNA encoding a molecule useful in wound healing, it would be desirable that VEGF exhibit vessel permeability activity and promotion of fibroblast migration and angiogenesis. It will be apparent from the teachings provided within the subject application which of the activities of VEGF are desirable to maintain.

The heparin-binding domain of VEGF was originally localized to exons 6 and 7 because VEGF₁₂₁ lacks these exons and does not bind heparin. Further experiments have shown that exon 7 alone (SEQ. ID. NO. 17) confers heparin binding capability.

VEGF promotes an array of responses in endothelium, including blood vessel hyperpermeability, endothelial cell growth, angiogenesis, and enhanced glucose transport. VEGF stimulates the growth of endothelial cells from a variety of sources (including brain capillaries, fetal and adult aortas, and umbilical veins) at low concentrations, but is reported to have no effect on the growth of vascular smooth muscle cells, adrenal cortex cells, keratinocytes, lens epithelial cells, or BHK-21 fibroblasts. VEGF also is a potent polypeptide regulator of blood vessel function; it causes a rapid but transient increase in microvascular permeability without causing endothelial cell damage or mast cell degranulation, and its action is not blocked by antihistamines. VEGF has also been reported to induce monocyte migration and activation and has been implicated as a tumor angiogenesis factor in some human gliomas. Also, VEGF is a chemoattractant for monocytes and VEGF has been shown to enhance the activity of the inflammatory mediator tumor necrosis factor (TNF).

Quiescent and proliferating endothelial cells display high-affinity binding to VEGF, and endothelial cell responses to VEGF appear to be mediated by high affinity cell surface receptors. Two tyrosine kinases have been identified as VEGF receptors. The first, known as fms-like tyrosine kinase or FLT is a receptor tyrosine kinase that is specific for VEGF. The second receptor KDR (human kinase insert domain-containing receptor), and its mouse homologue FLK-1, are closely related to FLT. Messenger RNA encoding FLT and KDR have been identified in tumor blood vessels and specifically by endothelial cells of blood vessels supplying glioblastomas. Similarly, FLT and KDR mRNAs are upregulated in tumor blood vessels in invasive human colon adenocarcinoma, but not in the blood vessels of adjacent normal tissues.

### 3. Heparin-binding epidermal growth factor-like growth factors

Several mitogens in the epidermal growth factor protein family have recently been identified that display the ability to bind heparin. Among these is the mitogen known as heparin-binding EGF-like growth factor (HBEGF), which elutes from heparin-Sepharose® columns at about 1.0 - 1.2 M NaCl. HBEGF was first identified as a secreted product of cultured human monocytes, macrophages, and the macrophage-like U-937 cell line (Higashiyama et al., *Science 251*:936-939, 1991; Besner et al., *Cell Regul. 1*:811-19, 1990). HBEGF has been shown to interact with the same high affinity receptors as EGF on bovine aortic smooth muscle cells and human A431 epidermoid carcinoma cells (Higashiyama, *Science 251*:936-939, 1991).

As used herein, "heparin-binding epidermal growth factor-like growth factor (HBEGF) polypeptides" refer to any polypeptide that specifically interacts with a HBEGF receptor, a receptor to which native human HBEGF polypeptide binds and which transports the HBEGF intracellularly, that has a heparin-binding domain, and that is transported into the cell by virtue of its interaction with the receptor. In particular, as used herein, HBEGF refers to polypeptides having amino acid sequences of a native HBEGF polypeptide, as well as variants, having amino acid substitutions, deletions, insertions or additions in the native protein. Such HBEGF polypeptides include, but are not limited to human HBEGF (SEQ ID NO. 13), monkey HBEGF (SEQ ID NO. 15) and rat HBEGF (SEQ ID NO. 16). HBEGF polypeptides include those having SEQ ID NOS. 12-16, N-terminally or C-terminally shortened versions thereof, including mature HBEGFs, and also including modified versions of HBEGF.

"Mature HBEGF" refers to processed HBEGFs. It has been found that various isoforms of mature HBEGF have variable N-termini, and include, but are not limited to, those having N-termini corresponding to amino acid positions 63, 73, 74, 77 and 82 of the precursor human protein *(see, e.g*., SEQ ID NOS. 12-14).

Reference to HBEGFs is intended to encompass HBEGF polypeptides isolated from natural sources as well as those made synthetically, as by recombinant means or by chemical synthesis. This term encompasses precursor forms, such as those in SEQ ID NOS. 12, 13, 15 and 16, and mature forms, such as in SEQ ID NO. 14. HBEGF also encompasses muteins of HBEGF that possess the ability to target a targeted agent, such as a cytotoxic agent, including but not limited to ribosome-inactivating proteins, such as saporin, light activated porphyrin, and antisense nucleic acids, to HBEGF-receptor expressing cells. Such muteins include, but are not limited to, those produced by replacing one or more of the cysteines with serine as described hereinafter or that have any other amino acids deleted or replaced as long as the resulting protein has the ability to bind to HBEGF-receptor bearing cells and internalize the linked targeted agent. Typically, such muteins will have conservative amino acid changes, such as those set forth below in Table 1. DNA encoding such muteins will, unless modified by replacement of degenerate codons, hybridize under conditions of at least low stringency to DNA encoding native HBEGF (*e.g.*, SEQ ID NO. 12) and encode an HBEGF polypeptide, as defined herein.

As used herein, "DNA encoding an HBEGF peptide or polypeptide" refers to any DNA fragment encoding an HBEGF, as defined above. Exemplary DNA fragments include: any such DNA fragments known to those of skill in the art; any DNA fragment that encodes an HBEGF that binds to an HBEGF receptor and is internalized thereby and may be isolated from a human cell library using any of the preceding DNA fragments as a probe; and any DNA fragment that encodes any of the HBEGF polypeptides set forth in SEQ ID NOS. 13-16. Such DNA sequences encoding HBEGF fragments are available from publicly accessible databases, such as: GENBANK Accession Nos. M93012 (monkey) and M60278 (human); the plasmid pMTN-HBEGF (ATCC #40900) and pAX-HBEGF (ATCC #40899) described in published International Application WO/92,06705; and Abraham et al., *Biochem. Biophys. Res. Comm. 190*:125-133, 1993). DNA encoding HBEGF polypeptides will, unless modified by replacement of degenerate codons, hybridize under conditions of at least low stringency to DNA encoding a native HBEGF (*e.g.*, SEQ ID NO. 12). In addition, any DNA fragment that may be produced from any of the preceding DNA fragments by substitution of degenerate codons is also contemplated for use herein. It is understood that since the complete amino acid sequence of HBEGF polypeptides, and DNA fragments encoding such peptides, are available to those of skill in this art, it is routine to substitute degenerate codons and produce any of the possible DNA fragments that encode such HBEGF polypeptides. It is also generally possible to synthesize DNA encoding such peptides based on the amino acid sequence.

HBEGFs exhibit a mitogenic effect on a wide variety of cells including BALB/c 3T3 fibroblast cells and smooth muscle cells, but unlike VEGFs, are not mitogenic for endothelial cells (Higashiyama et al., *Science 251*:936-939, 1991). HBEGF has a stimulatory effect on collateral vascularization and angiogenesis. For example, cell proliferation and angiogenesis are an integral aspect of tumor growth. Members of the HBEGF family are thought to play a pathophysiological role, for example, in a variety of tumors, such as bladder carcinomas, breast tumors and non-small cell lung tumors. Thus, these cell types are also likely candidates for delivery of cytotoxic agents.

HBEGF isolated from U-937 cells is heterogeneous in structure and contains at least 86 amino acids and two sites of *O*-linked glycosyl groups (Higashiyama et al., *J*. *Biol. Chem. 267*:6205-6212, 1992). The carboxyl-terminal half of the secreted HBEGF shares approximately 35% sequence identity with human EGF, including six cysteines spaced in the pattern characteristic of members of the EGF protein family. In contrast, the amino-terminal portion of the mature factor is characterized by stretches of hydrophilic residues and has no structural equivalent in EGF. Site-directed mutagenesis of HBEGF and studies with peptide fragments have indicated that the heparin-binding sequences of HBEGF reside primarily in a twenty one-amino acid stretch upstream of and slightly overlapping the EGF-like domain.

The "HBEGF receptors (HBEGF-R)" specifically interact with members of the HBEGF family of proteins and transport HBEGF into the cell, *e.g.*, by receptor-mediated endocytosis. The effects of HBEGFs are mediated by EGF receptor tyrosine kinases expressed on cell surfaces of HBEGF-responsive cells *(see, e.g*., U.S. Patent Nos. 5,183,884 and 5,218,090; and Ullrich et al., *Nature* 309:4113-425, 1984), which are incorporated herein by reference). The EGF receptor proteins, which are single chain polypeptides with molecular weights 170 kD, constitute a family of structurally related EGF receptors. Cells known to express the EGF receptors include, for example, smooth muscle cells, fibroblasts, keratinocytes, and numerous human cancer cell lines, such as the: A431 (epidermoid); KB3-1 (epidermoid); COLO 205 (colon); CRL 1739 (gastric); HEP G2 (hepatoma); LNCAP (prostate); MCF-7 (breast); MDA-MB-468 (breast); NCI 417D (lung); MG63 (osteosarcoma); U-251 (glioblastoma); D-54MB (glioma); and SW-13 (adrenal).

### 4. Heparin-binding cytokines

Several cytokines exhibit heparin-binding. These cytokines include GM-CSF, G-CSF, M-CSF, IL-8, IL-12, and δ-IFN. DNA sequences encoding these cytokines may be obtained from GenBank.

### 6. Modifications of heparin-binding moieties

Heparin-binding molecules or glycosaminoglycan-binding molecules may be customized for the particular application. Means for modifying proteins are provided in detail below. Briefly, additions, substitutions and deletions of amino acids as well as the construction of chimeric proteins may be produced by any commonly employed recombinant DNA method.

Any polypeptide, peptide analogue, or peptidomimetic that is reactive with an FGF receptor, a VEGF receptor, or an HBEGF receptor and binds heparin or other glycosaminoglycan may be used in the methods herein. These include members of the families described herein and fragments thereof, as well as constrained analogs of such peptides that bind to one of the receptors and internalize a linked targeted agent. Members of the FGF peptide family, including FGF-1 - FGF-9, are particularly preferred. Modified peptides, including FGF polypeptides that have the nuclear translocating sequence (NTS) removed and chimeric peptides, which retain the specific binding and internalizing activities are also contemplated for use herein. Modified peptides, especially those lacking proliferative function, and chimeric peptides, which retain the specific binding and internalization activities are also contemplated for use herein.

In certain embodiments, the heterogeneity of preparations may be reduced by mutagenizing the protein to replace reactive cysteines, leaving, preferably, only one available cysteine for reaction. The protein is modified by deleting or replacing a site(s) on the protein that causes the heterogeneity. Such sites are typically cysteine residues that, upon folding of the protein, remain available for interaction with other cysteines or for interaction with more than one cytotoxic molecule per molecule of heparin-binding peptide. Thus, such cysteine residues do not include any cysteine residue that are required for proper folding of the growth factor or for retention of the ability to bind to a growth factor receptor and internalize. For chemical conjugation, one cysteine residue that, in physiological conditions, is available for interaction, is not replaced because it is used as the site for linking the cytotoxic moiety. The resulting modified heparin-binding protein is conjugated with a single species of cytotoxic conjugate.

Alternatively, the contribution of each cysteine to the ability of the heparin-binding moiety to bind to its cell surface receptor may be determined empirically. Each cysteine residue is systematically replaced with a conservative amino acid change (see Table 1, above) or deleted. The resulting mutein is tested for the requisite biological activity: the ability to bind to receptor and internalize linked nucleic acid binding domain and agents. If the mutein retains this activity, then the cysteine residue is not required. Additional cysteines are systematically deleted and replaced and the resulting muteins are tested for activity. Each of the remaining cysteine residues may be systematically deleted and/or replaced by a serine residue or other residue that would not be expected to alter the structure of the protein. The resulting peptide is tested for biological activity. If the cysteine residue is necessary for retention of biological activity it is not deleted; if it not necessary, then it is preferably replaced with a serine or other residue that should not alter the secondary structure of the resulting protein. In this manner the minimum number and identity of the cysteines needed to retain the ability to bind to a heparin-binding growth factor receptor and internalize may be determined. It is noted, however, that modified or mutant heparin-binding proteins may exhibit reduced or no proliferative activity, but may be suitable for use herein, if they retain the ability to target a linked cytotoxic agent to cells bearing receptors to which the unmodified heparin-binding protein binds and result in internalization of the cytotoxic moiety. For example, monomeric VEGF, VEGF₁₂₁ contains 9 cysteines and each of VEGF₁₆₅, VEGF₁₈₉ and VEGF₂₀₆ contain 7 additional residues in the region not present in VEGF₁₂₁. Any of the 7 are likely to be non-essential for targeting and internalization of linked cytotoxic agents. Recently, the role of Cys-25, Cys-56, Cys-67, Cys-101, and Cys-145 in dimerization and biological activity was assessed (Claffery et al., *Biochem. Biophys. Acta 1246*:1-9, 1995). Dimerization requires Cys-25, Cys-56, and Cys-67. Substitution of any one of these cysteine residues resulted in secretion of a monomeric VEGF, which was inactive in both vascular permeability and endothelial cell mitotic assays. In contrast, substitution of Cys 145 had no effect on dimerization, although biological activities were somewhat reduced. Substitution of Cys-101 did not result in the production of a secreted or cytoplasmic protein. Thus, substitution of Cys-145 is preferred. In addition, fragments of these molecules that bind heparin may be constructed and used. Regions of many of these ligands have been delineated as mediating binding.

Modification of the polypeptide may be effected by any means known to those of skill in this art. The preferred methods herein rely on modification of DNA encoding the polypeptide and expression of the modified DNA.

As an example, DNA encoding the FGF polypeptide may be isolated, synthesized or obtained from commercial sources (the amino acid sequences of FGF-1-FGF-9 are set forth in SEQ ID NOS. 3-11; DNA sequences may be based on these amino acid sequences or may be those that are known to those of skill in this art *(see, e.g.,* GenBank; *see, also*, U.S. Patent No. 4,956,455, U.S. Patent No. 5,126,323, U.S. Patent No. 5,155,217, U.S. Patent No. 4,868,113, PCT Application WO 90/08771; U.S. Application Serial No. 07/304,281; EP Application 488 196 A2; and Miyamoto et al., *Mol. Cell. Biol. 13*:4251-4259, 1993). Expression of a recombinant bFGF protein in yeast and *E. coli* is described in Barr et al., *J. Biol. Chem.* 263:16471-16478, 1988, PCT Application PCT/US93/05702 and United States Application No. 07/901,718. Expression of recombinant FGF proteins may be performed as described herein or using methods known to those of skill in the art; and DNA encoding FGF proteins may be used as the starting materials for the methods herein.

Similarly, DNA encoding HBEGF or other heparin-binding or glycosaminoglycan-binding moiety may also be isolated, synthesized or obtained from commercial sources. DNA sequences are available in public databases, such as GenBank, or obtained from commercial vendors or private parties. Based on these sequences, oligonucleotide primers may be designed and used to amplify the gene from cDNA or mRNA by polymerase chain reaction technique.

Mutation may be effected by any method known to those of skill in the art, including site-specific or site-directed mutagenesis of DNA encoding the protein and the use of DNA amplification methods using primers to introduce and amplify alterations in the DNA template, such as PCR splicing by overlap extension (SOE). Site-specific mutagenesis is typically effected using a phage vector that has single- and double-stranded forms, such as M13 phage vectors, which are well-known and commercially available. Other suitable vectors that contain a single-stranded phage origin of replication may be used *(see, e.g.*, Veira et al., *Meth. Enzymol. 15*:3, 1987). In general, site-directed mutagenesis is performed by preparing a single-stranded vector that encodes the protein of interest (*e.g.,* a member of the FGF family). An oligonucleotide primer that contains the desired mutation within a region of homology to the DNA in the single-stranded vector is annealed to the vector followed by addition of a DNA polymerase, such as *E. coli* DNA polymerase I (Klenow fragment), which uses the double stranded region as a primer to produce a heteroduplex in which one strand encodes the altered sequence and the other the original sequence. The heteroduplex is introduced into appropriate bacterial cells and clones that include the desired mutation are selected. The resulting altered DNA molecules may be expressed recombinantly in appropriate host cells to produce the modified protein.

Suitable conservative substitutions of amino acids are known to those of skill in this art may be made generally without altering the biological activity of the resulting molecule. For example, such substitutions may be made in accordance with those set forth in Table 1 as follows:

**TABLE 1**

| **Original residue** | **Conservative substitution** |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other similarly conservative substitutions may be made. If necessary such substitutions may be determined empirically merely by testing the resulting modified protein for the ability to bind to and internalize upon binding to the appropriate receptor. Those that retain this ability are suitable for use in the conjugates and methods herein. In addition, muteins of the FGFs are known to those of skill in the art (*see, e.g*., U.S. Patent No. 5,175,147; PCT Application WO 89/00198; U.S. Application Ser. No. 07/070,797; PCT Application WO 91/15229; U.S. Application Ser. No. 07/505,124).

As discussed above, chimeras of heparin-binding growth factors and other moieties are useful within the context of this invention as long as the chimera binds heparin or a glycosaminoglycan, and a cell surface receptor and is internalized. For example, a heparin binding domain from a heparin-binding growth factor may be conjugated, preferably as a fusion protein, with a growth factor or other ligand. The heparin binding domains of the heparin-binding growth factors are known. For example, the heparin-binding domain of HBEGF is contained in amino acids 1-45 (Mesri et al., *J. Cell. Science 107*:2599-2608, 1994); the heparin binding domains for FGF-1 *(see* Imamura et al., *BBA 1266*:124-130, 1995); Thompson et al., *Biochemistry* 33:3831-3840, 1994); thrombospondin (Vogel et al., *J. Cellular Biochem. 53:74-84,* 1993); FGF-2 (Presta et al., *BBRC 185*:1098-1107, 1992; Heath et al., *Biochemistry 30*:5608-5615, 1991) and VEGF are known. The domains may be isolated from DNA molecules by restriction digestion or PCR amplification and inserted into a vector to be expressed as a fusion protein with another growth factor.

### B. Cytotoxic agent

As used herein, the term "cytotoxic agent" refers to a molecule capable of inhibiting cell function. The agent may inhibit proliferation or may be toxic to cells. The term includes agents whose toxic effects are mediated only when transported into the cell and also those whose toxic effect is mediated at the cell surface. A variety of cytotoxic agents can be used and include those that inhibit protein synthesis and those that inhibit expression of certain genes essential for cellular growth or survival. Cytotoxic agents include those that result in cell death and those that inhibit cell growth, proliferation and/or differentiation.

The cytotoxic agents include saporin, ricin, abrin, and other RIPs, *Pseudomonas* exotoxin, diphtheria toxin, angiogenin, tritin, dianthins 32 and 30 doxirubicin, momordin, pokeweed antiviral protein, mirabilis antiviral protein, bryodin, shigo exotoxin, ribozymes, antisense prodrugs, such as HSV-Tk, or other metabolic inhibitors that are known to those of skill in this art. Other cytotoxic molecules that inhibit cellular metabolic process, including transcription, translation, biosynthetic or degradative pathways, DNA, RNA or protein synthesis and other such process, or that kill cells are also useful in the present invention.

### 1. Ribosome inactivating proteins

Ribosome inactivating proteins (RIPs), including ricin, abrin and saporin, are plant proteins that catalytically inactivate eukaryotic ribosomes. Some RIPs, such as the toxins abrin and ricin, contain two constituent chains: a cell-binding chain that mediates binding to cell surface receptors and internalizing the molecule; and a chain responsible for toxicity. Single chain RIPs (type I RIPS), such as the saporins, do not have a cell-binding chain. As a result, unless internalized, they are substantially less toxic to whole cells than the RIPs that have two chains.

RIPs inactivate ribosomes by interfering with the protein elongation step of protein synthesis. For example, the RIP saporin (hereinafter also referred to as SAP) has been shown to inactivate 60S ribosomes by cleavage of the N-glycosidic bond of the adenine at position 4324 in the rat 28S ribosomal RNA (rRNA). The particular region in which A₄₃₂₄ is located in the rRNA is highly conserved among prokaryotes and eukaryotes. A₄₃₂₄ in 28S rRNA corresponds to A₂₆₆₀ in *Escherichia coli* (*E. coli*) 23S rRNA. Several RIPs also appear to interfere with protein synthesis in prokaryotes, such as *E. coli*.

Saporin and other ribosome inactivating proteins (RIPs) are the preferred cytotoxic agent for use herein. Other cytotoxic agents, considered to be functionally equivalent to the RIPs described herein, include, but are not limited to, saporin, the ricins, abrin and other RIPs, *Pseudomonas* exotoxin, inhibitors of DNA, RNA or protein synthesis or other metabolic inhibitors that are known to those of skill in this art. Saporin is preferred, but other suitable RIPs include, but are not limited to, ricin, ricin A chain, maize RIP, gelonin, diphtheria toxin, diphtheria toxin A chain, trichosanthin, tritin, pokeweed antiviral protein (PAP), mirabilis antiviral protein (MAP), Dianthins 32 and 30, abrin, monordin, bryodin, shiga and others known to those of skill in this art (*see, e.g.,* Barbieri et al., *Cancer Surveys 1*:489-520, 1982, and European application No. 0466 222; *see, also*, U.S. Patent No. 5,248,608).

Saporin (abbreviated herein as SAP) refers to polypeptides having amino acid sequences found in the natural plant host *Saponaria officinalis*, as well as modified sequences, having amino acid substitutions, deletions, insertions or additions, which still express substantial ribosome-inactivating activity. Purified preparations of saporin are frequently observed to include several molecular isoforms of the protein. It is understood that differences in amino acid sequences can occur in saporin from different species as well as between saporin molecules from individual organisms of the same species.

Several structurally related RIPs have been isolated from seeds and leaves of the plant *Saponaria officinalis* (soapwort). Among these, SAP-6 is the most active and abundant, representing 7% of total seed proteins. Saporin is very stable, has a high isoelectric point, does not contain carbohydrates, and is resistant to denaturing agents, such as sodium dodecyl sulfate (SDS), and a variety of proteases. The amino acid sequences of several saporin-6 isoforms from seeds are known and there appear to be families of saporin RIPs differing in few amino acid residues. Because saporin is a type I RIP, it does not possess a cell-binding chain. Consequently, its toxicity to whole cells is much lower than other toxins, such as ricin and abrin. When internalized by eukaryotic cells, however, its cytotoxicity is 100- to 1000-fold more potent than ricin A chain.

If necessary, the selected cytotoxic agent is derivatized to produce a group reactive with a cysteine on the selected FGF. If derivatization results in a mixture of reactive species, a mono-derivatized form of the cytotoxic agent is isolated and is then conjugated to the mutated FGF.

The saporin polypeptides include any of the isoforms of saporin that may be isolated from *Saponaria officinalis* or related species or modified form that retain cytotoxic activity. In particular, such modified saporin may be produced by modifying the DNA encoding the protein (*see, e.g.,* PCT Application PCT/US93/05702, United States Application Serial No. 07/901,718; *see, also,* U.S. Patent Application No. 07/885,242, and Italian Patent No. 1231914), by altering one or more amino acids or deleting or inserting one or more amino acids, such as a cysteine that may render it easier to conjugate to FGF or other cell surface binding protein. Any such protein, or portion thereof, that, when conjugated to FGF as described herein, that exhibits cytotoxicity in standard *in vitro* or *in vivo* assays within at least about an order of magnitude of the saporin conjugates described herein is contemplated for use herein.

Thus, the SAP used herein includes any protein that is isolated from natural sources or that is produced by recombinant expression *(see, e.g.*, PCT Application PCT/US93/05702, United States Application Serial No. 07/901,718).

DNA encoding SAP or any cytotoxic agent may be used in the recombinant methods provided herein. In instances in which the cytotoxic agent does not contain a cysteine residue, such as instances in which DNA encoding SAP is selected, the DNA may be modified to include a cysteine codon. The codon may be inserted into any locus that does not reduce or reduces by less than about one order of magnitude the cytotoxicity of the resulting protein may be selected. Such locus may be determined empirically by modifying the protein and testing it for cytotoxicity in an assay, such as a cell-free protein synthesis assay. The preferred loci in SAP for insertion of the cysteine residue is at or near the N-terminus (within about 10 residues of the N-terminus).

Host organisms include those organisms in which recombinant production of heterologous proteins have been carried out and in which the cytotoxic agent, such as saporin, is not toxic or is of sufficiently low toxicity to permit expression before cell death. Presently preferred host organisms are strains of bacteria. Most preferred host organisms are strains of *E. coli,* particularly, BL21(DE3) cells (Novagen, Madison, WI).

### 2. Methods for recombinant production of cytotoxic agents

Cytotoxic agents are conveniently produced by standard recombinant methods and techniques (*see* Sambrook et al., *infra*). These methods discussed below are generally suitable for any protein cytotoxic agent. Methods generally applicable for production of heparin-binding growth factors are discussed in section D, below. Here, for exemplary purposes only, recombinant production of saporin is discussed.

The DNA encoding the cytotoxic agent, such as saporin protein, is introduced into a plasmid in operative association with an appropriate promoter for expression of polypeptides in a selected host organism. The presently preferred saporin proteins are saporin proteins that have been modified by addition of a Cys residue or replacement of a non-essential residue at or near the amino- or carboxyl terminus of the saporin with Cys. Saporin, such as that of SEQ ID NO. 47, has been modified by insertion of Met-Cys residue at the N-terminus and has also been modified by replacement of the Asn or Ile residue at positions 4 and 10, respectively *(see* Example 4). The DNA fragment encoding the saporin may also include a protein secretion signal that functions in the selected host to direct the mature polypeptide into the periplasm or culture medium. The resulting saporin protein can be purified by methods routinely used in the art, including, methods described hereinafter in the Examples.

Methods of transforming suitable host cells, preferably bacterial cells, and more preferably *E. coli* cells, as well as methods applicable for culturing said cells containing a gene encoding a heterologous protein, are generally known in the art. See, for example, Sambrook et al., *Molecular Cloning: A Laboratory Manual,* Cold Spring Harbor Laboratory Press, Cold Spring Harbor, NY, 1989.

The DNA construct encoding the saporin protein is introduced into the host cell by any suitable means, including, but not limited to transformation employing plasmids, viral, or bacterial phage vectors, transfection, electroporation, lipofection, and the like. The heterologous DNA can optionally include sequences, such as origins of replication that allow for the extrachromosomal maintenance of the saporin-containing plasmid, or can be designed to integrate into the genome of the host (as an alternative means to ensure stable maintenance in the host).

Positive transformants can be characterized by Southern blot analysis (Sambrook et al., *supra)* for the site of DNA integration; Northern blots for inducible-promoter-responsive saporin gene expression; and product analysis for the presence of saporin-containing proteins in either the cytoplasm, periplasm, or the growth media.

Once the saporin-encoding DNA fragment has been introduced into the host cell, the desired saporin-containing protein is produced by subjecting the host cell to conditions under which the promoter is induced, whereby the operatively linked DNA is transcribed. In a preferred embodiment, such conditions are those that induce expression from the *E. coli* lac operon. The plasmid containing the DNA encoding the saporin-containing protein also includes the lac operator (O) region within the promoter and may also include the lac I gene encoding the lac repressor protein (*see*, *e.g.*, Muller-Hill et al., *Proc. Natl. Acad. Sci. USA* 59:1259-12649, 1968). The lac repressor represses the expression from the lac promoter until induced by the addition of IPTG in an amount sufficient to induce transcription of the DNA encoding the saporin-containing protein.

The expression of saporin in *E. coli* is thus accomplished in a two-stage process. In the first stage, a culture of transformed *E. coli* cells is grown under conditions in which the expression of the saporin-containing protein within the transforming plasmid, preferably a encoding a saporin, such as described in Example 4, is repressed by virtue of the lac repressor. In this stage, cell density increases. When an optimum density is reached, IPTG is added to prevent binding of repressor to the operator, thereby inducing the lac promoter and transcription of the saporin-encoding DNA.

In a preferred embodiment, the promoter is the T7 RNA polymerase promoter, which may be linked to the lac operator, and the *E. coli* host strain includes DNA encoding T7 RNA polymerase operably linked to the lac operator and a promoter, preferably the lacUV5 promoter. The presently preferred plasmid is pET 11a (Novagen, Madison, WI), which contains the T71ac promoter, T7 terminator, the inducible *E. coli* lac operator, and the lac repressor gene. The plasmid pET 15b (Novagen, Madison, WI), which contains a His-Tag™ leader sequence (SEQ ID NO. 18) for use in purification with a His column and a thrombin cleavage site that permits cleavage following purification over the column, the T7-lac promoter region and the T7 terminator, has been used herein for expression of saporin. Addition of IPTG induces expression of the T7 RNA polymerase and the T7 promoter, which is recognized by the T7 RNA polymerase.

Transformed strains, which are of the desired phenotype and genotype, are grown in fermentors by suitable methods well known in the art. In the first, or growth stage, expression hosts are cultured in defined minimal medium lacking the inducing condition, preferably IPTG. When grown in such conditions, heterologous gene expression is completely repressed, which allows the generation of cell mass in the absence of heterologous protein expression. Subsequent to the period of growth under repression of heterologous gene expression, the inducer, preferably IPTG, is added to the fermentation broth, thereby inducing expression of any DNA operatively linked to an IPTG-responsive promoter (a promoter region that contains lac operator). This last stage is the induction stage.

The resulting saporin-containing protein can be suitably isolated from the other fermentation products by methods routinely used in the art, *e.g.*, using a suitable affinity column as described in the Examples; precipitation with ammonium sulfate; gel filtration; chromatography, preparative flat-bed iso-electric focusing; gel electrophoresis, high performance liquid chromatography (HPLC); and the like. A method for isolating saporin is provided in Example 1 *(see, also*, Lappi et al., *Biochem. Biophys. Res. Commun. 129*:934-942, *1985*). The expressed saporin protein is isolated from either the cytoplasm, periplasm, or the cell culture medium (see, discussion herein and Examples).

### C. Cytocide-encoding agent

In one aspect of the present invention, the heparin-binding moiety or glycosaminoglycan-binding moiety is conjugated, either by chemical linkage or as a fusion protein, to a nucleic acid binding domain and the conjugate is complexed with a nucleic acid encoding a cytocide-encoding agent. Thus, the moiety is used to deliver a cytotoxic agent in the form of a nucleic acid molecule. In this context, the heparin-binding moiety is a receptor-binding internalized ligand.

A cytocide-encoding agent is a nucleic acid molecule (DNA or RNA) that, upon internalization by a cell, and subsequent transcription and/or translation into a cytocidal agent, is cytotoxic to a cell or inhibits cell growth by inhibiting protein synthesis.

Cytocides include saporin, the ricins, abrin and other ribosome-inactivating proteins, *Pseudomonas* exotoxin, diptheria toxin, angiogenin, tritin, dianthins 32 and 30, momordin, pokeweed antiviral protein, mirabilis antiviral protein, bryodin, angiogenin, and shiga exotoxin, as well as other cytocides that are known to those of skill in the art.

Especially of interest are DNA molecules that encode an enzyme that results in cell death or renders a cell susceptible to cell death upon the addition of another product. For example, saporin, a preferred cytocide, is an enzyme that cleaves rRNA and inhibits protein synthesis. Other enzymes that inhibit protein synthesis are especially well suited for use in the present invention. In addition, enzymes may be used where the enzyme activates a compound with little or no cytotoxicity into a toxic product that inhibits protein synthesis.

In addition to saporin discussed above, other cytocides that inhibit protein synthesis are useful in the present invention. The gene sequences for these cytocides may be isolated by standard methods, such as PCR, probe hybridization of genomic or cDNA libraries, antibody screenings of expression libraries, or obtain clones from commercial or other sources. The DNA sequences of many of these cytocides are well known, including ricin A chain (GenBank Accession No. X02388); maize ribosome-inactivating protein (GenBank Accession No. L26305); gelonin (GenBank Accession No. L12243; PCT Application WO 92/03155; U.S. Patent No. 5,376,546; diphtheria toxin (GenBank Accession No. K01722); trichosanthin (GenBank Accession No. M34858); tritin (GenBank Accession No. D13795); pokeweed antiviral protein (GenBank Accession No. X78628); mirabilis antiviral protein (GenBank Accession No. D90347); dianthin 30 (GenBank Accession No. X59260); abrin (GenBank Accession No. X55667); shiga (GenBank Accession No. M19437) and *Pseudomonas* exotoxin (GenBank Accession Nos. K01397, M23348).

In the case of cytotocide molecules such as the ribosome-inactivating proteins, very few molecules may need be present for cell killing. Indeed, only a single molecule of diphtheria toxoid introduced into a cell was sufficient to kill the cell. In other cases, it may be that propagation or stable maintenance of the construct is necessary to attain sufficient numbers or concentrations of the gene product for effective gene therapy. Examples of replicating and stable eukaryotic plasmids are found in the scientific literature.

In general, constructs will also contain elements necessary for transcription and translation. If the cytocide-encoding agent is DNA, then it must contain a promoter. The choice of the promoter will depend upon the cell type to be transformed and the degree or type of control desired. Promoters can be constitutive or active in any cell type, tissue specific, cell specific, event specific or inducible. Cell-type specific promoters and event type specific promoters are preferred. Examples of constitutive or nonspecific promoters include the SV40 early promoter (U.S. Patent No. 5,118,627), the SV40 late promoter (U.S. Patent No. 5,118,627), CMV early gene promoter (U.S. Patent No. 5,168,062), and adenovirus promoter. In addition to viral promoters, cellular promoters are also amenable within the context of this invention. In particular, cellular promoters for the so-called housekeeping genes are useful. Viral promoters are preferred, because generally they are stronger promoters than cellular promoters.

Tissue specific promoters are particularly useful when a particular tissue type is to be targeted for transformation. By using one of this class of promoters, an extra margin of specificity can be attained. For example, when the indication to be treated is ophthalmological, either the alpha-crystalline promoter or gamma-crystalline promoter is preferred. When a tumor is the target of gene delivery, cellular promoters for specific tumor markers or promoters more active in tumor cells should be chosen. Thus, to transform prostate tumor cells the prostate-specific antigen promoter is especially useful. Similarly, the tyrosinase promoter or tyrosinase-related protein promoter is a preferred promoter for melanoma treatment. For B lymphocytes, the immunoglobulin variable region gene promoter, for T lymphocytes, the TCR receptor variable region promoter, for helper T lymphocytes, the CD4 promoter, for liver, the albumin promoter, are but a few examples of tissue specific promoters. Many other examples of tissue specific promoters (*e.g.*, CEA, erb B-2, c-myc, alpha-fetoprotein promoters) are readily available to one skilled in the art.

Inducible promoters may also be used. These promoters include the MMTV LTR (PCT WO 91/13160), which is inducible by dexamethasone, metallothionein, which is inducible by heavy metals, and promoters with cAMP response elements, which are inducible by cAMP. By using an inducible promoter, the nucleic acid may be delivered to a cell and will remain quiescent until the addition of the inducer. This allows further control on the timing of production of the therapeutic gene.

Event-type specific promoters are active only upon the occurrence of an event, such as tumorigenicity or viral infection. The HIV LTR is a well known example of an event-specific promoter. The promoter is inactive unless the *tat* gene product is present, which occurs upon viral infection.

Additionally, promoters that are coordinately regulated with a particular cellular gene may be used. Merely by way of example, promoters of genes that are coordinately expressed when a particular FGF receptor gene is expressed may be used. Then, the nucleic acid will be transcribed when the FGF receptor is expressed. This type of promoter is especially useful when one knows the pattern of FGF receptor expression in a particular tissue, so that specific cells within that tissue may be killed upon transcription of a cytotoxic agent gene without affecting the surrounding tissues. Similarly, promoters of genes that are coordinately expressed with other heparin-binding or glycosaminoglycan-binding genes may be used. The choice of the promoter will depend, at least in part, on the receptor-binding internalized ligand chosen.

Alternatively, cytocide gene products may be noncytotoxic but activate a compound, which is endogenously produced or exogenously applied, from a nontoxic form to a toxic product that inhibits protein synthesis.

The construct must contain the sequence that binds to the nucleic acid binding domain, if the domain binds in a sequence specific manner. As described below, the target nucleotide sequence may be contained within the coding region of the cytocide, in which case, no additional sequence need be incorporated. It may be desirable to have multiple copies of target sequence. If the target sequence is coding sequence, the additional copies must be located in non-coding regions of the cytocide-encoding agent. The target sequences of the nucleic acid binding domains are typically generally known. The target sequence may be readily determined, in any case. Techniques are generally available for establishing the target sequence *(e.g., see* PCT Application WO 92/05285 and U.S. Serial No. 586,769).

Specificity of delivery is achieved by coupling a nucleic acid binding domain to a receptor-binding internalized ligand, either by chemical conjugation or by constructing a fusion protein. Linkers as described above may be used. The receptor-binding internalized ligand part confers specificity of delivery in a cell-specific manner. The choice of the receptor-binding internalized ligand to use will depend upon the receptor expressed by the target cells. The receptor type of the target cell population may be determined by conventional techniques such as antibody staining, PCR of cDNA using receptor-specific primers, and biochemical or functional receptor binding assays. It is preferable that the receptor be cell type specific or have increased expression or activity (*i.e.*, higher rate of internalization) within the target cell population.

The nucleic acid binding domain can be of two types, non-specific in its ability to bind nucleic acid, or highly specific so that the amino acid residues bind only the desired nucleic acid sequence. Nonspecific binding proteins, polypeptides, or compounds are generally polycations or highly basic. Lys and Arg are the most basic of the 20 common amino acids; proteins enriched for these residues are candidates for nucleic acid binding domains. Examples of basic proteins include histones, protamines, and repeating units of lysine and arginine. Poly-L-lysine is a well-used nucleic acid binding domain *(see* U.S. Patent Nos. 5,166,320 and 5,354,844). Other polycations, such as spermine and spermidine, may also be used to bind nucleic acids. By way of example, the sequence-specific proteins including Sp-1, AP-1, myoD and the *rev* gene product from HIV may be used. Specific nucleic acid binding domains can be cloned in tandem, individually, or multiply to a desired region of the receptor-binding internalized ligand of interest. Alternatively, the domains can be chemically conjugated to each other.

The corresponding response elements that bind sequence-specific domains are incorporated into the construct to be delivered. Complexing the cytocidal-encoding agent to the receptor-binding internalized ligand/nucleic acid binding domain allows specific binding of response element to the nucleic acid binding domain. Even greater specificity of binding may be achieved by identifying and using the minimal amino acid sequence that binds to the cytocidal-encoding agent of interest. For example, phage display methods can be used to identify amino acids residues of varying length that will bind to specific nucleic acid sequences with high affinity. *(See* U.S. Patent No. 5,223,409.) The peptide sequence can then be cloned into the receptor-binding internalized ligand as a single copy or multiple copies. Alternatively, the peptide may be chemically conjugated to the receptor-binding internalized ligand. Incubation of the cytocide-encoding agent with the conjugated proteins will result in a specific binding between the two.

These complexes may be used to deliver nucleic acids that encode saporin or other cytocidal proteins into cells that have appropriate receptors that are expressed, over-expressed or more active in internalization upon binding. The cytocide gene is cloned downstream of a mammalian promoter such as SV40, CMV, TK or Adenovirus promoter. As described above, promoters of interest may be active in any cell type, active only in a tissue-specific manner, such as α-crystalline or tyrosinase, event specific or inducible, such as the MMTV LTR.

Receptor-binding internalized ligands are prepared as discussed by any suitable method, including recombinant DNA technology, isolation from a suitable source, purchase from a commercial source, or chemical synthesis. The selected linker or linkers is (are) linked to the receptor-binding internalized ligands by chemical reaction, generally relying on an available thiol or amine group on the receptor-binding internalized ligands. Heterobifunctional linkers are particularly suited for chemical conjugation. Alternatively, if the linker is a peptide linker, then the receptor-binding internalized ligands, linker and nucleic acid binding domain can be expressed recombinantly as a fusion protein.

The heparin-binding moiety or glycosaminoglycan-binding moiety (*e.g.*, FGF, VEGF, HBEGF) may be isolated from a suitable source or may be produced using recombinant DNA methodology, discussed below. To effect chemical conjugation herein, the moiety (protein) is conjugated generally via a reactive amine group or thiol group to the nucleic acid binding domain directly or through a linker to the nucleic acid binding domain. The protein is conjugated either via its N-terminus, C-terminus, or elsewhere in the polypeptide. In preferred embodiments, the protein is conjugated via a reactive cysteine residue to the linker or to the nucleic acid binding domain. The protein can also be modified by addition of a cysteine residue, either by replacing a residue or by inserting the cysteine, at or near the amino or carboxyl terminus, within about 20, preferably 10 residues from either end, and preferably at or near the amino terminus.

The heparin-binding moiety is preferably linked via non-essential cysteine residues to the linkers or to the targeted agent. A protein that has been modified by introduction of a Cys residue at or near one terminus, preferably the N-terminus is preferred for use in chemical conjugation (see Examples). For use herein, when VEGF is used, preferably the VEGF is dimerized prior to linkage to the linker and/or targeted agent. Methods for coupling proteins to the linkers, such as the heterobifunctional agents, or to nucleic acids, or to proteins are known to those of skill in the art and are also described herein.

Methods for chemical conjugation of proteins are known to those of skill in the art. The preferred methods for chemical conjugation depend on the selected components, but preferably rely on disulfide bond formation. For example, if the targeted agent is SPDP-derivatized saporin, then it is advantageous to dimerize the VEGF moiety prior coupling or conjugating to the derivatized saporin. If VEGF is modified to include a cysteine residue at or near the N-, preferably, or C- terminus, then dimerization should follow coupling to the nucleic acid binding domain. To effect chemical conjugation herein, the VEGF polypeptide is linked via one or more selected linkers or directly to the nucleic acid binding domain. Other heparin-binding moieties do not need to dimerize for binding to their respective receptor.

A nucleic acid binding domain is prepared for chemical conjugation. For chemical conjugation, a nucleic acid binding domain may be derivatized with SPDP or other suitable chemicals. If the binding domain does not have a Cys residue available for reaction, one can be either inserted or substituted for another amino acid. If desired, mono-derivatized species may be isolated, essentially as described.

For chemical conjugation, the nucleic acid binding domain may be derivatized or modified such that it includes a cysteine residue for conjugation to the receptor-binding internalized ligand. Typically, derivatization proceeds by reaction with SPDP. This results in a heterogeneous population. For example, nucleic acid binding domain that is derivatized by SPDP to a level of 0.9 moles pyridine-disulfide per mole of nucleic acid binding domain includes a population of non-derivatized, mono-derivatized and di-derivatized SAP. Nucleic acid binding domain proteins, which are overly derivatized with SPDP, may lose ability to bind nucleic acid because of reaction with sensitive lysines (Lambert et al., *Cancer Treat. Res. 37*:175-209, 1988). The quantity of non-derivatized nucleic acid binding domain in the preparation of the non-purified material can be difficult to judge and this may lead to errors in being able to estimate the correct proportion of derivatized nucleic acid binding domain to add to the reaction mixture.

Because of the removal of a negative charge by the reaction of SPDP with lysine, the three species, however, have a charge difference. The methods herein rely on this charge difference for purification of mono-derivatized nucleic acid binding domain by Mono-S cation exchange chromatography. The use of purified mono-derivatized nucleic acid binding domain has distinct advantages over the non-purified material. The amount of receptor-binding internalized ligand that can react with nucleic acid binding domain is limited to one molecule with the mono-derivatized material, and it is seen in the results presented herein that a more homogeneous conjugate is produced. There may still be sources of heterogeneity with the mono-derivatized nucleic acid binding domain used here but is acceptable as long as binding to the cytocide-encoding agent is not impacted.

Because more than one amino group on the nucleic acid binding domain may react with the succinimidyl moiety, it is possible that more than one amino group on the surface of the protein is reactive. This creates potential for heterogeneity in the mono-derivatized nucleic acid binding domain. As an alternative to derivatizing to introduce a sulfhydryl, the nucleic acid binding domain can be modified by the introduction of a cysteine residue. Preferred loci for introduction of a cysteine residue include the N-terminus region, preferably within about one to twenty residues from the N-terminus of the nucleic acid binding domain. Using either methodology (reacting mono-derivatized nucleic acid binding domain introducing a Cys residue into nucleic acid binding domain), the resulting preparations of chemical conjugates are monogenous; compositions containing the conjugates also appear to be free of aggregates. As a preferred alternative, heterogeneity can be avoided by producing a fusion protein of receptor-binding internalized ligand and nucleic acid binding domain, as described below.

Expression of DNA encoding a fusion of a receptor-binding internalized ligand polypeptide linked to the nucleic acid binding domain results in a more homogeneous preparation of cytotoxic conjugates. Aggregate formation can be reduced in preparations containing the fusion proteins by modifying the receptor-binding internalized ligand, such as by removal of nonessential cysteines, and/or the nucleic acid binding domain to prevent interactions between conjugates via free cysteines.

DNA encoding the polypeptides may be isolated, synthesized or obtained from commercial sources or prepared as described herein. Expression of recombinant polypeptides may be performed as described herein; and DNA encoding these polypeptides may be used as the starting materials for the methods herein. DNA may be prepared synthetically based on the amino acid or DNA sequence or may be isolated using methods known to those of skill in the art, such as PCR, probe hybridization of libraries, and the like or obtained from commercial or other sources.

The DNA construct encoding the fusion protein can be inserted into a plasmid and expressed in a selected host, as described above, to produce a recombinant receptor-binding internalized ligand―nucleic acid binding domain conjugate. Multiple copies of the chimera can be inserted into a single plasmid in operative linkage with one promoter. When expressed, the resulting protein will then be a multimer. Typically, two to six copies of the chimera are inserted, preferably in a head to tail fashion, into one plasmid.

To produce monogenous preparations of fusion protein, DNA is modified so that, upon expression, the resulting heparin-binding moiety portion of the fusion protein does not include any cysteines available for reaction. In preferred embodiments, DNA encoding an FGF polypeptide is linked to DNA encoding protamine or poly-L-lysine. The DNA encoding the FGF polypeptide or other receptor-binding internalized ligand is modified in order to remove the translation stop codon and other transcriptional or translational stop signals that may be present and to remove or replace DNA encoding the available cysteines. The DNA is then ligated to the DNA encoding the nucleic acid binding domain polypeptide directly or via a linker region of one or more codons between the first codon of the nucleic acid binding domain and the last codon of the FGF. The size of the linker region may be any length as long as the resulting conjugate binds and is internalized by a target cell. Presently, spacer regions of from about one to about seventy-five to ninety codons are preferred. The order of the receptor-binding internalized ligand and nucleic acid binding domain in the fusion protein may be reversed. If the nucleic acid binding domain is N-terminal, then it is modified to remove the stop codon and any stop signals.

If the FGF or other ligand has been modified so as to lack mitogenic activity or other biological activities, binding and internalization may still be readily assayed by any one of the following tests or other equivalent tests. Generally, these tests involve labeling the ligand, incubating it with target cells, and visualizing or measuring intracellular label. For example, briefly, FGF may be fluorescently labeled with FITC or radiolabeled with ¹²⁵I. Fluorescein-conjugated FGF is incubated with cells and examined microscopically by fluorescence microscopy or confocal microscopy for internalization. When FGF is labeled with ¹²⁵I, the labeled FGF is incubated with cells at 4°C. Cells are temperature shifted to 37°C and washed with 2 M NaCl at low pH to remove any cell-bound FGF. Label is then counted and thereby measuring internalization of FGF. Alternatively, the ligand can be conjugated with an nucleic acid binding domain by any of the methods described herein and complexed with a plasmid encoding saporin. As discussed below, the complex may be used to transfect cells and cytotoxicity measured.

The DNA encoding the resulting receptor-binding internalized ligand nucleic acid binding domain can be inserted into a plasmid and expressed in a selected host, as described above, to produce a monogenous preparation.

Multiple copies of the modified receptor-binding internalized ligand/nucleic acid binding domain chimera can be inserted into a single plasmid in operative linkage with one promoter. When expressed, the resulting protein will be a multimer. Typically two to six copies of the chimera are inserted, preferably in a head to tail fashion, into one plasmid. Merely by way of example, DNA encoding human bFGF- has been mutagenized using splicing by overlap extension (SOE). Each application of the SOE method uses two amplified oligonucleotide products, which have complementary ends as primers and which include an altered codon at the locus at which the mutation is desired, to produce a hybrid product. A second amplification reaction that uses two primers that anneal at the non-overlapping ends amplify the hybrid to produce DNA that has the desired alteration.

The receptor-binding internalized ligand/nucleic acid binding domain is incubated with the cytocide-encoding agent, typically a DNA molecule, to be delivered under conditions that allow binding of the nucleic acid binding domain to the agent. Conditions will vary somewhat depending on the nature of the nucleic acid binding domain, but will typically occur in 0.1M NaCI and 20 mM HEPES or other similar buffer.

The desired application is the delivery of cytotocidal agents, such as saporin, in a nontoxic form. By delivering a nucleic acid molecule capable of expressing saporin, the timing of cytotoxicity may be exquisitely controlled. For example, if saporin is expressed under the control of a tissue-specific promoter, then uptake of the complex by cells having the tissue-specific factors necessary for promoter activation will result in the killing of those cells. On the other hand, if cells taking up the complex do not have those tissue-specific factors, the cells will be spared.

Merely by way of example, test constructs have been made and tested. One construct is a chemical conjugate of FGF2 and poly-L-lysine. The FGF2 molecule is a variant in which the Cys residue at position 96 has been changed to a serine; thus, only the Cys at position 78 is available for conjugation. This FGF2 is called FGF2-3. The poly-L-lysine, which was average length of 84 residues, was derivatized with SPDP and coupled to FGF2-3. This FGF2-3/poly-L-lysine conjugate (FGF2-K84) was used to deliver a plasmid able to express the β-galactosidase gene. In addition, FGF2-K84 was complexed with a plasmid able to express saporin, and transfected into COS cells. The complex transferred cytotoxicity.

The ability of a construct to bind nucleic acid molecules may be conveniently assessed by agarose gel electrophoresis. Briefly, a plasmid, such as pSVβ, is digested with restriction enzymes to yield a variety of fragment sizes. For ease of detection, the fragments may be labeled with ³²P either by filling in of the ends with DNA polymerase I or by phosphorylation of the 5'-end with polynucleotide kinase following dephosphorylation by alkaline phosphatase. The plasmid fragments are then incubated with the receptor-binding internalized ligand/nucleic acid binding domain in this case, FGF2-3/poly-L-lysine in a buffered saline solution, such as 20 mM HEPES, pH 7.3, 0.1M NaCl. The reaction mixture is electrophoresed on an agarose gel alongside similarly digested, but nonreacted fragments. If a radioactive label was incorporated, the gel may be dried and autoradiographed. If no radioactive label is present, the gel may be stained with ethidium bromide and the DNA visualized through appropriate red filters after excitation with UV. Binding has occurred if the mobility of the fragments is retarded compared to the control. In the example case, the mobility of the fragments was retarded after binding with the FGF2-3/poly-L-lysine conjugate.

Further testing of the conjugate is performed to show that it binds to the cell surface receptor and is internalized into the cell. It is not necessary that the receptor-binding internalized ligand part of the conjugate retain complete biological activity. For example, FGF is mitogenic on certain cell types. As discussed above, this activity may not always be desirable. If this activity is present, a proliferation assay is performed. Likewise, for each desirable activity, an appropriate assay may be performed. However, for application of the subject invention, the only criteria that need be met are receptor binding and internalization.

Receptor binding and internalization may be measured by the following three assays. (1) A competitive inhibition assay of the complex to cells expressing the appropriate receptor demonstrates receptor binding. (2) Receptor binding and internalization may be assayed by measuring β-gal expression (*e.g.*, enzymatic activity) in cells that have been transformed with a complex of a β-gal containing plasmid condensed with a receptor-binding internalized ligand/nucleic acid binding domain. This assay is particularly useful for optimizing conditions to give maximal transformation. Thus, the optimum ratio of receptor-binding internalized ligand/nucleic acid binding domain to nucleic acid and the amount of DNA per cell may readily be determined by assaying and comparing the enzymatic activity of β-gal. As such, these first two assays are useful for preliminary analysis and failure to show receptor binding or β-gal activity does not per se eliminate a candidate receptor-binding internalized ligand/nucleic acid binding domain conjugate or fusion protein from further analysis. (3) The preferred assay is a cytotoxicity assay performed on cells transformed with a cytocide-encoding agent bound by receptor-binding internalized ligand/nucleic acid binding domain. While, in general, any cytocidal molecule may be used, ribosome-inactivating proteins are preferred and saporin, or another type I ribosome-inactivating protein, is particularly preferred. A statistically significant reduction in cell number demonstrates the ability of the receptor-binding internalized ligand/nucleic acid binding domain conjugate or fusion to deliver nucleic acids into a cell.

### D. Other elements

### 1. Nuclear translocation signals

A "nuclear translocation sequence" or "nuclear targeting sequence" (NTS) is a sequence of amino acids in a protein that are required for translocation of the protein into a cell nucleus. Examples of NTS sequence are set forth in Table 2 below. Comparison with known NTSs, and if necessary testing of candidate sequences, should permit those of skill in the art to readily identify other amino acid sequences that function as NTSs. A heterologous NTS is an NTS that differs from the NTS occurring in the wild-type peptide, polypeptide, or protein. For example, the NTS may be derived from another polypeptide, synthesized, or derived from another region in the same polypeptide.

**TABLE 2**

| Source | Sequence * | SEQ ID NO. |
|---|---|---|
| SV40 large T | Pro¹²⁶LysLysArgLysValGlu | 19 |
| Polyoma large T | Pro²⁷⁹ ProLysLysAlaArgGluVal | 20 |
| Human c-myc | Pro¹²⁰ AlaAlaLysArgValLysLeuAsp | 21 |
| Adenovirus E1A | Lys²⁸¹ ArgProArgPro | 22 |
| Yeast mat α₂ | Lys³ IleProIleLys | 23 |
| c-erb-A | A. Gly²² LysArgLysArgLysSer | 24 |
| | B. Ser¹²⁷LysArgValAlaLysArgLysleu | 25 |
| | C. Ser¹⁸¹ HisTrpLysGlnLysArgLysPhe | 26 |
| c-myb | Pro⁵²¹ LeuLeuLysLysIleLysGln | 27 |
| p53 | Pro³¹⁶GlnProLysLysLysPro | 28 |
| Nucleolin | Pro²⁷⁷GlyLysArgLysLysGluMetThrLysGlnLysGluValPro | 29 |
| HIV tat | Gly⁴⁸ArgLysLysArgArgGlnArgArgArgAlaPro | 30 |
| FGF-1 | AsnTyrLysLysProLysLeu | 31 |
| FGF-2 | HisPheLysAspProLysArg | 32 |
| FGF-3 | AlaProArgArgArgLysLeu | 33 |
| FGF-4 | IleLysArgLeuArgArg | 34 |
| FGF-5 | GlyArgArg | --- |
| FGF-6 | IleLysArgGlnArgArg | 35 |
| FGF-7 | IleArgValArgArg | 36 |

| | | |
|---|---|---|
| * Superscript indicates position in protein | | |

When a nucleic acid is the cytotoxic agent, the nuclear translocation sequence (NTS) may be a heterologous sequence. All presently identified members of the FGF family of peptides contain an NTS *(see, e.g*., PCT Application WO 91/15229 and Table 2). A typical consensus NTS sequence contains an amino-terminal proline or glycine followed by at least three basic residues in a array of seven to nine amino acids *(see, e.g.* Dang et al., *J. Biol*. *Chem.* 264:18019-18023, 1989; Dang et al., *Mol*. *Cell. Biol.* 8:4049-4058, 1988, and Table 2, which sets forth examples of NTSs and regions of proteins that share homology with known NTSs).

### 2. Linkers

A "linker" is an N-terminal extension that links the receptor-binding internalized ligand or fragment thereof and the nucleic acid binding domain or links a heparin-binding or glycosaminoglycan-binding moiety and a cytotoxic agent. The linkers provided herein confer specificity, enhance intracellular availability, serum stability, solubility on the conjugate, and may alternatively or additionally, serve to condense nucleic acids.

The linkers provided herein confer specificity on the cytotoxic conjugate by, for example, conferring specificity for certain proteases, particularly proteases that are present in only certain subcellular compartments or that are present at higher levels in tumor cells than normal cells. A linker susceptible to cleavage by protease present in a lysosome or endosome and not present in serum is preferable. The linkers may also include sorting signals that direct the conjugate to particular intracellular loci or compartments. The linkers may also serve as spacers to reduce steric hindrance between the growth factor and other protein or linked nucleic acid.

In order to increase the serum stability, solubility and/or intracellular concentration of the targeted agent, one or more linkers (are) inserted between the receptor-binding internalized ligand and the nucleic acid binding domain. These linkers include peptide linkers, such as intracellular protease substrates, and chemical linkers, such as acid labile linkers, ribozyme substrate linkers and others. Peptides linkers may be inserted using heterobifunctional reagents, described below, or, preferably, are linked to FGF, other growth factors, including heparin-binding growth factors, or cytokines by linking DNA encoding the ligand of heparin-binding moiety to the DNA encoding the nucleic acid binding domain or cytotoxic agent.

Chemical linkers may be inserted by covalently coupling the linker to the FGF, other growth factor protein, or cytokine and the nucleic acid binding domain or cytotoxic agent. The heterobifunctional agents, described below, may be used to effect such covalent coupling.

### a. Protease substrates

Peptides encoding protease-specific substrates are introduced between the heparin-binding moiety and the nucleic acid binding domain or cytotoxic agent. The peptides may be inserted using heterobifunctional reagents, described below, or, preferably, inserted by recombinant means and expressing the resulting chimera.

Any protease specific substrate *(see, e.g.*, O'Hare et al., *FEBS 273*:200-204, 1990; Forsberg et al., *J. Protein Chem. 10:517-526,* 1991; Westby et al., *Bioconjugate Chem. 3*:375-381, 1992) may be introduced as a linker as long as the substrate is cleaved in an intracellular compartment. Preferred substrates include those that are specific for proteases that are expressed at higher levels in tumor cells or that are preferentially expressed in the endosome and not expressed in serum. The following substrates are among those contemplated for use in accord with the methods herein: cathepsin B substrate, cathepsin D substrate, trypsin substrate, thrombin substrate, and recombinant subtilisin substrate.

### b. Flexible linkers and linkers that increase the solubility of the conjugates

Flexible linkers and linkers that increase solubility of the conjugates are contemplated for use, either alone or with other linkers, such as the protease specific substrate linkers. Such linkers include, but are not limited to, (Gly₄Ser)ₙ, (Ser₄Gly)ₙ and (AlaAlaProAla)ₙ in which n is 1 to 6, preferably 1-4, such as:
a. Gly₄Ser SEQ ID NO: 37 CCATGGGCGG CGGCGGCTCT GCCATGG
b. (Gly₄Ser)₂ SEQ ID NO: 38 CCATGGGCGG CGGCGGCTCT GGCGGCGGCG GCTCTGCCAT GG
c. (Ser₄Gly)₄ SEQ ID NO: 39 
CCATGGCCTC GTCGTCGTCG GGCTCGTCGT CGTCGGGCTC GTCGTCGTCG GGCTCGTCGT
CGTCGGGCGC CATGG
d. (Ser₄Gly)₂ SEQ ID NO: 40 CCATGGCCTC GTCGTCGTCG GGCTCGTCGT CGTCGGGCGC CATGG
e. (AlaAlaProAla)ₙ, where n is 1 to 4, preferably 2 *(see,* SEQ ID NO: 41)

### c. Heterobifunctional cross-linking reagents

Numerous heterobifunctional cross-linking reagents that are used to form covalent bonds between amino groups and thiol groups and to introduce thiol groups into proteins, are known to those of skill in this art (*see, e.g.,* the Pierce Catalog, ImmunoTechnology Catalog & Handbook, 1992-1993, which describes the preparation of and use of such reagents and provides a commercial source for such reagents; *see, also, e.g*., Cumber et al., *Bioconjugate Chem. 3*:397-401, 1992; Thorpe et al., *Cancer Res. 47*:5924-5931, 1987; Gordon et al., *Proc. Natl*. *Acad Sci. 84*:308-312, 1987; Walden et al., *J*. *Mol. Cell Immunol. 2*:191-197, 1986; Carlsson et al., *Biochem. J. 173:723-737,* 1978; Mahan et al., *Anal. Biochem. 162*:163-170, 1987; Wawryznaczak et al., *Br. J. Cancer 66*:361-366, 1992; Fattom et al., *Infection & Immun. 60*:584-589, 1992). These reagents may be used to form covalent bonds between the receptor-binding internalized ligands with protease substrate peptide linkers and nucleic acid binding domain. These reagents include, but are not limited to: N-succinimidyl-3-(2-pyridyldithio)propionate (SPDP; disulfide linker); sulfosuccinimidyl 6-[3-(2-pyridyldithio)propionamido]hexanoate (sulfo-LC-SPDP); succinimidyloxycarbonyl-α-methyl benzyl thiosulfate (SMBT, hindered disulfate linker); succinimidyl 6-[3-(2-pyridyldithio) propionamido]hexanoate (LC-SPDP); sulfosuccinimidyl 4-(N-maleimidomethyl)cyclohexane-1-carboxylate (sulfo-SMCC); succinimidyl 3-(2-pyridyldithio)butyrate (SPDB; hindered disulfide bond linker); sulfosuccinimidyl 2-(7-azido-4-methylcoumarin-3-acetamide) ethyl-1,3'-dithiopropionate (SAED); sulfosuccinimidyl 7-azido-4-methylcoumarin-3-acetate (SAMCA); sulfosuccinimidyl 6-[alpha-methyl-alpha-(2-pyridyldithio)toluamido]hexanoate (sulfo-LC-SMPT); 1,4-di-[3'-(2'-pyridyldithio)propionamido]butane (DPDPB); 4-succinimidyloxycarbonyl-α-methyl-α-(2-pyridylthio)toluene (SMPT, hindered disulfate linker); sulfosuccinimidyl6[α-methyl-α-(2-pyridyldithio)toluamido]hexanoate (sulfo-LC-SMPT); m-maleimidobenzoyl-N-hydroxysuccinimide ester (MBS); m-maleimidobenzoyl-N-hydroxysulfosuccinimide ester (sulfo-MBS); N-succinimidyl(4-iodoacetyl)aminobenzoate (SIAB; thioether linker); sulfosuccinimidyl-(4-iodoacetyl)amino benzoate (sulfo-SIAB); succinimidyl4(*p*-maleimidophenyl)butyrate (SMPB); sulfosuccinimidyl4-(*p*-maleimidophenyl)butyrate (sulfo-SMPB); azidobenzoyl hydrazide (ABH).

These linkers should be particularly useful when used in combination with peptide linkers, such as those that increase flexibility.

### d. Acid cleavable linkers, photocleavable and heat sensitive linkers

Acid cleavable linkers include, but are not limited to, bismaleimideothoxy propane, adipic acid dihydrazide linkers *(see, e.g.,* Fattom et al., *Infection & Immun. 60*:584-589, 1992), and acid labile transferrin conjugates that contain a sufficient portion of transferrin to permit entry into the intracellular transferrin cycling pathway *(see, e.g.,* Welhöner et al., *J*. *Biol. Chem. 266*:4309-4314, 1991). Conjugates linked via acid cleavable linkers are cleaved in acidic intracellular compartments, such as the endosome.

Photocleavable linkers are linkers that are cleaved upon exposure to light *(see, e.g.*, Goldmacher et al., *Bioconj. Chem. 3*:104-107, 1992, which linkers are herein incorporated by reference), thereby releasing the targeted agent upon exposure to light *(see, e.g.,* Hazum et al., *Pept.*, *Proc. Eur. Pept. Symp.*, *16th,* Brunfeldt, K (Ed), pp. 105-110, 1981, which describes the use of a nitrobenzyl group as a photocleavable protective group for cysteine; Yen et al., *Makromol. Chem 190*:69-82, 1989, which describes water soluble photocleavable copolymers, including hydroxypropylmethacrylamide copolymer, glycine copolymer, fluorescein copolymer and methylrhodamine copolymer; Goldmacher et al., *Bioconj. Chem. 3*:104-107, 1992, which describes a cross-linker and reagent therefor that undergoes photolytic degradation upon exposure to near UV light (350 nm); and Senter et al., *Photochem. Photobiol 42*:231-237, 1985, which describes nitrobenzyloxycarbonyl chloride cross linking reagents that produce photocleavable linkages). Such linkers have particular use in treating dermatological or ophthalmic conditions and in other tissues, such as blood vessels during angioplasty in the prevention or treatment of restenosis, that can be exposed to light using fiber optics. After administration of the conjugate, the eye or skin or other body part is exposed to light, resulting in release of the targeted moiety from the conjugate. This should permit administration of higher dosages of such conjugates compared to conjugates that release a cytotoxic agent upon internalization. Heat sensitive linkers would also have similar applicability.

### 2. Cytoplasm-translocation signal

Cytoplasm-translocation signal sequence is a sequence of amino acids in a protein that cause retention of proteins in the lumen of the endoplasmic reticulum and/or translocate proteins to the cytosol. The signal sequence in mammalian cells is KDEL (Lys-Asp-Glu-Leu) (SEQ ID NO. 42) (Munro and Pelham, *Cell 48*:899-907, 1987). Some modifications of this sequence have been made without loss of activity. For example, the sequences RDEL (Arg-Asp-Glu-Leu) (SEQ ID NO. 43) and KEEL (Lys-Glu-Glu-Leu) (SEQ ID NO. 44) confer efficient or partial retention, respectively, in plants (Denecke et al., *Embo. J. 11*:2345-2355, 1992).

A cytoplasm-translocation signal sequence may be included in the heparin-binding or glycosaminoglycan-binding moiety or a nucleic acid binding domain. If cleavable linkers are used to link the moiety with the nucleic acid binding domain, the cytoplasm-translocation signal is preferably included in the nucleic acid binding domain, which will stay bound to the cytocide-encoding agent. Additionally, a cytoplasmic-translocation signal sequence may be included in the receptor-internalized binding ligand, as long as it does not interfere with receptor binding. Similarly, the signal sequence placed in the nucleic acid binding domain should not interfere with binding to the cytocide-encoding agent.

### 3. Endosome-disruptive peptides

In addition, or alternatively, membrane-disruptive peptides may be included in the conjugates. For example, adenoviruses are known to enhance disruption of endosomes. Virus-free viral proteins, such as influenza virus hemaglutinin HA-2, also disrupt endosomes and are useful in the present invention. Other proteins may be tested in the assays described herein to find specific endosome disrupting agents that enhance gene delivery. In general, these proteins and peptides are amphipathic *(see* Wagner et al., *Adv. Drug. Del. Rev. 14*:113-135, 1994).

Endosome-disruptive peptides, sometimes called fusogenic peptides, may be incorporated into the conjugates. Two such peptides derived from influenza virus are: GLFEAIEGFIENGWEGMIDGGGC (SEQ. ID NO. 45) and GLFEAIEGFIENGWEGMIDGWYGC (SEQ. ID NO. 46). Other peptides useful for disrupting endosomes may be identified by general characteristics: 25-30 residues in length, contain an alternating pattern of hydrophobic domains and acidic domains, and at low pH (*e.g.*, pH 5) from amphipathic α-helices. A candidate endosome-disrupting peptide is tested by incorporating it into the conjugate and determining whether it increases the total number of cells expressing the target gene. The endosome-disruptive peptide may be present as single or multiple copies at the N- or C- terminus of the moiety. A single fusion protein of the endosome-disruptive peptide, moiety, and cytotoxic agent may be constructed and expressed. For insertion into a construct, DNA encoding the endosome-disruptive peptide may be synthesized by PCR using overlapping oligonucleotides and incorporating a restriction site at the 5' and 3' end to facilitate cloning. The sequence may be verified by sequence analysis.

### E. Expression vector and host cells for expression of heparin-binding growth factors

Generally, DNA encoding the desired heparin-binding growth factor, polypeptide targeted agent or conjugate is inserted into a suitable vector and expressed in a suitable prokaryotic or eukaryotic host. Numerous suitable hosts and vectors are known and available to those of skill in this art.

Host organisms include those organisms in which recombinant production of heterologous proteins have been carried out, such as, but not limited to, bacteria (for example, *E. coli),* yeast (for example, *Saccharomyces cerevisiae* and *Pichia pastoris*), mammalian cells, and insect cells. Presently preferred host organisms are strains of bacteria, especially *E. coli*.

The DNA construct is introduced into a plasmid for expression in a desired host. In preferred embodiments, the host is a bacterial host. The sequences of nucleotides in the plasmids that are regulatory regions, such as promoters and operators, are operationally associated with one another for transcription. The sequence of nucleotides encoding the growth factor or growth factor-chimera may also include DNA encoding a secretion signal, whereby the resulting peptide is a precursor protein. The resulting processed protein may be recovered from the periplasmic space or the fermentation medium.

In preferred embodiments the DNA plasmids also include a transcription terminator sequence. The promoter regions and transcription terminators are each independently selected from the same or different genes.

The plasmids used herein preferably include a promoter in operable association with the DNA encoding the protein or polypeptide of interest and are designed for expression of proteins in a bacterial host. It has been found that tightly regulatable promoters are preferred for expression of saporin. Suitable promoters for expression of proteins and polypeptides herein are widely available and are well known in the art. Inducible promoters or constitutive promoters that are linked to regulatory regions are preferred. Such promoters include, but are not limited to, the T7 phage promoter and other T7-like phage promoters, such as the T3, T5 and SP6 promoters, the trp, lpp, and lac promoters, such as the lacUV5, from *E. coli;* the P10 or polyhedron gene promoter of baculovirus/insect cell expression systems *(see, e.g.,* U.S. Patent Nos. 5,243,041, 5,242,687, 5,266,317, 4,745,051, and 5,169,784) and inducible promoters from other eukaryotic expression systems. For expression of the proteins such promoters are inserted in a plasmid in operative linkage with a control region such as the lac operon.

Preferred promoter regions are those that are inducible and functional in *E. coli.* Examples of suitable inducible promoters and promoter regions include, but are not limited to: the *E. coli* lac operator responsive to isopropyl β -D-thiogalactopyranoside (IPTG; *see,* et al. Nakamura et al., *Cell 18*:1109-1117, 1979); the metallothionein promoter metal-regulatory-elements responsive to heavy-metal (*e*.*g.*, zinc) induction *(see, e.g.,* U.S. Patent No. 4,870,009 to Evans et al.); the phage T71ac promoter responsive to IPTG *(see, e.g.,* U.S. Patent No. 4,952,496; and Studier et al., *Meth. Enzymol. 185*:60-89, 1990) and the Tac promoter.

The plasmids also preferably include a selectable marker gene or genes that are functional in the host. A selectable marker gene includes any gene that confers a phenotype on the host cell that allows transformed cells to be identified and selectively grown from among a vast majority of untransformed cells. Suitable selectable marker genes for bacterial hosts, for example, include the ampicillin resistance gene (Amp^{r}), tetracycline resistance gene (Tc^{r}) and the kanamycin resistance gene (Kan^{r}). The kanamycin resistance gene is presently preferred.

The plasmids may also include DNA encoding a signal for secretion of the operably linked protein. Secretion signals suitable for use are widely available and are well known in the art. Prokaryotic and eukaryotic secretion signals functional in *E. coli may* be employed. The presently preferred secretion signals include, but are not limited to, those encoded by the following *E. coli* genes: ompA, ompT, ompF, ompC, beta-lactamase, and alkaline phosphatase, and the like (von Heijne, *J. Mol. Biol. 184*:99-105, 1985). In addition, the bacterial pelB gene secretion signal (Lei et al., *J*. *Bacteriol. 169*:4379*,* 1987), the phoA secretion signal, and the cek2 signal, which is functional in insect cells may be employed. The most preferred secretion signal is the *E. coli* ompA secretion signal. Other prokaryotic and eukaryotic secretion signals known to those of skill in the art may also be employed *(see, e.g.,* von Heijne, *J. Mol. Biol*. *184*:99-105, 1985). Using the methods described herein, one of skill in the art can substitute secretion signals that are functional in either yeast, insect or mammalian cells to secrete proteins from those cells.

Preferred plasmids for transformation of *E. coli* cells include the pET expression vectors *(see,* U.S patent 4,952,496; available from Novagen, Madison, WI). Such plasmids include pET 11 a, which contains the T71ac promoter, T7 terminator, the inducible *E*. *coli* lac operator, and the lac repressor gene; pET 12a-c, which contains the T7 promoter, T7 terminator, and the *E. coli* ompT secretion signal; and pET 15b, which contains a His-Tag™ leader sequence (SEQ ID NO. 18) for use in purification with a His column and a thrombin cleavage site that permits cleavage following purification over the column; the T7-lac promoter region and the T7 terminator.

Other preferred plasmids include the pKK plasmids, particularly pKK 223-3, which contains the tac promoter (available from Pharmacia; *see, also,* Brosius et al., *Proc.. Natl. Acad. Sci. 81*:6929, 1984; Ausubel et al., *Current Protocols in Molecular Biology;* U.S. Patent Nos. 5,122,463, 5,173,403, 5,187,153, 5,204,254, 5,212,058, 5,212,286, 5,215,907, 5,220,013, 5,223,483, and 5,229,279). Plasmid pKK has been modified by replacement of the ampicillin resistance marker gene, with a kanamycin resistance cassette (purchased from Pharmacia; obtained from pUC4K, *see, e.g.*, Vieira et al., *Gene 19*:259-268, 1982; and U.S. Patent No. 4,719,179).

Baculovirus vectors, such as a pBlueBac (also called pJVETL and derivatives thereof) vector, particularly pBlueBac III, *(see, e.g.,* U.S. Patent Nos. 5,278,050, 5,244,805, 5,243,041, 5,242,687, 5,266,317, 4,745,051, and 5,169,784; available from Invitrogen, San Diego) may also be used for expression of the polypeptides in insect cells. The pBlueBacIII vector is a dual promoter vector and provides for the selection of recombinants by blue/white screening as this plasmid contains the β-galactosidase gene (lacZ) under the control of the insect recognizable ETL promoter and is inducible with IPTG. A DNA construct is constructed a baculovirus vector pBluebac III (Invitrogen, San Diego, CA) and then co-transfected with wild type virus into insect cells *Spodoptera frugiperda* (sf9 cells; *see, e.g.,* Luckow et al., *Bio/technology* 6:47-55, 1988, and U.S. Patent No. 4,745,051).

Other plasmids include the pIN-IIIompA plasmids *(see,* U.S. Patent No. 4,575,013 to Inouye; *see, also,* Duffaud et al., *Meth. Enz. 153*:492-507, 1987), such as pIN-IIIompA2. The pIN-IIIompA plasmids include an insertion site for heterologous DNA linked in transcriptional reading frame with four functional fragments derived from the lipoprotein gene of *E. coli.* The plasmids also include a DNA fragment coding for the signal peptide of the ompA protein of *E. coli,* positioned such that the desired polypeptide is expressed with the ompA signal peptide at its amino terminus, thereby allowing efficient secretion across the cytoplasmic membrane. The plasmids further include DNA encoding a specific segment of the *E. coli* lac promoter-operator, which is positioned in the proper orientation for transcriptional expression of the desired polypeptide, as well as a separate functional *E. coli* lacI gene encoding the associated repressor molecule that, in the absence of lac operon inducer, interacts with the lac promoter-operator to prevent transcription therefrom. Expression of the desired polypeptide is under the control of the lipoprotein (lpp) promoter and the lac promoter-operator, although transcription from either promoter is normally blocked by the repressor molecule. The repressor is selectively inactivated by means of an inducer molecule thereby inducing transcriptional expression of the desired polypeptide from both promoters.

A particularly preferred vector for expressing VEGF and other heparin-binding growth factors is pPL-λ. This vector contains the tightly regulated leftward promoter of bacteriophage λ, which is controlled by the cI repressor. The promoter is temperature-inducible by using a bacterial host strain, such as N4830-1, which contains the temperature-sensitive cI857 repressor.

In a preferred embodiment, the DNA fragment is replicated in bacterial cells, preferably in *E. coli.* The preferred DNA fragment also includes a bacterial origin of replication, to ensure the maintenance of the DNA fragment from generation to generation of the bacteria. In this way, large quantities of the DNA fragment can be produced by replication in bacteria. Preferred bacterial origins of replication include, but are not limited to, the f1-ori and col El origins of replication. Preferred hosts contain chromosomal copies of DNA encoding T7 RNA polymerase operably linked to an inducible promoter, such as the lacUV promoter *(see,* U.S. Patent No. 4,952,496). Such hosts include, but are not limited to, lysogens *E. coli* strains HMS174(DE3)pLysS, BL21(DE3)pLysS, HMS174(DE3) and BL21(DE3). Strain BL21(DE3) is preferred. The pLys strains provide low levels of T7 lysozyme, a natural inhibitor of T7 RNA polymerase.

The DNA fragments may also contain a gene coding for a repressor protein. The repressor protein is capable of repressing the transcription of a promoter that contains sequences of nucleotides to which the repressor protein binds. The promoter can be derepressed by altering the physiological conditions of the cell. The alteration can be accomplished by the addition to the growth medium of a molecule that inhibits, for example, the ability to interact with the operator or with regulatory proteins or other regions of the DNA or by altering the temperature of the growth media. Preferred repressor proteins include, but are not limited to the *E. coli* lacI repressor responsive to IPTG induction, the temperature sensitive cI857 repressor, and the like. The cI857 repressor is particularly preferred.

DNA encoding full-length bFGF or the bFGF muteins has been linked to DNA encoding the mature saporin protein and introduced into the pET vectors, including pET-11a and pET-12a expression vectors, for intracellular and periplasmic expression, respectively, of FGF-SAP fusion proteins. The resulting fusion proteins exhibit cytotoxic activity and appear to be at least as potent as the chemically conjugated FGF-SAP preparations.

### F. Methods of preparation of heparin-binding moiety-cytotoxic agent conjugates

Cytotoxic conjugates with linked targeted agents can be prepared either by chemical conjugation, recombinant DNA technology, or combinations of recombinant expression and chemical conjugation. The methods herein are described with particular reference to FGF2 and saporin. It is understood, however, that the same methods may be used prepare and use conjugates of any member of the FGF family with saporin, modified saporin, a nucleic acid or any other targeted agent via linkers as described herein.

### 1. Chemical conjugation

To effect chemical conjugation herein, the FGF protein is linked via one ore more selected linkers or directly to the targeted agent. Chemical conjugation must be used if the targeted agent is other than a peptide or protein, such a nucleic acid or a non-peptide drug.

### a. Preparation of heparin-binding moieties

Heparin-binding moieties are prepared as discussed by any suitable method, including recombinant DNA technology, isolation from a suitable source, purchase from a commercial source, or chemical synthesis. The selected linker or linkers is (are) linked to the receptor-binding internalized ligands by chemical reaction, generally relying on an available thiol or amine group on the receptor-binding internalized ligands. Heterobifunctional linkers are particularly suited for chemical conjugation. Alternatively, if the linker is a peptide linker, then the heparin-binding moiety, linker and cytotoxic agent can be expressed recombinantly as a fusion protein.

Any protein that binds and internalizes through a receptor interaction may be used herein. For the chemical conjugation methods, the protein may be produced recombinantly, produced synthetically, or obtained from commercial or other sources. For the preparation of fusion proteins, the DNA encoding the FGF may be obtained from any known source or synthesized according to its DNA or amino acid sequences (*see* discussion above). Although any of the moieties may be conjugated in this manner, FGF, VEGF, and HBEGF conjugation are discussed merely by way of example and not by way of limitation.

The FGF protein is prepared by any suitable method, including recombinant DNA technology, isolation from a suitable source, purchase from a commercial source, or chemical synthesis. The selected linker or linkers is (are) linked to the FGF protein by chemical combination, generally relying on an available thiol or amine group on the FGF. Heterobifunctional linkers are particularly suited for chemical conjugation. Alternatively, if the linker is a peptide linker, then the FGF and linker can be expressed recombinantly as a fusion protein. If the targeted agent is a protein or peptide and the linker is a peptide, then the entire conjugate can be expressed as a fusion protein.

Any protein that is reactive with an FGF receptor may be used herein. In particular, any member of the FGF family of peptides or portion thereof that binds to an FGF receptor and internalizes a linked agent may be used herein. For the chemical conjugation methods the protein may be produced recombinantly, produced synthetically or obtained from commercial or other sources. For the preparation of fusion proteins, the DNA encoding the FGF may be obtained from any known source or synthesized according to its DNA or amino acid sequences (*see*, *e.g.,* SEQ ID NOS. 3-11).

In addition, any of the proteins may be modified as described herein in order to reduce the heterogeneity the resulting preparations of cytotoxic conjugates. Thus, heterogeneity of preparations of FGF protein-containing chemical conjugates and fusion proteins can be reduced by modifying the FGF protein by deleting or replacing a site(s) on the FGF that cause the heterogeneity and/or by modifying the targeted agent. Such sites in FGF are typically cysteine residues that, upon folding of the protein, remain available for interaction with other cysteines or for interaction with more than one cytotoxic molecule per molecule of FGF peptide. Thus, such cysteine residues do not include any cysteine residue that are required for proper folding of the FGF peptide or for retention of the ability to bind to an FGF receptor and internalize. For chemical conjugation, one cysteine residue that, in physiological conditions, is available for interaction, is not replaced because it is used as the site for linking the cytotoxic moiety. The resulting modified FGF is conjugated with a single species of targeted agent.

The polypeptide reactive with an FGF receptor may be modified by removing one or more reactive cysteines that are not required for receptor binding, but that are available for reaction with appropriately derivatized cytotoxic agent, so that the resulting FGF protein has only one cysteine residue available for conjugation with the cytotoxic agent. If necessary, the contribution of each cysteine to the ability to bind to FGF receptors may be determined empirically. Each cysteine residue may be systematically replaced with a conservative amino acid change (see Table 1, above) or deleted. The resulting mutein is tested for the requisite biological activity, the ability to bind to FGF receptors and internalize linked cytotoxic moieties. If the mutein retains this activity, then the cysteine residue is not required. Additional cysteines are systematically deleted and replaced and the resulting muteins are tested for activity. In this manner the minimum number and identity of the cysteines needed to retain the ability to bind to an FGF receptor and internalize may be determined. The resulting mutant FGF is then tested for retention of the ability to target a cytotoxic agent to a cell that expresses an FGF receptor and to internalize the cytotoxic agent into such cells. Retention of proliferative activity is indicative, though not definitive, of the retention of such activities. Proliferative activity may be measured by any suitable proliferation assay, such as the assay, exemplified below, that measures the increase in cell number of adrenal capillary endothelial cells.

It is noted, however, that modified or mutant FGFs may exhibit reduced or no proliferative activity, but may be suitable for use herein, if they retain the ability to target a linked targeted agent to cells bearing receptors to which the unmodified FGF binds and result in internalization of the targeted moiety.

Any of FGF-1 - FGF-9 may be so modified. The complete amino acid sequence of each of FGF-1 - FGF- 9 is known *(see, e.g.*, SEQ ID NO. 3 (FGF-1) and SEQ ID NOS. 5-11 (FGF-3 - FGF-9, respectively). The sequence is examined and cysteine residues are identified. Comparison among the amino acid sequences of FGF-1 -FGF-9 reveals that one Cys is conserved among FGF family of peptides (see Table 3). These cysteine residues may be required for secondary structure and should not be altered. Each of the remaining cysteine residues may be systematically deleted and/or replaced by a serine residue or other residue that would not be expected to alter the structure of the protein. The resulting peptide is tested for biological activity. If the cysteine residue is necessary for retention of biological activity it is not deleted; if it not necessary, then it is preferably replaced with a serine or other residue that should not alter the secondary structure of the resulting protein.

The cysteine residues from each of FGF-1 - FGF-9 that appear to be essential for retention of biological activity and that should not be deleted or replaced are as follows:

**TABLE 3**

| | |
|---|---|
| FGF-1 | cys⁹⁸ |
| FGF-2 | cys¹⁰¹ |
| FGF-3 | cys¹¹⁵ |
| FGF-4 | cys¹⁵⁵ |
| FGF-5 | cys¹⁶⁰ |
| FGF-6 | cys¹⁴⁷ |
| FGF-7 | cys¹³⁷ |
| FGF-8 | cys¹²⁷ |
| FGF-9 | cys¹³⁴ |

For example, FGF-1 has cysteines at positions 31, 98 and 132; FGF-2 has cysteines at positions 34, 78, 96 and 101; FGF-3 has cysteines at positions 50 and 115; FGF-4 has cysteines at positions 88 and 155; FGF-5 has cysteines at positions 19, 93, 160 and 202; FGF-6 has cysteines at positions 80 and 147; FGF-7 has cysteines at positions 18, 23, 32, 46, 71, 133 and 137; FGF-8 has cysteines at positions 10, 19, 109 and 127; and FGF-9 has cysteines at positions 68 and 134.

Since FGF-3, FGF-4 and FGF-6 have only two cysteines, for purposes of chemical conjugation, preferably neither cysteine is deleted or replaced, unless another residue, preferably one near either terminus, is replaced with a cysteine. With respect to the other FGF family members, at least one cysteine must remain available for conjugation with the cytotoxic conjugate and probably two cysteines, but at least the cysteine residues set forth in Table 3. A second cysteine may be required to form a disulfide bond. Thus, any FGF peptide that has more than three cysteines is be modified for chemical conjugation by deleting or replacing the other cysteine residues. FGF peptides that have three cysteine residues are modified by elimination of one cysteine, conjugated to a cytotoxic moiety and tested for the ability to bind to FGF receptors and internalize the cytotoxic moiety.

In accord with the methods herein, several muteins of basic FGF for chemical conjugation have been produced (preparation of muteins for recombinant expression of the conjugate is described below). DNA, obtained from pFC80 *(see,* PCT Application PCT/US93/05702; United States Application Serial No. 07/901,718; *see, also,* SEQ ID NO. 1) encoding basic FGF has been mutagenized. Mutagenesis of cysteine 78 of basic FGF to serine ([C78S]FGF) or cysteine 96 to serine ([C96S]FGF) produced two mutants that retain virtually complete proliferative activity of native basic FGF as judged by the ability to stimulate endothelial cell proliferation in culture. The activities of the two mutants and the native protein do not significantly differ as assessed by efficacy or maximal response. Sequence analysis of the modified DNA verified that each of the mutants has one codon for cysteine converted to that for serine. The construction and biological activity of FGF-1 with cysteine substitutions of one, two or all three cysteines has been disclosed (U.S. Patent No. 5,223,483). The mitogenic activity of the mutants was similar to or increased over the native protein. Thus, any of the cysteines may be mutated and FGF-2 will still bind and internalize.

The resulting mutein FGF or unmodified FGF is reacted with a single species of cytotoxic agent to produced. The bFGF muteins have been reacted with a single species of derivatized saporin (mono-derivatized saporin) thereby resulting in monogenous preparations of FGF-SAP conjugates and homogeneous compositions of FGF-SAP chemical conjugates. The resulting chemical conjugate does not aggregate and retains the requisite biological activities.

Briefly, VEGF or HBEGF may be isolated from a suitable source or may be produced using recombinant DNA methodology, discussed below. To effect chemical conjugation herein, the heparin-binding protein is conjugated generally via a reactive amine group or thiol group to the nucleic acid binding domain directly or through a linker to the nucleic acid binding domain. The protein is conjugated either via its N-terminus, C-terminus, or elsewhere in the polypeptide. In preferred embodiments, the protein is conjugated via a reactive cysteine residue to the linker or to the nucleic acid binding domain. The protein can also be modified by addition of a cysteine residue, either by replacing a residue or by inserting the cysteine, at or near the amino or carboxyl terminus, within about 20, preferably 10 residues from either end, and preferably at or near the amino terminus.

The contribution of each cysteine to the ability to bind to VEGF, HBEGF or other heparin-binding or glycosaminoglycan-binding moieties receptors may be determined empirically as described above. In the case of VEGF, VEGF₁₂₁ contains 9 cysteines and each of VEGF₁₆₅, VEGF₁₈₉ and VEGF₂₀₆ contain 7 additional residues in the region not present in VEGF₁₂₁. Any of the 7 are likely to be non-essential for targeting and internalization of linked cytotoxic agents. Recently, the role of Cys-25, Cys-56, Cys-67, Cys-101, and Cys-145 in dimerization and biological activity was assessed (Claffery et al., *Biochem. Biophys. Acta 1246:1-9,* 1995). Dimerization requires Cys-25, Cys-56, and Cys-67. Substitution of any one of these cysteine residues resulted in secretion of a monomeric VEGF, which was inactive in both vascular permeability and endothelial cell mitotic assays. In contrast, substitution of Cys 145 had no effect on dimerization, although biological activities were somewhat reduced. Substitution of Cys-101 did not result in the production of a secreted or cytoplasmic protein. Thus, substitution of Cys-145 is preferred.

The VEGF monomers are preferably linked via non-essential cysteine residues to the linkers or to the targeted agent. VEGF that has been modified by introduction of a Cys residue at or near one terminus, preferably the N-terminus is preferred for use in chemical conjugation. For use herein, preferably the VEGF is dimerized prior to linkage to the linker and/or targeted agent. Methods for coupling proteins to the linkers, such as the heterobifunctional agents, or to nucleic acids, or to proteins are known to those of skill in the art and are also described herein.

For recombinant expression using the methods described herein, up to all cysteines in the HBEGF polypeptide that are not required for biological activity can be deleted or replaced. Alternatively, for use in the chemical conjugation methods herein, all except one of these cysteines, which will be used for chemical conjugation to the cytotoxic agent, can be deleted or replaced. Each of the HBEGF polypeptides described herein have six cysteine residues. Each of the six cysteines may independently be replaced and the resulting mutein tested for the ability to bind to HBEGF receptors and to be internalized. Alternatively, the resulting mutein-encoding DNA is used as part of a construct containing DNA encoding the nucleic acid binding domain linked to the HBEGF-encoding DNA. The construct is expressed in a suitable host cell and the resulting protein tested for the ability to bind to HBEGF receptors and internalize. As long as this ability is retained the mutein is suitable for use herein.

Methods for chemical conjugation of proteins are known to those of skill in the art. The preferred methods for chemical conjugation depend on the selected components, but preferably rely on disulfide bond formation. For example, if the targeted agent is SPDP-derivatized saporin, then it is advantageous to dimerize the VEGF moiety prior coupling or conjugating to the derivatized saporin. If VEGF is modified to include a cysteine residue at or near the N-, preferably, or C- terminus, then dimerization should follow coupling to the nucleic acid binding domain. To effect chemical conjugation herein, the HBEGF polypeptide is linked via one or more selected linkers or directly to the nucleic acid binding domain.

### b. Preparation of cytotoxic agent

Cytotoxic agents are prepared for chemical conjugation preferably by derivatization with SPDP. Merely by way of example, preparation of saporin is discussed, although these methods are generally applicable to other agents.

Saporin for chemical conjugation may be produced by isolating the protein from the leaves or seeds of *Saponaria officinalis (see, e.g.*, Example 1) or using recombinant methods and the DNA provided herein or known to those of skill in the art or obtained by screening appropriate libraries (see Examples below and discussion herein).

For chemical conjugation, the SAP may be derivatized or modified such that it includes a cysteine residue for conjugation to the FGF protein. Typically, SAP is derivatized by reaction with SPDP. This results in a heterogeneous population. For example, SAP that is derivatized by SPDP to a level of 0.9 moles pyridine-disulfide per mole of SAP includes a population of non-derivatized, mono-derivatized and di-derivatized SAP. Ribosome-inactivating proteins, which are overly derivatized with SPDP, may lose activity because of reaction with sensitive lysines (Lambert et al., *Cancer Treat. Res. 37*:175-209, 1988). The quantity of non-derivatized SAP in the preparation of the non-purified material can be difficult to judge and this may lead to errors in being able to estimate the correct proportion of derivatized SAP to add to the reaction mixture.

Because of the removal of a negative charge by the reaction of SPDP with lysine, the three species, however, have a charge difference. The methods herein rely on this charge difference for purification of mono-derivatized SAP by Mono-S cation exchange chromatography. The use of purified mono-derivatized SAP has distinct advantages over the non-purified material. The amount of basic FGF that can react with SAP is limited to one molecule with the mono-derivatized material, and it is seen in the results presented herein that a more homogeneous conjugate is produced. There are still sources of heterogeneity with the mono-derivatized SAP used here. Native SAP as purified from the seed itself is a mixture of four isoforms, as judged by protein sequencing *(see, e.g.,* PCT Application PCT/US93/05702, and United States Application Serial No. 07/901,718; *see, also,* Montecucchi et al., *Int. J. Pept. Prot. Res*. *33:263-267,* 1989; Maras et al., *Biochem. Internat. 21:631-638,* 1990; and Barra et al., *Biotechnol. Appl. Biochem. 13*:48-53, 1991). This creates some heterogeneity in the conjugates, since the reaction with SPDP probably occurs equally each isoform. This source of heterogeneity can be addressed by use of SAP expressed in *E. coli.*

DNA provided herein includes a sequence of nucleotides encoding a saporin polypeptide or a modified saporin polypeptide and may include an N-terminal extension sequence linked to the amino terminus of the saporin that encodes a linker so that, if desired, the SAP and linker can be expressed as a fusion protein. The DNA molecules provided herein encode saporin that has substantially the same amino acid sequence and ribosome-inactivating activity as that of saporin-6 (SO-6), including any of four isoforms, which have heterogeneity at amino acid positions 48 and 91 *(see, e.g.,* Maras et al., *Biochem. Internat. 21*:631-638, 1990, and Barra et al., *Biotechnol*. *Appl. Biochem*. *13*:48-53, 1991 and SEQ ID NOS. 47-51). Other suitable saporin polypeptides include other members of the multi-gene family coding for isoforms of saporin-type RIP's including SO-1 and SO-3 (Fordham-Skelton et al., *Mol. Gen. Genet. 221*:134-138, 1990), SO-2 *(see, e.g.,* U.S. Application Serial No. 07/885,242, which corresponds to GB 2,216,891; *see, also*, Fordham-Skelton et al., *Mol. Gen*. *Genet.* 229:460-466, 1991), SO-4 *(see, e.g.,* GB 2,194,241 B; *see, also*, Lappi et al., *Biochem. Biophys*. *Res*. *Commun. 129*:934-942, 1985) and SO-5 *(see, e.g.,* GB 2,194,241 B; *see, also,* Montecucchi et al., *Int. J*. *Peptide Protein Res. 33:263-267,* 1989). SO-4, which includes the N-terminal 40 amino acids set forth in SEQ ID NO. 52, is isolated from the leaves of *Saponaria officinalis* by extraction with 0.1 M phosphate buffer at pH 7, followed by dialysis of the supernatant against sodium borate buffer, pH 9, and selective elution from a negatively charged ion exchange resin, such as Mono S (Pharmacia Fine Chemicals, Sweden) using gradient of 1 to 0.3 M. NaCl and first eluting chromatographic fraction that has SAP activity. The second eluting fraction is SO-5.

The saporin polypeptides exemplified herein include those having substantially the same amino acid sequence as those listed in SEQ ID NOS. 47-51. The isolation and expression of the DNA encoding these proteins is described in Example 1.

Because more than one amino group on SAP may react with the succinimidyl moiety, it is possible that more than one amino group on the surface of the protein is reactive. This creates the potential for heterogeneity in the mono-derivatized SAP. This source of heterogeneity has been solved by the conjugating modified SAP expressed in *E. coli* that has an additional cysteine inserted, as described above, in the coding sequence.

As discussed above, muteins of saporin that contain a Cys at or near the amino or carboxyl terminus can be prepared. Thus, instead of derivatizing saporin to introduce a sulfhydryl, the saporin can be modified by the introduction of a cysteine residue into the SAP such that the resulting modified saporin protein reacts with the FGF protein to produce a monogenous cytotoxic conjugate and the conjugate binds to FGF receptors on eukaryotic cells is cytotoxic upon internalization by such cells. Preferred loci for introduction of a cysteine residue include the N-terminus region, preferably within about one to twenty residues from the N-terminus of the cytotoxic agent, such as SAP. For expression of SAP in the bacterial host systems herein, it is also desirable to add DNA encoding a methionine linked to the DNA encoding the N-terminus of the saporin protein. DNA encoding SAP has been modified by inserting a DNA encoding Met-Cys (ATG TGT or ATG TGC) at the N-terminus immediately adjacent to the codon for first residue of the mature protein. Muteins have also been prepared in which the amino acid at position 4 or 10 has been replaced with cysteine. The modified DNA may be expressed and the resulting saporin protein purified, as described herein for expression and purification of the resulting SAP. The modified saporin is then reacted with the modified FGF to form disulfide linkages between the single exposed cysteine residue on the FGF and the cysteine residue on the modified SAP.

Using either methodology (reacting mono-derivatized SAP the FGF peptide or introducing a Cys residue into SAP), the resulting preparations of FGF-SAP chemical conjugates are monogenous; compositions containing the conjugates also appear to be free of aggregates. The above-described causes of heterogeneity may also be avoided by producing the cytotoxic conjugate as a fusion protein, in which DNA encoding the modified FGF protein is linked to DNA encoding the cytotoxic agent, as described below.

### 2. Fusion protein of a heparin-binding polypeptide and cytotoxic agent

Expression of DNA encoding a fusion of a heparin-binding protein linked to the targeted agent results in a monogenous preparation of cytotoxic conjugates and is suitable for use, when the selected targeting agent and linker are polypeptides. Preparations containing the fusion proteins may be rendered more homogeneous by modifying the heparin-binding protein and/or the targeted agent to prevent interactions between each conjugate, such as via unreacted cysteines.

For example, aggregate formation has been eliminated or substantially reduced by preparing mutein constructs in which the cysteine residues on the FGF are deleted or replaced *(see,* discussion above). Conjugates containing FGF-2 in which the cysteines at positions 78 and 96 residues have been replaced by serines have been prepared. The resulting preparations of cytotoxic conjugates retain cytotoxic activity, are monogenous and are free of aggregates.

### a. Preparation of muteins

Removal of cysteines not required for binding and internalization is preferred for both chemical conjugation and recombinant methods in the chemical conjugation methods, all except one cysteine, which is necessary for chemical conjugation are deleted or replaced. In practice, it appears that for FGF polypeptides only two cysteines (including each of the cysteine residues set forth in Table 3), and perhaps only the cysteines set forth in Table 3, are required for retention of the requisite biological activity of the FGF peptide. Thus, FGF peptides that have more than two cysteines are modified by replacing the remaining cysteines with serines. The resulting muteins may be tested for the requisite biological activity.

FGF peptides, such as FGF-3, FGF-4 and FGF-6, that have two cysteines can be modified by replacing the second cysteine, which is not listed in Table 3, and the resulting mutein used as part of a construct containing DNA encoding the cytotoxic agent linked to the FGF-encoding DNA. The construct is expressed in a suitable host cell and the resulting protein tested for the ability to bind to FGF receptors and internalize the cytotoxic agent.

As exemplified below, conjugates containing FGF-2 muteins in which Cys⁷⁸ and Cys⁹⁶ have been replaced with serine residues have been prepared. The resulting conjugates are at least as active as recombinant conjugates that have wild type FGF components and at least as active as chemical conjugates of FGF. In addition, it appears that the recombinantly produced conjugates are less toxic, and thus, can, if necessary, be administered in higher dosages.

### b. DNA constructs and expression of the DNA constructs

To produce monogenous preparations of cytotoxic conjugates using recombinant means, the DNA encoding the heparin-binding protein is modified so that, upon expression, it does not include any cysteines available for reaction. In preferred embodiments, DNA encoding an FGF polypeptide is linked to DNA encoding a saporin polypeptide. The DNA encoding the FGF polypeptide is modified in order to remove the translation stop codon and other transcriptional or translational stop signals that may be present and to remove or replace DNA encoding the available cysteines. The DNA is then ligated to the DNA encoding the saporin polypeptide directly or via a linker region of one or more codons between the first codon of the saporin and the last codon of the FGF. The size of the linker region is any length as long as the resulting conjugate exhibits cytotoxic activity upon internalization by a target cell. Presently, spacer regions of from about one to about seventy-five to ninety codons are preferred. The order of the heparin-binding moiety and cytotoxic agent in the fusion protein may be reversed. If the cytotoxic agent is N-terminal, then it is modified to remove the stop codon and any stop signals.

As discussed above, any heparin-binding protein, including FGF, VEGF, HBEGF, may be modified and expressed in accord with the methods herein. Modification includes the construction of a chimeric molecule between the heparin binding domain of one of the heparin-binding protein and a ligand for a cell surface receptor.

If FGF or other heparin-binding protein lacks mitogenic activity or other biological activities, binding and internalization may still be readily assayed by any one of the following tests or other equivalent tests. Generally, these tests involve labeling the ligand, incubating it with target cells, and visualizing or measuring intracellular label. For example, briefly, FGF may be fluorescently labeled with FITC or radiolabeled with ¹²⁵I. Fluorescein-conjugated FGF is incubated with cells and examined microscopically by fluorescence microscopy or confocal microscopy for internalization. When FGF is labeled with ¹²⁵I, the labeled FGF is incubated with cells at 4°C. Cells are temperature shifted to 37°C and washed with 2 M NaCl at low pH to remove any cell-bound FGF. Label is then counted and thereby measuring internalization of FGF. For chimeras, binding and internalization is assayed by an appropriate assay for the particular ligand in the chimera. Other heparin-binding or glycosaminoglycan-binding moieties may be tested for receptor-binding in a similar manner or by other receptor assays used in the art.

Multiple copies of the modified FGF-SAP chimera or modified heparin-binding protein-cytotoxic agent chimera can be inserted into a single plasmid in operative linkage with one promoter. When expressed, the resulting protein will be a multimer. Typically two to six copies of the chimera are inserted into one plasmid, preferably in a head to tail fashion.

Merely by way of an example, DNA encoding human FGF-2-SAP having SEQ ID NO. 2 has been mutagenized as described in the Examples using splicing by overlap extension (SOE). Another preferred coding region is set forth in SEQ ID NO. 2, nucleotides 1 - 465. In both instances, in preferred embodiments, the DNA is modified by replacing the cysteines at positions 78 and 96 with serine. The codons encoding cysteine residues at positions 78 and 96 of FGF in the FGF-SAP encoding DNA (SEQ ID NO. 2) were converted to serine codons by SOE. Each application of the SOE method uses two amplified oligonucleotide products, which have complementary ends as primers and which include an altered codon at the locus at which the mutation is desired, to produce a hybrid product. A second amplification reaction that uses two primers that anneal at the non-overlapping ends amplify the hybrid to produce DNA that has the desired alteration.

### G. Binding of the conjugate to medical device

As noted above, the present invention provides intravenous medical devices coated with heparin, the heparin being bound with heparin-binding moiety-cytotoxic agent conjugates. The medical devices useful in the present invention include any device implantable into a patient. The following devices are especially of interest: stents, tubing, needles, catheters, and other intravenous devices. The devices may be made of any material that can be coated with heparin. Heparin-coated devices and methods for coating devices with heparin are readily available. For example, U.S. Patent No. 5,342,621 describes biomedical polymers, including stents, that are coated with an antithrombogenic material, such as heparin. Similarly U.S. Patent 5,322,659, U.S. Patent 5,061,738, U.S. Patent 5,344,455, U.S. Patent 4,895,566, U.S. Patent 5,061,750, and U.S. Patent 5,380,299 disclose various medical devices coated with antithrombogenic materials, typically heparin. These and still other patents describe methods and compositions for coating devices with heparin *(see also*, U.S. Patent 5,013,717, U.S. Patent 5,322,659; and PCT application WO 93/05825). Within the context of this application, coating a device includes adhering heparin to the surface or impregnation of heparin into the device.

Any of the medical devices coated with heparin may be bound with a heparin-binding moiety conjugate. Briefly, a medical device coated with heparin is incubated under sterile conditions with a conjugate at preferably 0.25 mg/ml for 30 min at room temperature with agitation in 10 mM NaCitrate, pH 6, 140 mM NaCl, 0.1 mM EDTA buffer. The devices are washed three times in the same buffer and are ready for use. The extent of binding to the device may be measured. For example, trace amounts of labeled conjugate may be added during binding. ¹²⁵I-labeled conjugate is conveniently used, but any quantifiable label, such as other radiolabels or fluorescent labels, may also be used. The specific activity is used to calculate the amount of bound protein and bound protein is expressed as a function of the surface area of the device. A preferred quantity of bound protein is from 3 to 5 µg/cm². Alternatively, after binding, the conjugate is stripped under appropriate conditions, such as high salt, and the amount of conjugate measured by standard protein assay.

An intravenous device coated with heparin and bound with a heparin-binding growth factor-cytotoxic agent conjugate is tested for biological activity. Assays include *in vitro* and *in vivo* assays. The choice of an assay will depend in part on the cell type targeted, the cytotoxic agent, and the purpose of the device. For example, a stent coated with heparin and bound with an FGF-SAP conjugate is assayed for inhibition of proliferation *in vitro* and for prevention or diminishment of restenosis *in vivo*. Briefly, inhibition of proliferation *in vitro* is assayed by incubating the device with target cells and measuring proliferation. When a heparinized stent is bound with FGF-SAP, porcine aortic smooth muscle cells (SMC) may be used as targets. The SMC are seeded at a subconfluent density in 24 well tissue culture plates. At 48 hr later, a heparinized stent or heparinized stent bound with FGF-SAP is added. Five days later, cells are fixed and stained with methylene blue. An *in vivo* assay for restenosis is implantation of a stent following balloon denudation in the rabbit or other animal. In this assay, drug coated stents are implanted into the iliac arteries via the femoral arteries. Stents are deployed using balloon expansion, or alternatively are self-expanding. Evidence for decreased smooth muscle hyperplasia can be assessed at multiple time points over two months, using standard histologic and morphometric techniques. Larger animals, such as dog, pig, sheep, or baboon can also be studied. Stents can be deployed either into the coronary of peripheral circulatory systems of these larger animals. Peripheral vessels include carotid, iliac, brachial, and femoral arteries.

In certain aspects of the present invention, the heparin-binding moiety cytotoxic or cytocide-encoding agent is incorporated directly onto the device, in particular, a synthetic graft. Briefly, synthetic grafts made of expanded polytetrafluoroethylene (PTFE) or Dacron are impregnated with the conjugate. Local application of the conjugate through use of the graft will prevent or diminish stenosis at the graft anastamoses. Biodegradable polymers may be used to deliver FGF-SAP into the graft, using pressure-based delivery to deliver drug and carrier into the pores and interstices of the graft. The drug will be slowly released from the impregnated graft following implantation, resulting in high local levels of drug in areas of anastomoses prone to stenotic lesions. The drug may be combined with heparin to protect it from degradation. Biodegradable polymers include naturally occurring substances such as hyaluronic acid, other glycosaminoglycans, fibrin glue, collagen, phosphorycholine, and synthetic substances such as glycol and lactic acids, and protein chimeras (Diana). Such grafts could be used to decrease smooth muscle hyperplasia at graft anastomoses and within grafts in arterio-venous fistulas and in arterial bypass surgeries. When a biodegradable polymer is used, the conjugate will be slowly released. As an alternative to impregnating the graft, heparin or a glycosaminoglycan may be coated onto the graft and a heparin-binding or glycosaminoglycan binding protein conjugate bound thereto using methods described herein.

### H. Therapeutic uses

Cytotoxic agents are effective at inhibiting proliferation of cells when delivered intracellularly by internalization of ligand/cell surface receptor complex. The cytotoxic agents described herein are useful for inhibiting proliferation, and thus ameliorating symptoms or lessening the severity of various diseases and syndromes involving excessive or unwanted proliferation. Delivery of the conjugates bound to a medical device as described herein allow lower dosages than if the conjugates were administered systemically. In addition to higher specificity of delivery, undesirable side effects from large doses may be avoided or lessened.

As discussed herein, many interventional cardiology procedures for atherosclerosis, for example, result in restenosis, which is caused at least, in part, by excessive proliferation of smooth muscle cells lining the vessel. Implantation of stents fairly effectively prevents the recoiling of a vessel in these procedures, but some restenosis still results. Stents are used after angioplasty, AV shunts, intraluminal vascular grafts, or endoarterectomy, wherever blood vessels narrowed or occluded by disease are repaired.

In addition, implantation of a medical device allows for intralesional delivery into tumors or other localized areas of excessive or unwanted proliferation. Tumors, such as bladder carcinoma, breast carcinoma, and liver tumors are candidate tumors for therapy as described herein. The conjugates may also be bound to heparinized adhesion prevention barriers, such as disclosed in U.S. Patent No. 4,840,626, which are used to prevent adhesions forming between areas subject to surgical procedures and neighboring tissue.

The following examples are offered by way of illustration, and not by way of limitation.

### EXAMPLES

### EXAMPLE 1

### ISOLATION OF DNA ENCODING SAPORIN

### A. Materials and methods

### 1. Bacterial Strains

*E. coli* strain JA221 (lpp- hdsM+ trpE5 leuB6 lacY recAl F'[lacI^{q} lac⁺ pro⁺]) is publicly available from the American Type Culture Collection (ATCC), Rockville, MD 20852, under the accession number ATCC 33875. (JA221 is also available from the Northern Regional Research Center (NRRL), Agricultural Research Service, U.S. Department of Agriculture, Peoria, IL 61604, under the accession number NRRL B-15211; *see also* U.S. Patent No. 4,757,013 to Inouye; and Nakamura et al., *Cell 18*:1109-1117, 1979). Strain INV1α is commercially available from Invitrogen, San Diego, CA.

### 2. Methods

Native saporin and rabbit polyclonal antiserum to saporin were obtained as previously described in Lappi et al., *Biochem. Biophys. Res. Comm. 129*:934-942. Ricin A chain is commercially available from Sigma, Milwaukee, WI. Antiserum was linked to Affi-gel 10 (Bio-Rad, Emeryville, CA) according to the manufacturer's instructions. Sequencing was performed using the Sequenase kit of United States Biochemical Corporation (version 2.0) according to the manufacturer's instructions. Minipreparation and maxipreparation of plasmids, preparation of competent cells, transformation, M13 manipulation, bacterial media, Western blotting, and ELISA assays were according to Sambrook et al., (*Molecular Cloning: A Laboratory Manual,* Cold Spring Harbor Laboratory Press, Cold Spring Harbor, NY, 1989). The purification of DNA fragments was done using the Geneclean II kit (Bio 101) according to the manufacturer's instructions. SDS gel electrophoresis was performed on a Phastsystem (Pharmacia).

Western blotting was accomplished by transfer of the electrophoresed protein to nitrocellulose using the PhastTransfer system, as described by the manufacturer. The antiserum to SAP was used at a dilution of 1:1000. Horseradish peroxidase labeled anti-IgG was used as the second antibody (*see* Davis et al., *Basic Methods In Molecular Biology,* New York, Elsevier Science Publishing Co., pp 1-338, 1986).

### B. Isolation of DNA encoding saporin

### 1. Isolation of genomic DNA and preparation of polymerase chain reaction (PCR) primers

*Saponaria officinalis* leaf genomic DNA was prepared as described in Bianchi et al., *Plant Mol. Biol. 11*:203-214, 1988. Primers for genomic DNA amplifications were synthesized in a 380B automatic DNA synthesizer. The primer corresponding to the "sense" strand of saporin 5'-CTGCAGAATTCGCATGGATCCTGCTTCAAT-3' (SEQ ID NO. 53) includes an *Eco*R I restriction site adapter immediately upstream of the DNA codon for amino acid -15 of the native saporin N-terminal leader sequence. The primer 5'-CTGCAGAATTCGCCTCGTTTGACTACTTTG-3' (SEQ ID NO. 54) corresponds to the "antisense" strand of saporin and complements the coding sequence of saporin starting from the last 5 nucleotides of the DNA encoding the carboxyl end of the mature peptide. Use of this primer introduced a translation stop codon and an *Eco*RI restriction site after the sequence encoding mature saporin.

### 2. Amplification of DNA encoding saporin

Unfractionated *Saponaria officinalis* leaf genomic DNA (1 µl) was mixed in a final volume of 100 µl containing 10 mM Tris-HCl (pH 8.3), 50 mM KCl, 0.01% gelatin, 2 mM MgCl₂, 0.2 mM dNTPs, 0.8 µg of each primer. Next, 2.5 U *Taq* DNA polymerase (Perkin Elmer Cetus) were added and the mixture was overlaid with 30 µl of mineral oil (Sigma). Incubations were done in a DNA Thermal Cycler (Ericomp). One cycle included a denaturation step (94°C for I min), an annealing step (60°C for 2 min), and an elongation step (72°C for 3 min). After 30 cycles, a 10 µl aliquot of each reaction was run on a 1.5% agarose gel to verify the structure of the amplified product.

The amplified DNA was digested with *Eco*RI and subcloned into *Eco*RI-restricted M13mp18 (New England Biolabs, Beverly, MA; *see also* Yanisch-Perron et al. (1985), "Improved M13 phage cloning vectors and host strains: Nucleotide sequences of the M13mp18 and pUC19 vectors", *Gene 33*:103). Single-stranded DNA from recombinant phages was sequenced using oligonucleotides based on internal points in the coding sequence of saporin *(see* Bennati et al., *Eur. J. Biochem. 183*:465-470, 1989). Nine of the M13mp18 derivatives were sequenced and compared. Of the nine sequenced clones, five had unique sequences, set forth as SEQ ID NOS. 47-51, respectively. The clones were designated M13mp18-G4, -G1, -G2, -G7, and -G9. Each of these clones contains all of the saporin coding sequence and 45 nucleotides of DNA encoding the native saporin N-terminal leader peptide.

Saporin DNA sequence was also cloned in the pET11a vector. Briefly, the DNA encoding SAP-6 was amplified by polymerase chain reaction (PCR) from the parental plasmid pZ1B1. The plasmid pZ1B1 contains the DNA sequence for human FGF-2 linked to SAP-6 by a two-amino-acid linker (Ala-Met). PZ1B1 also includes the T7 promoter, lac operator, ribosomal binding site, and T7 terminator present in the pET11a vector. For SAP-6 DNA amplification, the 5' primer 5'-CATATGTGTGTCACATCAATCACATTAGAT-3' (SEQ ID NO. 55) corresponding to the sense strand of SAP-6, incorporated a *Nde*I restriction enzyme site used for cloning. It also contained a Cys codon at position -I relative to the start site of the mature protein sequence. No leader sequence was included. The 3' primer 5'-CAGGTTTGGATCCTTTACGTT-3' (SEQ ID NO. 56) corresponding to the antisense strand of SAP-6 had a *Bam*HI site used for cloning. The amplified DNA was gel-purified and digested with *Nde*I and *Bam*HI. The digested SAP-6 DNA fragment was subcloned into the *Nde*I/*Bam*HI-digested pZ1B1. This digestion removed FGF-2 and the 5' portion of SAP-6 (up to nucleotide position 650) from the parental rFGF2-SAP vector (pZ1B1) and replaced this portion with a SAP-6 molecule containing a Cys at position -1 relative to the start site of the native mature SAP-6 protein. The resultant plasmid was designated as pZ50B. pZ50B was transformed into *E. coli* strain NovaBlue for restriction and sequencing analysis. The appropriate clone was then transformed into *E. coli* strain BL21(DE3) for expression and large-scale production.

### C. pOMPAG4 Plasmid Construction

M13 mp18-G4 was digested with *Eco*R I, and the resulting fragment was ligated into the *Eco*R I site of the vector pIN-IIIompA2 *(see, e.g*., *see,* U.S. Patent No. 4,575,013 to Inouye; and Duffaud et al., *Meth. Enz. 153*:492-507, 1987) using the methods described herein. The ligation was accomplished such that the DNA encoding saporin, including the N-terminal extension, was fused to the leader peptide segment of the bacterial ompA gene. The resulting plasmid pOMPAG4 contains the *lpp* promoter (Nakamura et al., *Cell 18*:1109-1117, 1987), the *E. coli* lac promoter operator sequence (*lac* O) and the *E. coli* ompA gene secretion signal in operative association with each other and with the saporin and native N-terminal leader-encoding DNA listed in SEQ ID NO. 47. The plasmid also includes the *E. coli* lac repressor gene (lac I).

The M13 mp18-G1, -G2, -G7, and -G9 clones, containing SEQ ID NOS. 48-51, respectively, are digested with *Eco*R I and ligated into *Eco*R I digested pIN-IIIompA2 as described for M13 mp18-G4 above in this example. The resulting plasmids, labeled pOMPAG1, pOMPAG2, pOMPAG7, pOMPA9, are screened, expressed, purified, and characterized as described for the plasmid pOMPAG4.

INV1α competent cells were transformed with pOMPAG4 and cultures containing the desired plasmid structure were grown further in order to obtain a large preparation of isolated pOMPAG4 plasmid using methods described herein.

### D. Saporin expression in E. coli

The pOMPAG4 transformed *E. coli* cells were grown under conditions in which the expression of the saporin-containing protein is repressed by the lac repressor until the end of the log phase of growth, at which time IPTG was added to induce expression of the saporin-encoding DNA.

To generate a large-batch culture of pOMPAG4 transformed *E. coli* cells, an overnight culture (approximately 16 hours growth) of JA221 *E. coli* cells transformed with the plasmid pOMPAG4 in LB broth (*see, e.g.,* Sambrook et al., *Molecular Cloning: A Laboratory Manual,* Cold Spring Harbor Laboratory Press, Cold Spring Harbor, NY, 1989) containing 125 mg/ml ampicillin was diluted 1:100 into a flask containing 750 ml LB broth with 125 mg/ml ampicillin. Cells were grown at logarithmic phase with shaking at 37°C until the optical density at 550 nm reached 0.9 measured in a spectrophotometer.

In the second step, saporin expression was induced by the addition of IPTG (Sigma) to a final concentration of 0.2 mM. Induced cultures were grown for 2 additional hours and then harvested by centrifugation (25 min., 6500 x g). The cell pellet was resuspended in ice cold 1.0 M TRIS, pH 9.0, 2 mM EDTA (10 ml were added to each gram of pellet). The resuspended material was kept on ice for 20-60 minutes and then centrifuged (20 min., 6500 x g) to separate the periplasmic fraction of *E. coli*, which corresponds to the supernatant, from the intracellular fraction corresponding to the pellet.

The *E. coli* cells containing C-SAP construct in pET1 1a were grown in a high-cell density fed-batch fermentation with the temperature and pH controlled at 30°C and 6.9, respectively. A glycerol stock (1 ml) was grown in 50 ml Luria broth until the *A*₆₀₀ reached 0.6 Inoculum (10 ml) was injected into a 7-1-Applikon (Foster City CA) fermentor containing 21 complex batch medium consisting of 5 g/l of glucose, 1.25 g/l each of yeast extract and tryptone (Difco Laboratories), 7 g/l of K₂HPO₄, 8 g/l of KH₂PO₄, 1.66 g/l of (NH₄)₂SO₄, 1 g/l of MgSO₄ • 7H₂O, 2 ml/l of a trace metal solution (74 g/l of trisodium citrate, 27 g/l of FeCl₃ • 6H₂O, 2.0 g/l of CoCl₂ • 6H₂O, 2.0 g/l of Na₂MoO₄ • 2H₂O, 1.9 g/l of CuSO₄ • 5H₂O, 1.6 g/l of MnCl₂ • 4H₂O, 1.4 g/l of ZnCl₂ • 4H₂O, 1.0 g/l of CaCl₂ • 2H₂O, 0.5 g/l of H₃BO₃). 2 ml/l of a vitamin solution (6 g/l of thiamin • HCI, 3.05 g/l of niacin, 2.7 g/l of pantothenic acid, 0.7 g/l of pyridoxine • HC1, 0.21 g/l of riboflavin, 0.03 g/l of biotin, 0.02 g/l of folic acid), and 100 mg/l of carbenicillin. The culture was grown for 12 h before initiating the continuous addition of a 40× solution of complex batch media lacking the phosphates and containing only 25 ml/l, each, of trace metal and vitamin solutions. The feed addition continued until the *A*₆₀₀ of the culture reached 85, at which time (approximately 9 h) the culture was induced with 0.1 mM isopropyl β-D-thiogalactopyranoside. During 4 h of post-induction incubation, the culture was fed with a solution containing 100 g/l of glucose, 100 g/l of yeast extract, and 200 g/l of tryptone. Finally, the cells were harvested by centrifugation (8000×*g*, 10 min) and frozen at -80°C until further processed.

The cell pellet (≈400 g wet mass) containing C-SAP was resuspended in 3 vol Buffer B (10 mM sodium phosphate pH 7.0, 5 mM EDTA, 5 mM EGTA, and 1 mM dithiothreitol). The suspension was passed through a microfluidizer three times at 124 Mpa on ice. The resultant lysate was diluted with NanoPure H₂O until conductivity fell below 2.7 mS/cm. All subsequent procedures were performed at room temperature.

The diluted lysate was loaded onto an expanded bed of Streamline SP cation-exchange resin (300 ml) equilibrated with buffer C (20 mM sodium phosphate pH 7.0, 1 mM EDTA) at 100 ml/min upwards flow. The resin was washed with buffer C until it appeared clear. The plunger was then lowered at 2 cm/min while washing continued at 70 ml/min. Upwards flow was stopped when the plunger was approximately 8 cm away from the bed and the plunger was allowed to move to within 0.5 cm of the packed bed. The resin was further washed at 70 ml/min downwards flow until A₂₈₀ reached baseline. Buffer C plus 0.25 M NaCl was then used to elute proteins containing C-SAP at the same flow rate.

The eluate was buffer exchanged into buffer D (50 mM sodium borate pH 8.5, 1 mM EDTA) using the Sartocon Mini crossflow filtration system with a 10000 NMolecular Massco module (Sartorius). The sample was then applied to a column of Source 15S (30 ml) equilibrated with buffer D. A 10-column-volume linear gradient of 0-0.3 M NaCl in buffer D was used to elute C-SAP at 30 ml/min.

### E. Purification of secreted recombinant saporin

### 1. Anti-SAP immuno-affinity purification

The periplasmic fraction from Example 1.D. was dialyzed against borate-buffered saline (BBS: 5 mM boric acid, 1.25 mM borax, 145 mM sodium chloride, pH 8.5). The dialysate was loaded onto an immunoaffinity column (0.5 x 2 cm) of anti-saporin antibodies, obtained as described in Lappi et al., *Biochem. Biophys. Res. Comm., 129*:934-942, 1985, bound to Affi-gel 10 and equilibrated in BBS at a flow rate of about 0.5 ml/min. The column was washed with BBS until the absorbance at 280 nm of the flow-through was reduced to baseline. Next the column containing the antibody bound saporin was eluted with 1.0 M acetic acid and 0.5 ml fractions were collected in tubes containing 0.3 ml of 2 M ammonium hydroxide, pH 10. The fractions were analyzed by ELISA *(see, e.g.*, Sambrook et al., *Molecular Cloning: A Laboratory Manual,* Cold Spring Harbor Laboratory Press, Cold Spring Harbor, NY, 1989). The peak fraction of the ELISA was analyzed by Western blotting and showed a single band having a slightly higher molecular weight than native saporin. The fractions that contained saporin protein, as determined by the ELISA, were then pooled for further purification.

### 2. Reversed phase high performance liquid chromatography purification

To further purify the saporin secreted into the periplasm, the pooled fractions from above were diluted 1:1 with 0.1% trifluoroacetic acid (TFA) in water and chromatographed in reverse phase high pressure liquid chromatography (HPLC) on a Vydac C4 column (Western Analytical) equilibrated in 20% acetonitrile, 0.1% TFA in water. The protein was eluted with a 20 minute gradient to 60% acetonitrile. The HPLC produced a single peak that was the only area of immunoreactivity with anti-SAP antiserum when analyzed by a western blot as described in Example 1.E.1. Samples were assayed by an ELISA.

Sequence analysis was performed by Edman degradation in a gas-phase sequenator (Applied Biosystems) *(see, e.g.,* Lappi et al., *Biochem. Biophys. Res. Comm. 129*:934-942, 1985). The results indicated that five polypeptides were obtained that differ in the length, between 7 and 12 amino acids, of the N-terminal saporin leader before the initial amino acid valine of the mature native saporin (SEQ ID NO. 47: residue -12 through -7). All of the N-terminal extended variants retained cytotoxic activity. The size of the native leader is 18 residues, indicating that the native signal peptide is not properly processed by bacterial processing enzymes. The ompA signal was, however, properly processed.

To obtain homogeneous saporin, the recombinantly produced saporin can be separated by size and one of the five polypeptides used to produce the conjugates.

### F. Purification of intracellular soluble saporin

To purify the cytosolic soluble saporin protein, the pellet from the intracellular fraction of Example I.E. above was resuspended in lysis buffer (30 mM TRIS, 2 mM EDTA, 0.1% Triton X-100, pH 8.0, with 1 mM PMSF, 10 µg/ml pepstatin A, 10 µg aprotinin, µg/ml leupeptin and 100 µg/ml lysozyme, 3.5 ml per gram of original pellet). To lyse the cells, the suspension was left at room temperature for one hour, then frozen in liquid nitrogen and thawed in a 37°C bath three times, and then sonicated for two minutes. The lysate was centrifuged at 11,500 x g for 30 min. The supernatant was removed and stored. The pellet was resuspended in an equal volume of lysis buffer, centrifuged as before, and this second supernatant was combined with the first. The pooled supernatants were dialyzed versus BBS and chromatographed over the immunoaffinity column as described in Example 1.E.1. This material also retained cytotoxic activity.

### G. Assay for cytotoxic activity

The ribosome inactivating protein activity of recombinant saporin was compared to the ribosome inactivating protein activity of native SAP in an *in vitro* assay measuring cell-free protein synthesis in a nuclease-treated rabbit reticulocyte lysate (Promega). Samples of immunoaffinity-purified saporin were diluted in PBS and 5 µl of sample was added on ice to 35 µl of rabbit reticulocyte lysate and 10 µl of a reaction mixture containing 0.5 µl of Brome Mosaic Virus RNA, 1 mM amino acid mixture minus leucine, 5 µCi of tritiated leucine and 3 µl of water. Assay tubes were incubated 1 hour in a 30°C water bath. The reaction was stopped by transferring the tubes to ice and adding 5 µl of the assay mixture, in triplicate, to 75 µl of 1 N sodium hydroxide, 2.5% hydrogen peroxide in the wells of a Millititer HA 96-well filtration plate (Millipore). When the red color had bleached from the samples, 300 µl of ice cold 25% trichloroacetic acid (TCA) were added to each well and the plate left on ice for another 30 min. Vacuum filtration was performed with a Millipore vacuum holder. The wells were washed three times with 300 µl of ice cold 8% TCA. After drying, the filter paper circles were punched out of the 96-well plate and counted by liquid scintillation techniques.

The IC₅₀ for the recombinant and native saporin were approximately 20 pM. Therefore, recombinant saporin-containing protein has full protein synthesis inhibition activity when compared to native saporin.

### EXAMPLE 2

### PREPARATION OF MONO-DERIVATIZED SAPORIN

### A. Materials and Methods

### 1. Reagents

Native SAP was obtained from *Saponaria officinalis (see, e.g.,* Stirpe et al., *Biochem. J. 216*:617-625, 1983). Briefly, the seeds were extracted by grinding in 5 mM sodium phosphate buffer, pH 7.2 containing 0.14 M NaCl, straining the extracts through cheesecloth, followed by centrifugation at 28,00 g for 30 min to produce a crude extract, which was dialyzed against 5 mM sodium phosphate buffer, pH 6.5, centrifuged and applied to CM-cellulose (CM 52, Whatman, Maidstone, Kent, U.K.). The CM column was washed and SO-6 was eluted with a 0 to 0.3 M NaCl gradient in the phosphate buffer.

Plasmid isolation, production of competent cells, transformation and M13 manipulations were carried out according to published procedures (Sambrook et al. *supra*). Purification of DNA fragments was achieved using the Geneclean II kit, purchased from Bio 101 (La Jolla, CA). Sequencing of the different constructions was performed using the Sequenase kit (version 2.0) of USB (Cleveland, OH).

NovaBlue and BL21(DE3) strains were obtained (Novagen, Madison WI).

### 2. Sodium dodecyl sulfate (SDS) gel electrophoresis and Western blotting

SDS gel electrophoresis was performed on a PhastSystem™ utilizing 20% gels (Pharmacia). Western blotting was accomplished by transfer of electrophoresed protein to nitrocellulose using the PhastTransfer system (Pharmacia), as described by the manufacturer. The antisera to SAP and basic FGF were used at a dilution of 1:1000. Horseradish peroxidase labeled anti-IgG was used as the second antibody as described (Davis, L., Dibner et al., *Basic Methods in Molecular Biology,* Elsevier Science Publishing Co., New York, 1986).

### 3. Cytotoxicity assays of conjugates

Cytotoxicity experiments were performed with the Promega (Madison, WI) CellTiter 96 Cell Proliferation/Cytotoxicity Assay. Cell types used were SK-Mel-28, human melanoma Swiss 3T3 mouse fibroblasts, B16F10, mouse melanoma, PA-1, human ovarian carcinoma (from Dr. Julie Beitz, Roger Williams Hospital, Providence RI), and baby hamster kidney (BHK) (ATCC CRL 281). 2500 cells were plated per well.

### B. Derivatization and purification of mono-derivatized SAP

Saporin (49 mg) at a concentration of 4.1 mg/ml was dialyzed against 0.1 M sodium phosphate, 0.1 M sodium chloride, pH 7.5. A 1.1 molar excess (563 µg in 156 µl of anhydrous ethanol) of SPDP (Pharmacia, Uppsala, Sweden) was added and the reaction mixture immediately agitated and put on a rocker platform for 30 minutes. The solution was then dialyzed against the same buffer. An aliquot of the dialyzed solution was examined for extent of derivatization according to the Pharmacia instruction sheet. The extent of derivatization was 0.86 moles of SPDP per mole of SAP. During these experiments, another batch of SAP was derivatized using an equimolar quantity of SPDP in the reaction mixture with a resulting 0.79 molar ratio of SPDP to SAP.

Derivatized SAP (32.3 mg) was dialyzed in 0.1 M sodium borate, pH 9.0 and applied to a Mono S 16/10 column equilibrated with 25 mM sodium chloride in dialysis buffer. A gradient of 25 mM to 125 mM sodium chloride in dialysis buffer was run to elute SAP and derivatized SAP. The flow rate was 4.0 ml/min. and 4 ml fractions were collected. Aliquots of fractions were assayed for protein concentration (BCA Protein Assay, Pierce Chemical, Chicago, IL) and for pyridylthione released by reducing agent. Individual fractions (25 to 37) were analyzed for protein concentration and pyridyl-disulfide concentration. The data indicated a separation according to the level of derivatization by SPDP. The initial eluting peak was composed of SAP that is approximately di-derivatized; the second peak is mono-derivatized and the third peak shows no derivatization. The di-derivatized material accounts for 20% of the three peaks; the second accounts for 48% and the third peak contains 32%. Material from the second peak was pooled and gave an average ratio of pyridyl-disulfide to SAP of 0.95. Fraction 33, which showed a divergent ratio of pyridine-2-thione to protein, was excluded from the pool. Fractions that showed a ratio of SPDP to SAP greater than 0.85 but less than 1.05 were pooled, dialyzed against 0.1 M sodium chloride, 0.1 M sodium phosphate, pH 7.5 and used for derivatization with basic FGF. A pool of these materials had a molar ratio SPDP:SAP of 0.9 with a final yield of 4.6 mg.

### EXAMPLE 3

### RECOMBINANT PRODUCTION OF FGF-SAP FUSION PROTEIN

### A. General Descriptions

### 1. Bacterial Strains and Plasmids

*E. coli* strains BL21(DE3), BL21(DE3)pLysS, HMS174(DE3) and HMS174(DE3)pLysS were purchased from Novagen, Madison, WI. Plasmid pFC80, described below, has been described in the PCT Application No. WO 90/02800, except that the bFGF coding sequence in the plasmid designated pFC80 herein has the sequence set forth as SEQ ID NO. 1, nucleotides 1-465. The plasmids described herein may be prepared using pFC80 as a starting material or, alternatively, by starting with a fragment containing the cII ribosome binding site (SEQ ID NO. 57) linked to the FGF-encoding DNA (SEQ ID NO. 1).

*E. coli* strain JA221 (lpp- hdsM+ trpE5 leuB6 lacY recAl F'[lacI^{q} lac⁺ pro⁺]) (ATCC 33875) *(see, also,* U.S. Patent No. 4,757,013 to Inouye; and Nakamura et al., *Cell* 18:1109-1117, 1979). Strain INV1α is commercially available from Invitrogen, San Diego, CA.

### 2. DNA Manipulations

Native SAP, chemically conjugated bFGF-SAP and rabbit polyclonal antiserum to SAP and FGF were obtained as described in Lappi et al., *(Biochem. Biophys. Res. Comm. 129*:934-942, 1985), and Lappi et al. (*Biochem*. *Biophys*., *Res. Comm. 160*:917-923, 1989). The pET System Induction Control was purchased from Novagen, Madison, WI. The sequencing of the different constructions was done using the Sequenase kit of United States Biochemical Corporation (version 2.0). Minipreparation and maxipreparations of plasmids, preparation of competent cells, transformation, M13 manipulation, bacterial media and Western blotting were performed using routine methods *(see, e.g.,* Sambrook et al., *Molecular Cloning: A Laboratory Manual,* Cold Spring Harbor Laboratory Press, Cold Spring Harbor, NY, 1989). The purification of DNA fragments was done using the Geneclean II kit, purchased from Bio 101. SDS gel electrophoresis was performed on a PhastSystem™ (Pharmacia).

Rabbit polyclonal antiserum to SAP and FGF were obtained as described in Lappi et al., *Biochem. Biophys. Res. Comm. 129*:934-942, 1985, and Lappi et al., *Biochem. Biophys*., *Res. Comm. 160*:917-923, 1989. The pET System Induction Control was purchased from Novagen, Madison, WI. Minipreparation and maxipreparations of plasmids, preparation of competent cells, transformation, M13 manipulation, bacterial media and Western blotting were performed using routine methods *(see, e.g.,* Sambrook et al., *supra*). The purification of DNA fragments was done using the Geneclean II kit, purchased from Bio 101. SDS gel electrophoresis was performed on a PhastSystem™ (Pharmacia).

Western blotting was accomplished by transfer of the electrophoresed protein to nitrocellulose using the PhastTransfer system, as described by the manufacturer. Horseradish peroxidase labeled anti-IgG was used as the second antibody *(see* Davis et al., *Basic Methods In Molecular Biology,* New York, Elsevier Science Publishing Co., pp. 1-338, 1986).

### B. Construction of plasmids encoding FGF-SAP fusion proteins

### 1. Construction of FGFM13 that contains DNA encoding the CI ribosome binding site linked to FGF

A *Nco* I restriction site was introduced into the SAP-encoding DNA the M13mp18-G4 clone, prepared as described above by site-directed mutagenesis method using the Amersham *In vitro*-mutagenesis system 2.1. The oligonucleotide employed to create the *Nco* I restriction site was synthesized using a 380B automatic DNA synthesizer (Applied Biosystems) and is listed as: SEQ ID NO. 58 - CAACAACTGCCATGGTCACATC. This oligonucleotide containing the *Nco* I site replaced the original SAP-containing coding sequence at SEQ ID NO. 47, nts 32-53. The resulting M13mp18-G4 derivative is termed mpNG4.

In order to produce a bFGF coding sequence in which the stop codon was removed, the FGF-encoding DNA was subcloned into a M13 phage and subjected to site-directed mutagenesis. Plasmid pFC80 is a derivative of pDS20 *(see, e.g.*, Duester et al., *Cell* 30:855-864, 1982; *see, also*, U.S. Patent Nos. 4,914,027, 5,037,744, 5,100,784, and 5,187,261; *see, also,* PCT Application WO 90/02800; and European Patent Application No. EP 267703 A1), which is very similar to plasmid pKG1800 *(see,* Bernardi et al., *DNA Sequence 1*:147-150, 1990; *see, also,* McKenney et al. (1981) pp. 383-415 in *Gene Amplification and Analysis 2: Analysis of Nucleic Acids by Enzymatic Methods,* Chirikjian et al. (eds.), North Holland Publishing Company, Amsterdam) except that it contains an extra 440 bp at the distal end of *galK* between nucleotides 2440 and 2880 in pDS20. Plasmid pKG1800 includes the 2880 bp *Eco*R I-*Pvu* II of pBR322 that contains the contains the ampicillin resistance gene and an origin of replication.

Plasmid pFC80 was prepared from pDS20 by replacing the entire *galK* gene with the FGF-encoding DNA of SEQ ID NO. 1, inserting the trp promoter (SEQ ID NO. 59) and the bacteriophage lambda cII ribosome binding site (SEQ ID NO. 57; *see, e.g.,* Schwarz et al., *Nature 272:410,* 1978) upstream of and operatively linked to the FGF-encoding DNA. The trp promoter can be obtained from plasmid pDR720 (Pharmacia PL Biochemicals) or synthesized according to SEQ ID NO. 59. Plasmid pFC80, contains the 2880 bp *Eco*R I-*Bam*H I fragment of plasmid pSD20, a synthetic *Sal* I-*Nde* I fragment that encodes the trp promoter region (SEQ ID NO. 59): 
EcoR 1

GAATTCCCCTGTTGACAATTAATCATCGAACTAGTTAACTAGTACGCAGCTTGGCTGCAG
 and the cII ribosome binding site (SEQ ID NO. 57):

Sal I Nde I
GTCGACCAAGCTTGGGCATACATTCAATCAATTGTTATCTAAGGAAATACTTACATATG

The FGF-encoding DNA was removed from pFC80 by treating it as follows. The pFC80 plasmid was digested by *Hga* I and *Sal I,* which produces a fragment containing the cII ribosome binding site linked to the FGF-encoding DNA. The resulting fragment was blunt ended with DNA pol I (Klenow fragment) and inserted into *Sma* I digested M13mp18 that was treated with alkaline phosphatase. In order to remove the stop codon, an insert in the ORI minus direction was mutagenized using the Amersham kit, as described above, using the following oligonucleotide (SEQ ID NO. 60): GCTAAGAGCGCCATGGAGA. SEQ ID NO. 60 contains one nucleotide between the FGF carboxy terminal serine codon and a *Nco* I restriction site; it replaced the following wild type FGF encoding DNA having SEQ ID NO. 61:

GCT AAG AGC TGA  CCA TGG AGA
Ala Lys Ser STOP Pro Trp Arg

The resulting mutant derivative of M13mp18, lacking a native stop codon after the carboxy terminal serine codon of bFGF, was designated FGFM13. The mutagenized region of FGFM13 contained the correct sequence (SEQ ID NO. 62).

### 2. Preparation of plasmids pFS92 (PZ1A), PZ1B and PZ1C that encode the FGF-SAP fusion protein (FPFS1)

### a. Plasmid pFS92 (also designated PZ1A)

Plasmid FGFM13 was cut with *Nco* I and *Sac* I to yield a fragment containing the cII ribosome binding site linked to the bFGF coding sequence with the stop codon replaced.

The M13mp18 derivative mp18-NG4 containing the saporin coding sequence was also cut with restriction endonucleases *Nco* I and *Sac* I, and the bFGF coding fragment from FGFM13 was inserted by ligation to DNA encoding the fusion protein bFGF-SAP into the M13mp18 derivative to produce mpFGF-SAP, which contains the cII ribosome binding site linked to the FGF-SAP fusion gene. The sequence of the fusion gene is set forth in SEQ ID NO. 7 and indicates that the FGF protein carboxy terminus and the saporin protein amino terminus are separated by 6 nucleotides (SEQ ID NO. 2, nts 466-471) that encode two amino acids Ala Met.

Plasmid mpFGF-SAP was digested with *Xba* I and *EcoR I* and the resulting fragment containing the bFGF-SAP coding sequence was isolated and ligated into *Eco* RI and *Xba* I digested plasmid pET 11a (available from Novagen, Madison, WI; for a description of the plasmids *see* U.S. Patent No. 4,952,496; Studier et al., *Meth. Enz. 185*:60-89, 1990; Studier et al., *J. Mol. Biol. 189*:113-130, 1986; Rosenberg et al., *Gene 56*:125-135, 1987). The resulting plasmid was designated pFS92. It was renamed PZ1A.

Plasmid pFS92 (or PZ1A) contains DNA of the entire basic FGF protein (SEQ ID NO. 2), a 2-amino acid long connecting peptide, and amino acids 1 to 253 of the mature SAP protein. Plasmid pFS92 also includes the cII ribosome binding site linked to the FGF-SAP fusion protein and the T7 promoter region from pET 11a.

*E. coli* strain BL21(DE3)pLysS (Novagen, Madison WI) was transformed with pFS92 according to manufacturer's instructions and the methods described herein.

### b. Plasmid PZ1B

Plasmid pFS92 was digested with *EcoR I,* the ends repaired by adding nucleoside triphosphates and DNA polymerase (Klenow fragment), and then digested with *Nde* I to release the FGF-encoding DNA without the cII ribosome binding site. This fragment was ligated into pET 11a, which had been *Bam*H I digested, treated to repair the ends, and digested with *Nde* I. The resulting plasmid was designated PZ1B. PZ1B includes the T7 transcription terminator and the pET-11a ribosome binding site.

### c. Plasmid PZ1C

Plasmid PZ1C was prepared from PZ1B by replacing the ampicillin resistance gene with a kanamycin resistance gene.

### d. Plasmid PZ1D

Plasmid pFS92 was digested with *Eco*R I and *Nde* I to release the FGF-encoding DNA without the cII ribosome binding site and the and the ends were repaired. This fragment was ligated into pET 12a, which had been *Bam*H I digested and treated to repair the ends. The resulting plasmid was designated PZ1D. PZ1D includes DNA encoding the OMP T secretion signal operatively linked to DNA encoding the fusion protein.

### C. Expression of the recombinant bFGF-SAP fusion proteins (FPFS1)

The two-stage method described above was used to produce recombinant bFGF-SAP protein (hereinafter bFGF-SAP fusion protein).

### 1. Expression of rbFGF-SAP from pFS92 (PZ1A)

Three liters of LB broth containing ampicillin (50 µg/ml) and chloramphenicol (25 µg/ml) were inoculated with pFS92 plasmid-containing bacterial cells (strain BL21(DE3)pLysS) from an overnight culture (1:100 dilution) that were obtained according to Example 2.B. Cells were grown at 37°C in an incubator shaker to an OD₆₀₀ of 0.7. IPTG (Sigma Chemical, St. Louis, MO) was added to a final concentration of 0.2 mM and growth was continued for 1.5 hours at which time cells were centrifuged. Subsequent experiments have shown that growing the BL21(DE3)pLysS cells at 30°C instead of 37°C improves yields. When the cells are grown at 30°C they are grown to an OD₆₀₀ of 1.5 prior to induction. Following induction, growth is continued for about 2 to 2.5 hours at which time the cells are harvested by centrifugation.

The pellet was resuspended in lysis solution (45-60 ml per 16 g of pellet; 20 mM Tris, pH 7.4, 5 mM EDTA, 10% sucrose, 150 mM NaCl, lysozyme, 100 µg/ml, 10 µg/ml aprotinin, 10 µg/ml leupeptin, 10 µg/ml pepstatin A, and 1 mM PMSF) and incubated with stirring for 1 hour at room temperature. The solution was frozen and thawed three times and sonicated for 2.5 minutes. The suspension was centrifuged at 12,000 X g for 1 hour; the resulting first-supernatant was saved and the pellet was resuspended in another volume of lysis solution without lysozyme. The resuspended material was centrifuged again to produce a second-supernatant, and the two supernatants were pooled and dialyzed against borate buffered saline, pH 8.3.

### 2. Expression of bFGF-SAP fusion protein from PZ1B and PZ1C

Two hundred and fifty ml of LB medium containing ampicillin (100 µg/ml) were inoculated with a fresh glycerol stock of PZ1D. Cells were grown at 30°C in an incubator shaker to an OD₆₀₀ of 0.7 and stored overnight at 4°C. The following day the cells were pelleted and resuspended in fresh LB medium (no ampicillin). The cells were divided into 5 1-liter batches and grown at 30°C in an incubator shaker to an OD₆₀₀ of 1.5. IPTG (Sigma Chemical, St. Louis, MO) was added to a final concentration of 0.1 mM and growth was continued for about 2 to 2.5 hours at which time cells were harvested by centrifugation.

For growing PZIC, the cells are grown in medium containing kanamycin (50µg/ml) in place of ampicillin prior to induction.

### 3. Expression of bFGF-SAP fusion protein from PZ1D

Two hundred and fifty mls of LB medium containing ampicillin (100 µg/ml) were inoculated with a fresh glycerol stock of PZ1B. Cells were grown at 30°C in an incubator shaker to an OD₆₀₀ of 0.7 and stored overnight at 4°C. The following day the cells were pelleted and resuspended in fresh LB medium (no ampicillin). The cells were used to inoculate a 1 liter batch of LB medium and grown at 30°C in an incubator shaker to an OD₆₀₀ of 1.5. IPTG (Sigma Chemical, St. Louis, MO) was added to a final concentration of 0.1 mM and growth was continued for about 2 to 2.5 hours at which time cells were harvested by centrifugation. The cell pellet was resuspended in ice cold 1.0 M Tris pH 9.0. 2 mM EDTA. The resuspended material is kept on ice for another 20-60 minutes and then centrifuged to separate the periplasmic fraction (supernatant) from the intracellular fraction (pellet).

### D. Affinity purification of bFGF-SAP fusion protein

Thirty ml of the dialyzed solution containing the bFGF-SAP fusion protein was applied to HiTrap™ heparin-Sepharose® column (Pharmacia, Uppsala, Sweden) equilibrated with 0.15M NaCl in 10 mM TRIS, pH 7.4 (buffer A). The column was washed: first with equilibration buffer; second with 0.6 M NaCI in buffer A; third with 1.0 M NaCl in buffer A; and finally eluted with 2 M NaCl in buffer A into 1.0 ml fractions. Samples were assayed by the ELISA method.

The results indicate that the bFGF-SAP fusion protein elutes from the heparin-Sepharose® column at the same concentration (2 M NaCI) as native and recombinantly-produced bFGF. This indicates that the heparin affinity is retained in the bFGF-SAP fusion protein.

### E. Characterization of the bFGF-SAP fusion protein

### 1. Western blot of affinity-purified bFGF-SAP fusion protein

SDS gel electrophoresis was performed on a PhastSystem™ utilizing 20% gels (Pharmacia). Western blotting was accomplished by transfer of the electrophoresed protein to nitrocellulose using the PhastTransfer system (Pharmacia), as described by the manufacturer. The antisera to SAP and bFGF were used at a dilution of 1:1000 dilution. Horseradish peroxidase labeled anti-IgG was used as the second antibody (Davis et al., *Basic Methods in Molecular Biology,* New York, Elsevier Science Publishing Co., 1986).

The anti-SAP and anti-FGF antisera bound to a protein with an approximate molecular weight of 48,000 kd, which corresponds to the sum of the independent molecular weights of SAP (30,000) and bFGF (18,000).

### 2. Assays to assess the cytotoxicity of the FGF-SAP fusion protein

The RIP activity of bFGF-SAP fusion protein compared to the FGF-SAP chemical conjugate was assayed as described in Example 1. The results indicated that the IC₅₀ of the bFGF-SAP fusion protein is about 0.2 nM and the IC₅₀ of chemically conjugated FGF-SAP is about 0.125 nm.

Cytotoxicity experiments were performed with the Promega (Madison, WI) CellTiter 96 Cell Proliferation/Cytotoxicity Assay. About 1,500 SK-Mel-28 cells (available from ATCC), a human melanoma cell line, were plated per well in a 96 well plate in 90 µl HDMEM plus 10% FCS and incubated overnight at 37°C, 5% CO₂. The following morning 10 µl of media alone or 10 µl of media containing various concentrations of the rbFGF-SAP fusion protein, basic FGF or saporin were added to the wells. The plate was incubated for 72 hours at 37°C. Following the incubation period, the number of living cells was determined by measuring the incorporation and conversion of the dye MTT supplied as a part of the Promega kit. Fifteen µl of the MTT solution was added to each well, and incubation was continued for 4 hours. Next, 100 µl of the standard solubilization solution supplied as a part of the Promega kit was added to each well. The plate was allowed to stand overnight at room temperature and the absorbance at 560 nm was read on an ELISA plate reader (Titertek Multiskan PLUS, ICN, Flow, Costa Mesa, CA).

The results indicated that the chemical FGF-SAP conjugate has an ID₅₀ of 0.3 nM, the bFGF-SAP fusion protein has a similar ID₅₀ of 0.6 nM, and unconjugated SAP, which is unable to bind to the cell surface, has an ID₅₀ of 200 nM. Therefore, when internalized, the bFGF-SAP fusion protein appears to have approximately the same cytotoxic activity as the chemically conjugated FGF-SAP.

### EXAMPLE 4

### PREPARATION OF MODIFIED SAPORIN

Saporin was modified by addition of a cysteine residue at the N-terminus-encoding portion of the DNA or by the addition of a cysteine at position 4 or 10. The resulting saporin is then reacted with an available cysteine on an FGF to produce conjugates that are linked via the added Cys or Met-Cys on saporin.

Modified SAP has been prepared by altering the DNA encoding the SAP by inserting DNA encoding Met-Cys at position -1 or by replacing the Ile or the Asp codon within 10 or fewer residues of the N-terminus with Cys. The resulting DNA has been inserted into pET 11a and pET 15b and expressed in BL21(DE3) cells. The resulting saporin proteins are designated FPS1 (saporin with Cys at -1), FPS2 (saporin with Cys at position 4) and FPS3 (saporin with Cys at position 10). A plasmid that encodes FPS1 and that has been used for expression of FPS1 has been designated PZ50B. Plasmids that encode FPS2 and that have been used for expression of FPS2 have been designated PZ51B (pET11a-based plasmid) and PZ51E (pET15b-based plasmid). Plasmids that encode FPS3 and that have been used for expression of FPS3 have been designated PZ52B (pET11a-based plasmid) and PZ52E (pET 15b-based plasmid).

### A. Preparation of saporin with an added cysteine residue at the N-terminus

The DNA encoding SAP-6 was amplified by polymerase chain reaction (PCR) from the parental plasmid pZ1B1 as described by McDonald et al. (1995). The plasmid pZ1B1 contains the DNA sequence for human FGF-2 linked to SAP-6 by a two amino acid linker (Ala-Met). pZ1B1 also includes the T7 promoter, lac operator, ribosomal binding site, and T7 terminator present in the pET-11a vector. For SAP-6 DNA amplification, the 5' primer (5' CATATGTGTGTCACATCAATCAC ATTAGAT-3') (SEQ ID NO. 55) corresponding to the "sense" strand of SAP-6 incorporated a *Nde*I restriction enzyme site used for cloning. It also contained a Cys codon at position -1 relative to the start site of the mature protein sequence. No leader sequence was included. The 3' primer (5' CAGGTTTGGATCCTTTACGTT 3') (SEQ. ID NO. 56), corresponding to the "antisense" strand of SAP-6 has a *Bam*HI site used for cloning. The amplified DNA was gel purified and digested with *Nde*I and *Bam*HI. The digested SAP-6 DNA fragment was subcloned into the *Nde*I and *Bam*HI digested pZ1B1. This digestion removed FGF-2 and the 5' portion of SAP-6 (up to nucleotide position 650) from the parental rFGF2-SAP vector (pZ1B1) and replaced this portion with a SAP-6 molecule containing a Cys at position -1 relative to the start site of the native mature SAP-6 protein. The resultant plasmid was designated as pZ50B. pZ50B was transformed into *E. coli* strain NovaBlue for restriction and sequencing analysis. The appropriate clone was then transformed into *E. coli* strain BL21(DE3) for expression and large scale production.

### B. Preparation of saporin with a cysteine residue at position 4 or 10 of the native protein

These constructs were designed to introduce a cysteine residue at position 4 or 10 of the native protein by replacing the isoleucine residue at position 4 or the asparagine residue at position 10 with cysteine.

SAP was amplified by polymerase chain reaction (PCR) from the parental plasmid pZ1B encoding the FGF-SAP fusion protein using a primer corresponding to the sense strand of saporin, spanning nucleotides 466-501 of SEQ ID NO. 2, which incorporates a *Nde*I site and replaces the Ile codon with a Cys codon at position 4 of the mature protein (SEQ ID NO. 63): CATATGGTCACATCATGTACATTAGATCTAGTAAAT. or a primer corresponding to the sense strand of saporin, nucleotides 466-515 of SEQ ID NO. 2, incorporates a *Nde*I site and replaces the Asp codon with a Cys codon at position 10 of the mature protein (SEQ ID NO. 64) CATATGGTCACATCAATCACATTAGATCTAGTATGTCCGACCGCGGGTCA. The 3' primer complements the coding sequence of saporin spanning nucleotides 547-567 of SEQ ID NO. 12 and contains a *BamH*I site (SEQ ID NO. 56):

CAGGTTTGGATCCTTTACGTT.

The PCR amplification reactions were performed as described above, using the following cycles: denaturation step 94°C for 1 min, annealing for 2 min at 60°C, and extension for 2 min at 72°C for 35 cycles. The amplified DNA was gel purified, digested with *Nde*I and *BamH*I, and subcloned into *Nde*I and *Bam*HI digested pZ1B. This digestion removed the FGF and 5' portion of SAP (up to the *BamH*I site) from the parental FGF-SAP vector (pZ1B) and replaced this portion with a SAP molecule containing a Cys at position 4 or 10 relative to the start site of the native mature SAP protein (see SEQ ID NOS. 67 and 68, respectively). The resulting plasmids are designated pZ51B and pZ52B, respectively.

### C. Cloning of DNA encoding SAP mutants in vector pET15b

### 1. The SAP-Cys-1 mutants

The initial step in this construction was the mutagenesis of the internal *Bam*HI site at nucleotides 555-560 (SEQ ID NO. 2) in pZ1B by PCR using a sense primer corresponding to nucleotides 543-570 (SEQ ID NO. 2) but changing the G at nucleotide 555 (the third position in the Lys codon) to an A. The complement of the sense primer was used as the antisense primer (SEQ ID NO. 65). The first round of amplification used primers SEQ ID NOS. 69 and 65 or 66 and 70 conducted as above. Individual fragments were gel purified and a second round of amplification was performed using primers of SEQ ID NOS. 69 and 66 as above. This amplification introduced a *Nde*I site and a Cys codon onto the 5' end of the saporin-encoding DNA. The antisense primer was complementary to the 3' end of the saporin protein and encoded a *Bam*HI site for cloning and a stop codon (SEQ ID NO. 66):

GGATCCGCCTCGTTTGACTACTT.

The resulting fragment was digested with *Nde*I/*Bam*HI and inserted into pET15b (Novagen, Madison, WI), which has a His-Tag™ leader sequence (SEQ ID NO. 18), that had also been digested with *Nde*I/*Bam*HI.

### 2. The SAP-Cys+4 and Sap-Cys+10 mutants

This construction was performed similarly to the SAP-Cys-1 using pZ1B as the starting material, and splice overlap extension (SOE) using PZ1B as the starting plasmid, including mutagenesis of the internal *Bam*HI site at nucleotides 555-560 (SEQ ID NO. 2) in pZlB by PCR using a sense primer corresponding to nucleotides 543-570 (SEQ ID NO. 2) but changing the G at nucleotide 555 (the third position in the Lys codon) to an A and introduction of the cys at position 4 or 10 in place of the native amino acid.

The first round of amplification used primers of SEQ ID NOS. 63 and 65 (for the cys+4 saporin mutants) or SEQ ID NOS. 64 and 65 for the cys+10 saporin mutants):

CATATGGTCACATCATGTACATTAGATCTAGTAAAT (SEQ ID NO. 63);

CATATGGTCACATCAATCACATTAGATCTAGTATGTCCGACCGCGGGTCA (SEQ ID NO. 64);

TTTCAGGTTTGGATCTTTTACGTTGTTT (SEQ ID NO. 65).

Amplification conditions were as follows: denaturation for 1 min at 94°C, annealing for 2 min at 70°C and extension for 2 min at 72°C for 35 cycles. Individual fragments were gel purified and subjected to a second round of amplification, following the same protocol, using only the external oligos of SEQ ID NO. 66 and SEQ ID NO. 63 for the cys+4 mutant or SEQ ID NO. 64 for the cys+10 mutant. The resulting fragments had a *Nde*I site on the 5' end of the saporin-encoding DNA and a *Bam*HI site for cloning and a stop codon on the 3' end. The resulting fragment was digested with *Nde*I/*Bam*HI and inserted into pET 15b (Novagen, Madison, WI), which has a His-Tag™ leader sequence (SEQ ID NO. 18), that had also been digested *Nde*I/*Bam*HI.

DNA encoding unmodified SAP (EXAMPLE 1) can be similarly inserted into a pET15b or pET1 1a and expressed as described below for the modified SAP-encoding DNA.

### E. Expression of the modified saporin-encoding DNA

The *E. coli* cells containing Cys-1 SAP construct were grown in a high cell density fed-batch fermentation with the temperature and pH controlled at 30°C and 6.9, respectively. A glycerol stock (1 mL) was grown in 50 ml of Luria Broth until A₆₀₀ reached 0.6. Inoculum (10 mL) was injected into a 7 L Applikon (Foster City, CA) fermentor containing 2 L of complex batch media consisting of 5 g/L of glucose, 1.25 g/L, each, of yeast extract and tryptone (Difco Laboratories, Detroit, MI, U.S.A.), 7 g/L of K₂HPO₄, 8 g/L of KH₂PO₄, 1.66 g/L of (NH₄)₂SO₄, 1 g/L of MgSO₄•7H₂O, 2 mL/L of a trace metal solution (74 g/L of Na₃Citrate, 27 g/L of FeCl₃•6H₂O, 2.0 g/L of CoCl₂•6H₂O, 2.0 g/L of Na₂MoO₄•2H₂O, 1.9 g/L of CuSO₄•5H₂O, 1.6 g/L of MnCl₂•4H₂O, 1.4 g/L of ZnCl₂•4H₂O, 1.0 g/L of CaCl₂•2H₂O, 0.5 g/L of H₃BO₃), 2 mL/L of a vitamin solution (6 g/L thiamine•HCl, 3.05 g/L of niacin, 2.7 g/L of pantothenic acid, 0.7 g/L of pyridoxine•HCl, 0.21 g/L of riboflavin, 0.03 g/L of biotin, 0.02 g/L of folic acid), and 100 mg/L of carbenicillin. The culture was grown for 12 hour before initiating the continuous addition of a 40x solution of complex batch media lacking the phosphates and containing only 25 mL/L, each, of trace metal and vitamin solutions. The feed addition continued until the A₆₀₀ of the culture reached 85, at which time (approximately 9 h) the culture was induced with 0.1 mM IPTG. During 4 h of post-induction incubation, the culture was fed with a solution containing 100 g/L of glucose, 100 g/L of yeast extract, and 200 g/L of tryptone. Finally, the cells were harvested by centrifugation (8,000 x g, 10 min) and frozen at -80°C until further processed.

### F. Purification and conjugation of modified saporin

The cell pellet (~400 g wet weight) containing Cys-1 SAP was resuspended in 3 volumes of buffer B (10 mM sodium phosphate, pH 7.0, 5 mM EGTA, and 1 mM DTT). The suspension was passed through a microfluidizer three times at 18,000 lb/in² on ice. The resultant lysate was diluted with NanoPure H₂O until conductivity fell below 2.7 mS/cm. All subsequent procedures were performed at room temperature.

The dilute lysate was loaded onto an expanded bed of Streamline SP cation-exchange resin (300 ml) pre-equilibrated with buffer C (20 mM sodium phosphate, pH 7.0, 1 mM EDTA) at 100 mL/min upwards flow. The resin was washed with buffer C until it appeared clear. The plunger was then lowered at 2 cm/min while washing continued at 70 mL/min. Upwards flow was stopped when the plunger was approximately 8 cm away from the bed, and the plunger was allowed to move to within 0.5 cm of the packed bed. The resin was further washed at 70 mL/min downwards flow until A₂₈₀ reached baseline. Buffer C plus 0.25 M NaCl was then used to elute proteins containing Cys-1 SAP at the same flow rate.

The eluate was buffer exchanged into buffer D (50 mM sodium borate, pH 8.5, 1 mM EDTA) using the Sartocon Mini crossflow filtration system with a 10,000 NMWCO module (Sartorious, Goettingen, Germany). The sample was then applied to a column of Source 15S (30 mL) pre-equilibrated with buffer D. A 10 column volume linear gradient of 0 to 0.3 M NaCl in buffer D was used to elute Cys-1 SAP at 30 mL/min.

Both Cys-1 SAP and C96S FGF-2 were reduced with a final concentration of 10 mM DTT prior to gel filtration with buffer E (0.1 M sodium phosphate, pH 7.5, 0.1 M NaCl, 1 mM EDTA). The Cys-1 SAP was then reacted with 80-fold molar excess of DTNB at room temperature for 1 h, and the amount of Cys-1 SAP-TNB was determined by measuring absorbance at 412 nm using the molar absorption coefficient of 14,150 M⁻¹cm⁻¹. The Cys-1 SAP/DTNB mixture was subjected to size exclusion chromatography and eluted with buffer E. The C96S FGF-2 was added to the DTNB-treated Cys-1 SAP in a molar ratio of 3:1, and the reaction was carried out at 4°C overnight.

The reaction mixture was loaded onto a column of Heparin-Sepharose CL-4B pre-equilibrated with 0.5 M NaCl in buffer F (10 mM sodium phosphate, pH 6.0, 1 mM EDTA). The column was washed with 0.5 M then 1 MNaCl in buffer F, and the conjugate eluted with 2 M NaCl in buffer F. Fractions containing FGF2-Cys-1 SAP were combined, concentrated, and applied to a column of Superdex 75. Buffer G (10 mM sodium phosphate, pH 6.0, 0.15 M NaCl, 0.1 mM EDTA) was used for the Superdex 75 column.

During Cys-1 SAP purification, SDS-PAGE was performed on 12% acrylamide Mini-PROTEAN II Ready Gels (Bio-Rad, Hercules, CA, U.S.A.) according to the method of Laemmli (1970) under non-reducing conditions. PhastSystem using 10-15% acrylamide gradient gels.

### EXAMPLE 5

### PREPARATION OF FGF MUTEINS

### A. Materials and Methods

### 1. Reagents

Restriction and modification enzymes were purchased from BRL (Gaithersburg, MD), Stratagene (La Jolla, CA) and New England Biolabs (Beverly, MA). Native SAP, chemically conjugated basic FGF-SAP and rabbit polyclonal antiserum to SAP and basic FGF were obtained from *Saponaria officinalis (see, e.g.,* Stirpe et al., *Biochem. J. 216*:617-625, 1983). Briefly, the seeds were extracted by grinding in 5 mM sodium phosphate buffer, pH 7.2 containing 0.14 M NaCl, straining the extracts through cheesecloth, followed by centrifugation at 28,00 g for 30 min to produce a crude extract, which was dialyzed against 5 mM sodium phosphate buffer, pH 6.5, centrifuged and applied to CM-cellulose (CM 52, Whatman, Maidstone, Kent, U.K.). The CM column was washed and SO-6 was eluted with a 0-0.3 M NaCI gradient in the phosphate buffer.

Plasmid pFC80, containing the basic FGF coding sequence, was a gift of Drs. Paolo Sarmientos and Antonella Isacchi of Farmitalia Carlo Erba (Milan, Italy). Plasmid pFC80 has been described in the PCT Applications WO 90/02800 and PCT/US93/05702, which are herein incorporated in their entirety by reference. The sequence of DNA encoding bFGF in pFC80 is presented in SEQ ID NO. 1. The construction of pFC80 is set forth above in Example 2.

Plasmid isolation, production of competent cells, transformation and M13 manipulations were carried out according to published procedures (Sambrook et al., *supra).* Purification of DNA fragments was achieved using the Geneclean II kit, purchased from Bio 101 (La Jolla, CA). Sequencing of the different constructions was performed using the Sequenase kit (version 2.0) of USB (Cleveland, OH).

### 2. SDS gel electrophoresis and Western blotting

SDS gel electrophoresis was performed on a PhastSystem™ utilizing 20% gels (Pharmacia). Western blotting was accomplished by transfer of electrophoresed protein to nitrocellulose using the PhastTransfer system (Pharmacia), as described by the manufacturer. The antisera to SAP and basic FGF were used at a dilution of 1:1000. Horseradish peroxidase labeled anti-IgG was used as the second antibody as described (Davis, L., et al., *supra).*

### B. Preparation of the mutagenized FGF by site-directed mutagenesis

Cysteine to serine substitutions were made by oligonucleotide-directed mutagenesis using the Amersham (Arlington Heights, IL) *in vitro*-mutagenesis system 2.1. Oligonucleotides encoding the new amino acid were synthesized using a 380B automatic DNA synthesizer (Applied Biosystems, Foster City, CA).

The oligonucleotide used for *in vitro* mutagenesis of cysteine 78 was AGGAGTGTCTGCTAACC (SEQ ID NO. 71), which spans nucleotides 225-241 of SEQ ID NO. 1). The oligonucleotide for mutagenesis of cysteine 96 was TTCTAAATCGGTTACCGATGACTG (SEQ ID NO. 72), which spans nucleotides 279-302 of SEQ ID NO. 1). The mutated replicative form DNA was transformed into *E. coli* strain JM109 and single plaques were picked and sequenced for verification of the mutation. The FGF mutated gene was then cut out of M13, ligated into the expression vector pFC80, which had the non-mutated form of the gene removed, and transformed into *E. coli* strain JM109. Single colonies were picked and the plasmids sequenced to verify the mutation was present. Plasmids with correct mutation were then transformed into the *E. coli* strain FICE 2 and single colonies from these transformations were used to obtain the mutant basic FGFs. An excellent level of expression, approximately 20 mg per liter of fermentation broth, was achieved.

Cells were grown overnight in 20 ml of LB broth containing 100 µg/ml ampicillin. The next morning the cells were pelleted and transferred to 500 ml of M9 medium with 100 µg/ml ampicillin and grown for 7 hours. The cells were pelleted and resuspended in lysis solution (10 mM Tris, pH 7.4, 150 mM NaCI, lysozyme, 10 µg/mL, aprotinin, 10 µg/mL, leupeptin, 10 µg/mL, pepstatin A, 10 µg/mL and 1 mM PMSF; 45-60 ml per 16 g of pellet) and incubated while stirring for 1 hour at room temperature. The solution was frozen and thawed three times and sonicated for 2.5 minutes. The suspension was centrifuged; the supernatant saved and the pellet resuspended in another volume of lysis solution without lysozyme, centrifuged again and the supernatants pooled. Extract volumes (40 ml) were diluted to 50 ml with 10 mM TRIS, pH 7.4 (buffer A). Pools were loaded onto a 5 ml Hi-Trap heparin-Sepharose® column (Pharmacia, Uppsala, Sweden) equilibrated in 150mM sodium chloride in buffer A. The column was washed with 0.6 M sodium chloride and 1 M sodium chloride in buffer A and then eluted with 2 M sodium chloride in buffer A. Peak fractions of the 2 M elution, as determined by optical density at 280 nm, were pooled and purity determined by gel electrophoresis. Yields were 10.5 mg of purified protein for the Cys₇₈ mutant and 10.9 mg for the Cys₉₆ mutant.

The biological activity of [C78S]FGF and [C96S]FGF was measured on adrenal capillary endothelial cells in culture. Cells were plated 3,000 per well of a 24 well plate in 1 ml of 10% calf serum-HDMEM. When cells were attached, samples were added in triplicate at the indicated concentration and incubated for 48 h at 37°C. An equal quantity of samples was added and further incubated for 48 h. Medium was aspirated; cells were treated with trypsin (1 ml volume) to remove cells to 9 ml of Hematall diluent and counted in a Coulter Counter. The results show that the two mutants that retain virtually complete proliferative activity of native basic FGF as judged by the ability to stimulate endothelial cell proliferation in culture.

### EXAMPLE 6

### PREPARATION OF CONJUGATES CONTAINING FGF MUTEINS

### A. Coupling of FGF muteins to SAP

### 1. Preparation of [C78S]FGF-SAP (CCFS2) and [C96S]FGF-SAP (CCFS3)

[C78S]FGF or [C96S]FGF (1 mg; 56 nmol) that had been dialyzed against phosphate-buffered saline was added to 2.5 mg mono-derivatized SAP (a 1.5 molar excess over the basic FGF mutants) and left on a rocker platform overnight. The next morning the ultraviolet-visible wavelength spectrum was taken to determine the extent of reaction by the release of pyridylthione, which adsorbs at 343 nm with a known extinction coefficient. The ratio of pyridylthione to basic FGF mutant for [C78S]FGF was 1.05 and for [C96S]FGF was 0.92. The reaction mixtures were treated identically for purification in the following manner: reaction mixture was passed over a HiTrap™ heparin-Sepharose® column (1 ml) equilibrated with 0.15 M sodium chloride in buffer A at a flow rate of 0.5 ml/min. The column was washed with 0.6 M NaCl and 1.0 M NaCl in buffer A and the product eluted with 2.0 M NaCl in buffer A. Fractions (0.5 ml) were analyzed by gel electrophoresis and absorbance at 280 nm. Peak tubes were pooled and dialyzed versus 10 mM sodium phosphate, pH 7.5 and applied to a Mono-S 5/5 column equilibrated with the same buffer. A 10 ml gradient between 0 and 1.0 M sodium chloride in equilibration buffer was used to elute the product. Purity was determined by gel electrophoresis and peak fractions were pooled. The yield for [C78S]FGF-SAP was 1.6 mg (60% with respect to starting amount of [C78S]FGF) and for was 0.96 mg [C96S]FGF-SAP (35%). Virtually 100% of the mutant FGFs reacted with mono-derivatized SAP ([C78S]FGF: 105%, [C96S]FGF: 92%). Because the free surface cysteine of each mutant acts as a free sulfhydryl, it was unnecessary to reduce cysteines after purification from the bacteria. The resulting product was purified by heparin-Sepharose® (data not shown), thus establishing that heparin binding activity of the conjugate is retained.

Coomassie staining and Western blotting of the purified proteins showed a prominent band at a molecular weight of about 48,000, corresponding to the combined molecular weights of SAP and bFGF. A much lighter band at a slightly lower molecular weight was detected and attributed to the described mobility of an artifact produced by the high isoelectric point (10.5) (Gelfi et al., *J. Biochem. Biophys. Meth. 15*:41-48, 1987) of SAP that causes a smearing in SDS gel electrophoresis *(see, e.g.,* Lappi et al., *Biochem. Biophys. Res. Commun. 129*:934-942, 1985). No higher molecular weight bands, corresponding to conjugates containing more than one molecule of SAP per molecule of basic FGF or more than one molecule of basic FGF per molecule of SAP were detected on Coomassie-stained gels of [C78S]FGF-SAP) and of ([C96S]FGF-SAP). Such bands were present in lanes on the gel in which an equal quantity (by weight) of heterogeneous FGF-SAP, synthesized from wild-type bFGF and non-purified derivatized SAP, had been loaded.

Western blotting using antibodies to SAP or basic FGF revealed that, while 480 ng of either [C78S]FGF-SAP or [C96S]FGF-SAP results in a well-visualized band (with the additional slight lower molecular weight band) the same quantity of conjugate produced by the previous procedure is almost undetectable. As in the Coomassie staining, the Western blotting of the mutant FGF-SAPs reveals much greater homogeneity than with heterogeneous FGF-SAP synthesized with non-mutagenized basic FGF and non-purified derivatized SAP.

### 2. Preparation of [C96S]FGF-rSAP (CCFS4)

Recombinant saporin that has the cys added at the N-terminus (SAP-CYS-(-1)) that was cloned and expressed in BL21 cells and isolated as described in EXAMPLE 4 was coupled to [C96S]FGF using (5,5'-dithiobis-(2-nitrobenzoic acid)) DTNB also called Ellman's reagent. The rSAP and [C96S]FGF were each treated with 10 mM dithiothreitol (DTT), incubated for 1 h at room temperature, and the DTT was removed by gel filtration in conjugation buffer (0.1 M NaPO₄, 100 NaCl and 1 mM EDTA, pH 7.5). A 100-fold molar excess of DTNB was added to the rSAP, incubated for 1 h at room temperature. Unreacted DTNB was removed by gel filtration. The [C96S]FGF was added to DTNB-treated SAP (3:1 molar ratio of [C96S]FGF:SAP) and incubated at room temperature for about 1 hr or for 16 hrs at 4°C. The mixture was loaded on heparin sepharose in 10 mM NaPO₄, 1 mM EDTA, pH 6 and the conjugate and free [C96S]FGF were eluted with 2 M NaCl in 10 mM NaPO₄, 1 mM EDTA, pH 6. The free [C96S]FGF was removed by gel filtration on Sephacryl S100 (Pharmacia). The resulting conjugate was designated CCFS4.

### C. Cytotoxicity of [C78S]FGF-SAP (CCFS2), [C96S]FGF-SAP (CCFS3) and [C96S]FGF-rSAP (CCFS4)

Cytotoxicity experiments were performed with the Promega (Madison, WI) CellTiter 96 Cell Proliferation/Cytotoxicity Assay. Cell types used were SK-Mel-28, human melanoma Swiss 3T3 mouse fibroblasts (from Dr. Pamela Maher, La Jolla, CA), B16F10, mouse melanoma, PA-1, human ovarian carcinoma (from Dr. Julie Beitz, Roger Williams Hospital, Providence RI), and baby hamster kidney (BHK) [obtained from the American Type Culture Collection (ATCC)]. 2500 cells were plated per well.

Cytotoxicity of the two mutant FGF-SAPs to several cell types has been tested. Heterogeneous FGF-SAP (CCFS1) is very cytotoxic to SK-MEL-28 cells, human melanoma cells, with an ED₅₀ of approximately 8 ng/ml. The mutant FGF-SAPs are also potently cytotoxic to these cells. [C78S]FGF-SAP and [C96S]FGF-SAP each have an ED₅₀ comparable to the heterogeneous chemically conjugates, indicting that mutant FGFs are able to internalize SAP to virtually the same extent as the heterogeneous FGF-SAP. Similar results were obtained with an ovarian carcinoma cell type, PA-1, Swiss 3T3 cells, B16F10, a mouse melanoma and BHK cells. CCFS4 was tested in the *in vitro* cytotoxicity assay and its activity is as good or better than the wild-type chemical conjugate (CCFS1).

### D. Preparation of homogeneous mixtures of FGF-SAP muteins by splicing by overlap extension (SOE)

### 1. Conversion of Cys 78 to Ser 78

Plasmid PZ1B (designated PZ1B1) described in Example 2 was used as the DNA template. The primers were prepared as follows:
Primer #1 spanning the *Nde*I site at the 5' end of the FGF-encoding DNA from plasmid pZ1B AAATACTTACATATGGCAGCAGGATC (SEQ ID NO. 73).
Primer #2 - Antisense primer to nucleotides spanning the Cys 78 (nucleotides 220-249 of SEQ ID NO. 1 with base change to generate Ser 78) CAGGTAACGGTTAGCAGACACTCCTTTGAT (SEQ ID NO. 74).
Primer #3 - Sense primer to nucleotides spanning the Cys 78 (nucleotides 220-249 of SEQ ID NO. 1 with base change to generate Ser 78) ATCAAAGGAGTGTCTGCTAACCGTTACCTG (SEQ ID NO. 75).
Primer #4 - Antisense primer to spanning the *Nco*I site of FGF in pZ1B (corresponding to nucleotides 456-485 of SEQ ID NO. 2)

GTGATTGATGTGACCATGGCGCTCTTAGCA (SEQ ID NO. 76).

### (a) Reaction A

PZ1B1 DNA (100 ng) was mixed (final volume of 100 µl upon addition of the Taq polymerase) with primer #1 (50 µM); primer #2 (50 µM), 10 mM Tri-HCl (pH 8.3), 50 mM KCI, 0.01% gelatin, 2 mM MgCl₂, 0.2 mM dNTPs.

### (b) Reaction B

Same as above except that primer #3 (50 µM) and primer #4 (50 µM) were used in place of primers #1 and #2.

Each reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Ericomp). Each cycle included a denaturation step (95°C for 1 min.), an annealing step (60°C for 1.5 min.), and an elongation step (75°C for 3 min.). After 20 cycles, the reaction mixture was incubated at 75°C for 10 minutes for a final elongation. The products were resolved on a 2% agarose gel and DNA of the correct size (247 bp and 250 bp) was purified. The ends were repaired by adding nucleoside triphosphates and Klenow DNA polymerase.

### (c) Reaction C

One µl of each product of reactions A and B were mixed (final volume of 100 µL upon addition of Taq polymerase) with primers #1 and #4 (final concentration of each was 50 µM); 10 mM Tri-HCl (pH 8.3), 50 mM KCI, 0.01% gelatin, 2 mM MgCl₂, 0.2 mM dNTPs.

The resulting reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Erricomp). Each cycle included a denaturation step (95°C for 1 min.), an annealing step (60°C for 1.5 min.), and an elongation step (75°C for 3 min.), followed, after 20 cycles, by a final elongation step at 75°C for 10 minutes.

The amplified product was resolved on a 1.5% agarose gel and the correct size fragment (460 bp), designated FGFC78S-SAP was purified.

### 2. Generation of DNA encoding FGFC78/C96S-SAP

DNA encoding FGFC78S-SAP served as the template. Primers used were Primer #5, Sense primer spanning the Cys 96 (nucleotides 275-300 of SEQ ID NO. 1 with base change to generate Ser 96)

TGGCTTCTAAATCTGTTACGGATGAG (SEQ ID NO. 77);

Primer #6, Antisense primer spanning the Cys 96 (nucleotides 275-300 of SEQ ID NO. 1 with base change to generate Ser 96)

CTCATCCGTAACAGATTTAGAAGCCA (SEQ ID NO. 78).

### (a) Reaction D

FGFC78S-SAP-encoding DNA (100 ng) was mixed (final volume of 100 µl upon addition of the Taq polymerase) with primer #1 (50 µM); primer #5 (50 µM), 10 mM Tri-HCl (pH 8.3), 50 mM KCl, 0.01% gelatin, 2 mM MgCl₂ and 0.2 mM dNTPs.

### (b) Reaction E

Same as above, except that primers #4 and #6 (50 µM final concentration of each) were used instead of primers #1 and #5.

Each reaction mixture was heated to 95°C for 5 min, 0.5 U TaqI DNA polymerase (1 µl; Boehringer Mannheim) was added and the mixture was overlaid with 100 µl of mineral oil (Perkin Elmer Cetus). Incubations were done in a DNA Thermal Cycler (Ericomp). Each cycle included a denaturation step (95°C for 1 min.), an annealing step (60°C for 1.5 min.), and an elongation step (75°C for 3 min.) for 20 cycles, followed by a final elongation step at 75°C for 10 minutes. The products were resolved on a 2% agarose gel and DNA of the correct size (297 bp and 190 bp) was purified. The ends were repaired by adding nucleoside triphosphates and Klenow DNA polymerase.

### (c) Reaction F

The product of reactions D and E (100 ng of each) were mixed (final volume of 100 µL upon addition of Taq polymerase) with primers #1 and #4 and amplified as described above. The amplified product resolved on a 1.5% agarose gel and the correct size fragment (465 bp) was purified. The resulting product, DNA that encodes FGFC78/96S-SAP, had *Nde*I and *Nco*I ends. It was digested with *Nde*I and *Nco*I and ligated into *Nde*I/*Nco*I-digested PZ1B1 and into *Nde*I/*Nco*I-digested PZ1C1 (PZIC described in Example 2 above). The resulting constructs were designated PZ2B1 and PZ2C1, respectively.

### E. Expression of the recombinant FGFC78/96S-SAP fusion proteins (FPFS4) from PZ2B1 and PZ2C1

The two-stage method described above for production of FPFS1 was used to produce recombinant FGFC78/96S-SAP protein (hereinafter FPFS4). Two hundred and fifty mls of LB medium containing ampicillin (100 µg/ml) were inoculated with a fresh glycerol stock of PZ1B. Cells were grown at 30°C in an incubator shaker to an OD₆₀₀ of 0.7 and stored overnight at 4°C. The following day the cells were pelleted and resuspended in fresh LB medium (no ampicillin). The cells were divided into 5 1-liter batches and grown at 30°C in an incubator shaker to an OD₆₀₀ of 1.5. IPTG (SIGMA CHEMICAL, St. Louis, MO) was added to a final concentration of 0.1 mM and growth was continued for about 2 to 2.5 hours at which time cells were harvested by centrifugation.

In order to grow PZ2C1, prior to induction, the cells were grown in medium containing kanamycin (50µg/ml) in place of ampicillin.

### F. Biological Activity

The cytotoxicity of the mutein FGF-SAP produced from PZ2B1 (FPFS4) was assessed on SK MEL 28 cells and was at least equivalent to the activity of the wild type FGF-SAP chemical conjugate, and recombinant FGF-SAP produced from PZ1B1.

The *in vivo* activity of the mutein FGF-SAP produced from PZ2B1 has been tested in animals, and it appears to be less toxic than FGF-SAP from PZ1B1 (FPFS1).

### EXAMPLE 7

### PREPARATION OF FGF-SAP CONJUGATES THAT CONTAIN LINKERS ENCODING PROTEASE SUBSTRATES

### A. Synthesis of oligos encoding protease substrates

Complementary single-stranded oligos in which the sense strand encodes a protease substrate, have been synthesized either using a cyclone machine (Millipore, MA) according the instructions provided by the manufacturer, were made by Midland Certified Reagent Co. (Midland, TX), or by National Biosciences, Inc. (MN). The following oligos have been synthesized and introduced into constructs encoding bFGF-SAP.
**1.** Cathepsin B substrate linker: 5'- CCATGGCCCTGGCCCTGGCCCTGGCCCTGGCCATGG SEQ ID NO. 79
2. Cathepsin D substrate linker 5'- CCATGGGCCGATCGGGCTTCCTGGGCTTCGGCTTCCTGG GCTTCGCCAT GG -3' SEQ ID NO. 80
3x. Trypsin substrate linker 
5'- CCATGGGCCGATCGGGCGGTGGGTGCGCTGGTAATAGAGT
CAGAAGATCAGTCGGAAGCAGCCTGTCTTGCGGTGGTCTC
GACCTGCAGG CCATGG-3' SEQ ID NO. 81
**4.** Gly₄Ser 5'- CCATGGGCGG CGGCGGCTCT GCCATGG -3' SEQ ID NO. 37
5. (Gly₄Ser)₂ 
5'- CCATGGGCGGCGGCTCTGGCGGCGGCTC
TGCCATGG -3' SEQ ID NO. 38
**6.** (Ser₄Gly)₄ 
5'- CCATGGCCTCGTCGTCGTCGGGCTCGTCGTCGTC
GGGCTCGTCGTCGTCGGGCTCGTCGTCGTCGGGC
GCCATGG -3' SEQ ID NO. 39
7. (Ser₄Gly)₂ 
5- CCATGGCCTCGTCGTCGTCGGGCTCGTCGTCGTC
GGGCGCCATGG -3' SEQ ID NO. 40
8. Thrombin substrate linker 
CTG GTG CCG CGC GGC AGC  SEQ ID NO. 82
Leu Val Pro Arg Gly Ser
9. Enterokinase substrate linker 
GAC GAC GAC GAC CCA  SEQ ID NO. 83
Asp Asp Asp Asp Lys
10. Factor Xa substrate

ATC GAA GGT CGT   SEQ ID NO. 84
Ile Glu gly Arg

### B. Preparation of DNA constructs encoding FGF-Linker-SAP

The complementary oligos were annealed by heating at 95°C for 15 min., cooled to room temperature, and then incubated at 4°C for a minute to about an hour. Following the incubation, the oligos were digested with *Nco*I and ligated overnight, at a 3:1 (insert:vector) ratio, at 15°C to *Nco*I-digested PZ1B, PZ1C or PZ2B (see Examples 2B and 6), which had been treated with alkaline phosphatase (Boehringer Mannheim).

Bacteria (NovaBlue) (Novagen, Madison, WI)) were transformed with ligation mixture (1 µl) and plated on LB-amp or LB-Kan, depending upon the plasmid). Colonies were selected, clones isolated and sequenced to determine orientation of the insert. Clones with correct orientation were used to transform strain expression strain BL21(DE3). Glycerol stocks were generated from single transformed colonies. The transformed strains were cultured as described in Example 2 and fusion proteins with linkers were expressed.

The DNA and amino acid sequences of exemplary fusion proteins, containing cathepsin B substrate (FPFS9), cathepsin D substrate (FPFS5), Gly₄Ser (FPFS7), (Gly₄Ser)₂ (FPFS8), trypsin substrate (FPFS6), (Ser₄Gly)₄ (FPFS12) and (Ser₄Gly)₂ (FPFS11) linkers, respectively, are set forth in SEQ ID NOS. 45-51 *(see, also*, Table 4).

### C. Expression of conjugates with linkers

DNA encoding the conjugates set forth above and summarized in Table 4 have been expressed in BL21 cells as described above for PZ1B1 using plasmids prepared as described above.

### EXAMPLE 8

### EFFECTS OF AN FGF-SAPORIN CONJUGATE IN A RAT BALLOON DENUDATION MODEL

Balloon catheter denudation is performed on the left carotid artery of 5-6 month old male Sprague-Dawley rats by intraluminal passage of a 2F Fogarty balloon. Body weights range from 300-350 g the day prior to surgery. At 0, 3, 6 and 9 days after balloon injury, FGF-SAP (15 ug/kg/dose) or vehicle (0.9% NaCl, 0.1% human serum albumin (HSA)) is injected via the tail vein. The therapeutic composition is prepared by mixing the test materials with appropriate volumes of 0.9% NaCl, 0.1% HSA. FGF-SAP is supplied in 10 mM NaCitrate, 140 mM NaCl, 0.1 mM EDTA, pH 6 at a concentration of 0.5 mg/ml prior to being prepared in appropriate dosages. On day 14 after balloon denudation, approximately 120 hr after the final dose, animals are sacrificed with an overdose of intravenous KCl under deep anesthesia. One hour before sacrifice, animals are injected intravenously with Evans blue dye (0.5 ml, 5% in saline) to confirm endothelial denudation. At one and 17 hours prior to sacrifice, animals are injected intraperitoneally with Bromodeoxyuridine (BrdU, 30 mg/kg) for quantitation of cellular proliferation. At sacrifice, the arterial tree is perfused at 80 mm Hg with Hank's balanced salt solution, 15 mM HEPES, pH 7.4 until the perfusate from the jugular is clear of blood. The arterial tree is then perfused with 2% paraformaldehyde in 0.1M Na Cacodylate buffer, pH 7.4, for 15 minutes. The carotid arteries are then removed, cut into sections, and processed for light microscopy. Tissue samples are dehydrated and embedded in paraffin, cut into 4 µ sections, and stained with hematoxylin-eosin and Movat pentachrome stain. Vessels are then measured for intimal, medial, and neointimal areas by computerized planimetry. Anti-BrdU antibody is used for detection of BrdU positive cells; smooth muscle cell proliferation is quantitated by counting BrdU positive cells as a percent of total smooth muscle cells.

### EXAMPLE 9

### EFFECT OF STENT TREATMENT ON PIG RESTENOSIS

An *in vivo* assay for restenosis entails implantation of a stent in the coronary vessels of pigs with an overstretch injury at a 1:1.2-1.3 artery to balloon ratio. In this model, heparin-coated stents with and without FGF-SAP bound are delivered and deployed into the left anterior descending coronary artery (LAD), right coronary artery (RCA), or the left circumflex coronary artery (LCx) of juvenile farm swine. The appropriate vasculature is accessed via the right or left femoral artery. Animals are sacrificed 28 days after stent implantation. The heart is then removed in its entirety, flushed with lactated Ringers solution, and fixed by perfusion technique. Gross and microscopic examination of the heart and implanted vasculature is then performed.

### EXAMPLE 10

### BINDING OF CONJUGATE TO HEPARINIZED STENTS

FGF-SAP was bound to heparin-coated stents. The amount of FGF-SAP fusion protein that bound to each stent was determined as follows. Heparin-coated stents were incubated with various concentrations of FGF-SAP (0-600 µg/ml) and 60 ng/ml of ¹²⁵I-FGF-SAP for 30 min at 22°C with shaking. Stents were washed three times in 10 mM NaCitrate, pH 6, 140 mM NaCl, 0.1 mM EDTA, 0.1% gelatin, 0.05% Tween-20 and counted in a gamma counter. Bound protein was calculated based on the specific activity of ¹²⁵I-FGF-SAP and the ratio of labeled to unlabeled FGF-SAP, Scatchard analysis was performed on the data obtained and corrected for actual surface area. In Figure 1, the extent of specific binding to heparin-coated stents is presented, and shows that FGF-SAP bound in a dose-dependent manner.

The specificity of FGF-SAP binding was established by inhibition of binding of a fixed quantity of ¹²⁵I-FGF-SAP to heparinized stents in the presence or absence of 4 mg/ml heparin or 2.5 mg/ml unlabeled FGF-SAP. Stents were washed with buffer as above, and incubated for 30 min at 22°C with ¹²⁵I-FGF-SAP alone, with added heparin, or with added unlabeled FGF-SAP. Stents were washed three times and counted in a gamma counter. Stents incubated with FGF-SAP alone were also washed in 2M NaCl to disrupt specific FGF-heparin binding. As seen in Figure 2, FGF-SAP bound to heparin-coated stents, but not in the presence of excess free heparin or FGF-SAP. Bound FGF-SAP was eluted off with 2M NaCl. In addition, ¹²⁵I-BSA was incubated with heparinized stents but did not bind.

The release of FGF-SAP from heparin-coated stents in human plasma was assessed over a 19-hour time period during a 37°C incubation in the presence of increasing concentrations of heparin. Approximately 3.0 µg/cm² of unlabeled FGF-SAP doped with a trace amount of ¹²⁵I-FGF-SAP was bound to two different heparin-coated stent preparations. Stents were then incubated in normal human plasma collected in EDTA along with heparin. Samples were removed over a 19 hour time course and counted. As seen in Figure 3, release of FGF-SAP occurred to a small extent in the absence of heparin. Appreciable release occurred in the presence of 100 µg/ml heparin.

Biological activity was measured on both proliferating and quiescent smooth muscle cells. Porcine aortic smooth muscle cells were seeded at 10,000 cells/well (subconfluent density) in 24 well tissue culture plates. Two days later, FGF-SAP (from 0.01 nM to 50 nM) was added to the cells. Five days later, cells were fixed and stained with methylene blue. As shown in Figure 4, increasing doses of FGF-SAP had an increasing anti-proliferative effect on the cells.

The effect of FGF-SAP on proliferating and quiescent cells was also assessed. Porcine aortic smooth muscle cells were seeded as above. Either 2 days later (proliferative cells) or seven days later (quiescent cells), a stent, heparinized stent or a heparinized stent bound with FGF-SAP was added to the wells. Five days later, cells were fixed and stained with methylene blue. As can be seen in Figures 4 and 5, the heparinized stent bound with FGF-SAP had an anti-proliferative effect on proliferating cells, and less of an effect on quiescent cells.

From the foregoing, it will be appreciated that, although specific embodiments of the invention have been described herein for purposes of illustration, various modifications may be made without deviating from the scope of the invention. Accordingly, the invention is not limited except as by the appended claims.

## Claims

1. An intravenous medical device coated with a glycosaminoglycan, the glycosaminoglycan having bound thereto a glycosaminoglycan-binding moiety conjugated to a cytotoxic agent.

2. An intravenous medical device coated with a glycosaminoglycan, the glycosaminoglycan having bound thereto a glycosaminoglycan-binding moiety conjugated to a cytotocide encoding agent.

3. The device of claim 2 wherein the glycosaminoglycan-cytocide encoding agent conjugate further comprises a nucleic acid binding domain.

4. An intravenous medical device coated with heparin, the heparin having bound thereto a heparin-binding growth factor (HBGF) conjugated to a cytotoxic agent.

5. An intravenous medical device coated with heparin, the heparin having bound thereto a heparin-binding growth factor (HBGF) conjugated to a cytocide encoding agent.

6. The device of claim 5 wherein the HBGF-cytocide encoding agent conjugate further comprises a nucleic acid binding domain.

7. The device of either of claims 4 or 5 wherein the HBGF is a polypeptide reactive with a fibroblast growth factor (FGF) receptor.

8. The device of either of claims 4 or 5 wherein the HBGF is selected from the group consisting of FGF-1, FGF-2, FGF-3, FGF-4, FGF-5, FGF-6, FGF-7, FGF-8 and FGF-9.

9. The device of either of claims 1 or 4 wherein the cytotoxic agent is a ribosome inactivating protein.

10. The device of claim 9 wherein the ribosome inactivating protein is saporin.

11. The device as in any one of claims 1, 2, 4, or 5 wherein the device is a stent.

12. The device as in any one of claims 1, 2, 4, or 5 wherein the device is a synthetic graft.

13. A synthetic vascular graft comprising a conjugate of HBGF and a cytotoxic agent, wherein the conjugate is impregnated in the graft.

14. A synthetic vascular graft comprising a conjugate of HBGF and a cytocide encoding agent. wherein the conjugate is impregnated in the graft.

15. The synthetic vascular graft according to either of claims 13 or 14 wherein the graft is made of expanded polytetrafluoroethylene.

16. A stent according to claim 11 for use in a method of inhibiting proliferation of smooth muscle cells following balloon angioplasty, vein graft or endarterectomy, comprising contacting said stent with smooth muscle cells of a vessel.

17. A shunt or graft coated with heparin. the heparin having bound thereto an HBGF conjugated to a cytotoxic agent for use in a method of inhibiting proliferation of smooth muscle cells following arteriovenous shunt or synthetic vascular graft surgery, comprising surgically implanting said shunt or graft.

18. A shunt or graft coated with heparin, the heparin having bound thereto an HBGF conjugated to a cytocide encoding agent for use in a method of inhibiting proliferation of smooth muscle cells following arteriovenous shunt or synthetic vascular graft surgery, comprising surgically implanting said shunt or graft.

19. The shunt or graft of either of claims 17 or 18 wherein the HBGF is a polypeptide reactive with an FGF receptor.

20. The shunt or graft of claim 19 wherein the polypeptide reactive with an FGF receptor is FGF-2.

21. A stent coated with heparin for use in a method of inhibiting restenosis or stenosis, comprising contacting said stent with smooth muscle cells of a vessel, the heparin having bound thereto an HBGF conjugated to a cytotoxic agent.

22. A stent coated with heparin for use in a method of inhibiting restenosis or stenosis, comprising contacting said stent with smooth muscle cells of a vessel, the heparin having bound thereto an HBGF conjugated to a cytocide encoding agent.

23. A synthetic graft according to claim 14 for use in a method of inhibiting stenosis in a vessel at a graft anastamosis comprising joining the vessel with said synthetic graft.

24. A stent according to claim 11 for use in a method of inhibiting restenosis or stenosis following balloon angioplasty, vein graft or endarterectomy, comprising implanting said stent in a vessel.

## Patentansprüche

1. Intravenöse medizinische Vorrichtung, beschichtet mit einem Glykosaminoglykan, wobei das Glykosaminoglykan eine daran gebundene Glykosaminoglykan-bindende Gruppe aufweist, die mit einem zytotoxischen Mittel konjugiert ist.

2. Intravenöse medizinische Vorrichtung, beschichtet mit einem Glykosaminoglykan, wobei das Glykosaminoglykan eine daran gebundene Glykosaminoglykan-bindende Gruppe aufweist, die mit einem für ein Zytozid-kodierenden Mittel konjugiert ist.

3. Vorrichtung nach Anspruch 2, wobei das Glykosaminoglykan-für ein Zytozid kodierendes Mittel-Konjugat weiterhin eine Nukleinsäure-bindende Domäne umfaßt.

4. Intravenöse medizinische Vorrichtung, beschichtet mit Heparin, wobei das Heparin einen daran gebundenen Heparin bindenden Wachstumsfaktor (HBGF), konjugiert mit einem zytotoxischen Mittel, aufweist.

5. Intravenöse medizinische Vorrichtung, beschichtet mit Heparin, wobei das Heparin einen daran gebundenen Heparin-bindenden Wachstumsfaktor (HBGF), konjugiert mit ein für ein Zytozid kodierendes Mittel, aufweist.

6. Vorrichtung nach Anspruch 5, wobei das HBGF- für das Zytozid-kodierende Mittel-Konjugat weiterhin eine Nukleinsäure-bindende Domäne umfaßt.

7. Vorrichtung nach einem der Ansprüche 4 oder 5, wobei das HBGF ein mit einem Fibroblasten-Wachstumsfaktor (FGF) Rezeptor reaktives Polypeptid ist.

8. Vorrichtung nach Anspruch 4 oder 5, wobei das HBGF ausgewählt ist aus der Gruppe bestehend aus FGF-1, FGF-2, FGF-3, FGF-4, FGF-5, FGF-6, FGF-7, FGF-8 und FGF-.

9. Vorrichtung nach einem der Ansprüche 1 oder 4, wobei das zytotoxische Mittel ein Ribosom-inaktivierendes Protein ist.

10. Vorrichtung nach Anspruch 9, wobei das Ribosom-inaktivierende Protein Saporin ist.

11. Vorrichtung wie in einem der Ansprüche 1, 2, 4 oder 5, wobei die Vorrichtung ein Stent ist.

12. Vorrichtung wie in einem der Ansprüche 1, 2, 4 oder 5, wobei die Vorrichtung ein synthetisches Implantat ist.

13. Synthetisches vaskuläres Implantat, umfassend ein Konjugat von HBGF und einem zytotoxischen Mittel, wobei das Implantat mit dem Konjugat imprägniert ist.

14. Synthetisches vaskuläres Implantat, umfassend ein Konjugat von HBGF mit einem für ein Zytozid kodierenden Mittel, wobei das Implantat mit dem Konjugat imprägniert ist.

15. Synthetisches vaskuläres Implantat nach einem der Ansprüche 13 oder 14, wobei das Implantat aus expandiertem Polytetrafluorethylen gemacht ist.

16. Stent nach Anspruch 11 zur Verwendung in einem Verfahren zur Inhibierung der Proliferation von glatten Muskelzellen im Anschluß an eine Ballon-Angioplastie, Venenimplantation oder Endarteriektomie, umfassend, in Kontakt bringen des Stents mit glatten Muskelzellen eines Gefäßes.

17. Shunt oder Implantat, beschichtet mit Heparin, wobei das Heparin ein daran gebundenes HBGF, das mit einem zytotoxischen Mittel konjugiert ist, aufweist, zur Verwendung in einem Verfahren zur Inhibierung der Proliferation von glatten Muskelzellen im Anschluß an einen arterio-venösen Shunt oder synthetischer vaskulärer Implantat-Chirurgie, umfassend chirurgische Einpflanzung des Shunts oder Implantats.

18. Shunt oder Implantat, beschichtet mit Heparin, wobei das Heparin ein daran gebundenes HBGF, konjugiert mit einem für ein Zytozid kodierenden Mittel, aufweist, zur Verwendung in einem Verfahren zur Inhibierung der Proliferation von glatten Muskelzellen im Anschluß an einen arterio-venösen Shunt oder synthetische vaskuläre Implantat-Chirurgie, umfassend chirurgisches Einpflanzen des Shunts oder Implantats.

19. Shunt oder Implantat nach einem der Ansprüche 17 oder 18, wobei das HBGF ein mit einem FGF-Rezeptor reaktives Polypeptid ist.

20. Shunt oder Implantat von Anspruch 19, wobei das Polypeptid, das reaktiv mit einem FGF-Rezeptor ist, FGF-2 ist.

21. Mit Heparin beschichteter Stent zur Verwendung in einem Verfahren zur Inhibierung der Restenosis oder Stenosis, umfassend in Kontakt bringen des Stents mit glatten Muskelzellen eines Gefäßes, wobei das Heparin einen daran gebundenen HBGF, konjugiert mit einem zytotoxischen Mittel, aufweist.

22. Mit Heparin beschichteter Stent zur Verwendung in einem Verfahren zur Inhibierung des Restenosis oder Stenosis, umfassend in Kontakt bringen des Stents mit glatten Muskelzellen eines Gefäßes, wobei das Heprin einen daran gebundenen HBGF, konjugiert mit einem für ein Zytozid kodierenden Mittel, aufweist.

23. Synthetisches Implantat nach Anspruch 14 zur Verwendung in einem Verfahren zur Inhibierung der Stenosis in einem Gefäß an einer Implantat-Anastamosis, umfassend Verbinden des Gefäßes mit dem synthetischen Implantat.

24. Stent nach Anspruch 11, zur Verwendung in einem Verfahren zur Inhibierung der Restenosis oder Stenosis im Anschluß an Ballon-Angioplastie, Venenimplantation oder Endarteriektomie, umfassend Implantieren des Stents in ein Gefäß.

## Revendications

1. Dispositif médical intraveineux revêtu d'un glycosaminoglycane, un groupement de liaison de glycosaminoglycane, conjugué à un agent cytotoxique, étant lié audit glycosaminoglycane.

2. Dispositif médical intraveineux revêtu d'un glycosaminoglycane, un groupement de liaisons de glycosaminoglycane, conjugué à un agent codant pour un cytocide, étant lié audit glycosaminoglycane.

3. Dispositif suivant la revendication 2, dans lequel le conjugué glycosaminoglycane - agent codant pour un cytocide comprend en outre un domaine de liaison d'acide nucléique.

4. Dispositif médical intraveineux revêtu d'héparine, un facteur de croissance de liaison à l'héparine (HBGF), conjugué à un agent cytotoxique, étant lié à ladite héparine.

5. Dispositif médical intraveineux revêtu d'héparine, un facteur de croissance de liaison à l'héparine (HBGF), conjugué à un agent codant pour un cytocide, étant lié à ladite héparine.

6. Dispositif suivant la revendication 5, dans lequel le conjugué HBGF - agent codant pour un cytocide comprend en outre un domaine de liaison d'acide nucléique.

7. Dispositif suivant la revendication 4 ou 5, dans lequel le HBGF est un polypeptide réactif avec un récepteur de facteur de croissance de fibroblastes (FGF).

8. Dispositif suivant la revendication 4 ou 5, dans lequel le HBGF est choisi dans le groupe consistant en les FGF-1, EGF-2, FGF-3, FGF-4, FGF-5, FGF-6, FGF-7, EGF-8 et FGF-9.

9. Dispositif suivant la revendication 1 ou 4, dans lequel l'agent cytotoxique est une protéine d'inactivation de ribosome.

10. Dispositif suivant la revendication 9, dans lequel la protéine d'inactivation de ribosome est la saponine.

11. Dispositif suivant l'une quelconque des revendications 1, 2, 4 et 5, qui consiste en un extenseur.

12. Dispositif suivant l'une quelconque des revendications 1, 2, 4 et 5, qui consiste en un greffon synthétique.

13. Greffon vasculaire synthétique comprenant un conjugué de HBGF et d'un agent cytotoxique, dans lequel le conjugué imprègne le greffon.

14. Greffon vasculaire synthétique comprenant un conjugué de HBGF et un agent codant pour un cytocide, dans lequel le conjugue imprègne le greffon.

15. Greffon vasculaire synthétique suivant la revendication 13 ou 14, ledit greffon étant constitué de polytétrafluoréthylène expansé.

16. Extenseur suivant la revendication 11, destiné à être utilisé dans une méthode d'inhibition de la prolifération des cellules des muscles lisses après angioplastie par ballonnet, greffe veineuse ou endartériectomie, comprenant la mise en contact dudit extenseur avec les cellules des muscles lisses d'un vaisseau.

17. Elément de dérivation ou greffon revêtu d'héparine, un HBGF conjugué à un agent cytotoxique étant lié à ladite héparine, destiné à être utilisé dans une méthode d'inhibition de la prolifération des cellules des muscles lisses après une intervention chirurgicale de dérivation artérioveineuse ou de greffe vasculaire synthétique, comprenant l'implantation chirurgicale dudit élément de dérivation ou greffon.

18. Elément de dérivation ou greffon revêtu d'héparine, un HBGF conjugué à un agent codant pour un cytocide étant lié à ladite héparine, destiné à être utilisé dans une méthode d'inhibition de la prolifération des cellules des muscles lisses après une intervention chirurgicale de dérivation artérioveineuse ou de greffe vasculaire synthétique, comprenant l'implantation chirurgicale dudit élément de dérivation ou greffon.

19. Elément de dérivation ou greffon suivant la revendication 17 ou 18, dans lequel le HBGF est un polypeptide réactif avec un récepteur de FGF.

20. Elément de dérivation ou greffon suivant la revendication 19, dans lequel le polypeptide réactif avec un récepteur de FGF est le FGF-2.

21. Extenseur revêtu d'héparine, destiné à être utilisé dans une méthode d'inhibition d'une resténose ou sténose, comprenant la mise en contact dudit extenseur avec les cellules des muscles lisses d'un vaisseau, un H3GF conjugué à un agent cytotoxique étant lié à l'héparine.

22. Extenseur revêtu d'héparine, destiné à être utilisé dans une méthode dite d'inhibition d'une resténose ou sténose, comprenant la mise en contact dudit extenseur avec les cellules des muscles lisses d'un vaisseau, un HBGF conjugué à un agent codant pour un cytocide étant lié à l'héparine.

23. Greffon synthétique suivant la revendication 14, destiné à être utilisé dans une méthode d'inhibition d'une sténose dans un vaisseau au niveau d'une anastomose du greffon, comprenant la jonction du vaisseau audit greffon synthétique.

24. Extenseur suivant la revendication 11, destiné à être utilisé dans une méthode d'inhibition d'une resténose ou sténose après angioplastie à ballonnet, greffe veineuse ou endartériectomie, comprenant l'implantation dudit extenseur dans un vaisseau.
